# EUROPEAN PATENT SPECIFICATION

(11) **EP 1 836 312 B1**
(45) Date of publication and mention of the grant of the patent: **12.10.2011**
(21) Application number: 05851755.8
(22) Date of filing: 18.11.2005
(51) Int. Cl.: C12Q 1/68

(54) **METHODS AND COMPOSITIONS FOR DIAGNOSING AGE-RELATED MACULAR DEGENRATION**
VERFAHREN UND ZUSAMMENSETZUNGEN ZUR DIAGNOSE VON ALTERSBEDINGTER MACULADEGENERATION
METHODES ET PREPARATIONS POUR LE DIAGNOSTIC DE LA LA DÉGÉNÉRATION MACULAIRE LIÉE À L'ÂGE

(30) Priority: 18.11.2004 US 629363 P; 02.02.2005 US 649479 P; 18.04.2005 US 672346 P
(43) Date of publication of application: 26.09.2007
(73) Proprietor: Yale University, New Haven, CT 06511 (US); THE ROCKEFELLER UNIVERSITY, New York, NY 10021 (US)
(72) Inventor: HOH, Josephine, Westport, CT 06880 (US); KLEIN, Robert, J., New York, NY 10023 (US)
(74) Representative: Lock, Graham James
(86) International application number: PCT/US2005/041664
(87) International publication number: WO 2006/062716

(56) References cited:
- WO-A-01/84149
- DATABASE SNPDB NCBI; 13 September 2000 (2000-09-13), XP002392012 retrieved from HTTP://WWW.NCBI.NLM.NIH.GOV Database accession no. rs1061170
- DATABASE SNPDB ncbi; 2 September 2000 (2000-09-02), XP002392013 retrieved from HTTP://WWW.NCBI.NLM.NIH.GOV Database accession no. rs800292
- PEREZ-CABALLERO DAVID ET AL: "Clustering of missense mutations in the C-terminal region of factor H in atypical hemolytic uremic syndrome" AMERICAN JOURNAL OF HUMAN GENETICS, vol. 68, no. 2, February 2001 (2001-02), pages 478-484, XP002391904 ISSN: 0002-9297
- SANCHEZ-CORRAL PILAR ET AL: "Molecular basis for factor H and FHL-1 deficiency in an Italian family" IMMUNOGENETICS, vol. 51, no. 4-5, April 2000 (2000-04), pages 366-369, XP002391905 ISSN: 0093-7711
- KLEIN ROBERT J ET AL: "Complement factor H polymorphism in age-related macular degeneration" SCIENCE (WASHINGTON D C), vol. 308, no. 5720, 10 March 2005 (2005-03-10), pages 385-389, XP002391906 ISSN: 0036-8075
- HAINES JONATHAN L ET AL: "Complement factor H variant increases the risk of age-related macular degeneration" SCIENCE (WASHINGTON D C), vol. 308, no. 5720, 10 March 2005 (2005-03-10), pages 419-421, XP002391907 ISSN: 0036-8075
- EDWARDS ALBERT O ET AL: "Complement factor H polymorphism and age-related macular degeneration" SCIENCE (WASHINGTON D C), vol. 308, no. 5720, 10 March 2005 (2005-03-10), pages 421-424, XP002391908 ISSN: 0036-8075
- HAGEMAN GREGORY S ET AL: "A common haplotype in the complement regulatory gene factor H (HF1/CFH) predisposes individuals to age-related macular degeneration" PROCEEDINGS OF THE NATIONAL ACADEMY OF SCIENCES OF THE UNITED STATES OF AMERICA, vol. 102, no. 20, 3 May 2005 (2005-05-03), pages 7227-7232, XP002391909 ISSN: 0027-8424
- ZAREPARSI SEPIDEH ET AL: "Strong association of the Y402H variant in complement factor H at 1q32 with susceptibility to age-related macular degeneration" AMERICAN JOURNAL OF HUMAN GENETICS, vol. 77, no. 1, 13 May 2005 (2005-05-13), pages 149-153, XP002391910 ISSN: 0002-9297
- SCHOLL H P N ET AL: "Y402H polymorphism in complement factor H and age-related macula degeneration (AMD)" OPHTHALMOLOGE 2005 GERMANY, vol. 102, no. 11, 17 September 2005 (2005-09-17), pages 1029-1035, XP002391911 ISSN: 0941-293X
- DE CORDOBA SANTIAGO RODRIGUEZ ET AL: "The human complement factor H: functional roles, genetic variations and disease associations" MOLECULAR IMMUNOLOGY, vol. 41, no. 4, 3 April 2004 (2004-04-03), pages 355-367, XP002391912 ISSN: 0161-5890
- SHARMA A K ET AL: "Biologically active recombinant human complement factor H: synthesis and secretion by the baculovirus system." GENE. 10 JUN 1994, vol. 143, no. 2, 10 June 1994 (1994-06-10), pages 301-302, XP002392003 ISSN: 0378-1119

## Description

### BACKGROUND OP THE INVENTION

Age-related macular degeneration (AMD) is the leading cause of age-related blindness in the developed world. Its incidence is increasing as lifespan lengthens and the elderly population expands (D.S. Friedman et al., Arch Ophthalmol 122, 564 (2004)). It is a chronic disease characterized by progressive destruction of the retina's ccntral region (macula), causing centrat field visual loss (J. Tuo, C. M. Bojanowski, C. C. Chan, Prog Retin Eyc Res 23, 229 (2004)). One key characteristic of AMD is the formation of extracellular deposits called drusen that are concentrated in and around the macula behind the retina between the retina pigment epithelium (RPE) and choroid. To date, no therapy for this disease has proven to be broadly effective, especially in more advanced forms. Several risk factors have been linked to AMD, including age, smoking, and family history (AREDS Rosoarch Group, Ophthamology 107, 2224 (2000)). Candidate gene association studies and genome-wide linkage scans have been performed to identify genetic risk factors for AMD. A variety of candidate genes have been proposed b-ased on their association with other retinal diseases or their known function. While some rare variants of some of these genes are associated with disease phenotype, no genetic differences have been observed that can account for a largo proportion of the overall prevalence (J. Tuo, C. M. Bojanowski, C. C. Chan, Prog Retin Eye Res 23, 299 (2004)). Additional information about genetic daterminants of AMD is badly needed.

### SUMMARY OF THE INVENTION

The present invention relates to methods for identifying or aiding i identifying individuals at risk for developing AMD methods for diagnosing or aiding in the diagnosis of AMD.

In its various embodiments the present invention provides methods as defined in the claims.

The polynucleotides described herein (e.g., a polynucleotide probe or a polynucleotide printer) may be DNA or RNA. the subject polynucleotide may be single-strauded or double-stranded. Polynucleotide probes and primers may be from about 5 nucleotides to about 3000 nucleotides, or from about 8 nucleotides to about 500 nucleotides, or from about 10 nucleotides to about 250 nucleotides, or about 20 nucleotides (e.g., 15, 16, 17, 18, 19, 20, 21, 22, 23, 24, 25, 26, 27, 28, 29, or 30 nucleotides), or from about 50 to about 100 nucleotides (eg., 45, 50, 55, 60, 65, 75, 85, or 100 nucleotides). The polynucleotides may comprise one or more non-natural or modified nucleolides. Non-natural or modified nucleotides include, without limitation, radioactively, fluorescently, or chemically labeled nucleotides.

The polynucleotide primer may hybridize upstream or downstream from a variation in the CFH gone that is correlated with the occurrence of AMD in humans. In one embodiment, the polynucleotide hybridizes vicinal to a variation in the CFH gene that is correlated with the occurrence of AMD in humans. For example, hybridization may occur in such a manner that fewer than 10 or 1-3 nucleotides separate the variation and the end of the hybridized primer proximal to the variation. Alternatively, , the polynucleotide primer hybridizes immediately adjacent to the variation or a distance (e.g., at least 10 nucleotides) from a variation in the CFH gene that is correlated with the occurrence of AMD in humans. For example, hybridization may occur in such a manner that the end of the hybridized primer proximal to the variation is 10, 25, 50, 100, 250, 1000, 5000, or up to 10,000 nucleotides from the variation in the CFH gene. Also described herein is a pair of polynucleotide primers that specifically detect a variation in the CFH gene that is correlated with the occurrence of AMD in humans, wherein the first polynucleotide primer hybridizes to one side of the variation and the second polynucleotide primer hybridizes to the other side of the variation. A pair of polynucleotide primers that hybridize to a region of DNA that comprises a variation in the CFH gene that is correlated with the occurrence of AMD in humans may hybridize to the region in such a manner that the ends uf the hybridized primers proximal to the variation are from about 20 to about 10,000 nucleotides apart. Alternatively, the pair of polynucleotide primers that hybridize to a regions of DNA that comprises a variation in tho CFH gene that is correlated with the occurrence of AMD in humans may hybridize to the region in such a manner that the ends of the hybridized primers proximal to the variation are from about 100 to about 7,500 nucleotides apart, or from about 200 to about 5,000 nucleotides apart.

The disclosure also provides three or more polynucleotide primers useful for distinguishing between two alleles of the CFH gene (for example, a wildtype allele and an allele that is correlated with the occurrence of AMD in humans). The first primer hybridizes to a nucleotide sequence that is common to both alleles, such as a non-allelic nucleotide sequence that is upstream or downstream of the variation in the CFH gene that is correlated with the occurrence of AMD. A second primer specifically hybridizes to a sequence that is unique to a first allele (e.g., a variation in the CFH gene that is correlated with the occurrence of AMD in humans). A third primer specifically hybridizes to a nucleotide sequence that is unique to the second allele (e.g., a wildtype CFH gene). The set of three primers result in the amplification of a region of DNA that is dependent on which CFH allele is present in the sample. For instance, one region of DNA is amplified if the CFH gene has a variation in the CFH gene that is correlated with the occurrence of AMD, and another region is amplified if a wildtype CFH gene is present in the sample. Alternatively, two primers out of the set may hybridize to a nucleotide sequence that is common to two alleles of the CFH gene, such as non-allelic nucleotide sequences that are upstream and downstream of a variation in the CFH gene that is correlated with the occurrence of AMD in humans, and a third primer specifically hybridizes to one of the two alleles of the CFH gene (such as a wildtype allele or an allele that is correlated with the occurrence of AMD in humans.

A variety of variations in the CFH gene that predispose an individual to AMD may be detected by the methods and compositions described herein. In a particular embodiment, the variation encodes an amino acid other than histidine at position 402 of the CFH protein. In a specific embodiment, the variation encodes tyrosine at position 402 of the CFH protein. In another embodiment, the variation encodes an amino acid other than valine at position 62 of the CFH protein. In a specific embodiment, the variation encodes isoleucine at position 62 of the CFH protein. In other embodiments, the methods and compositions described herein may be used to detect variations in the CFH gene that predispose an individual to AMID, such as those listed in Tables 4, 5 and 7. For example, other variant genes, such as those in which the variation is in a coding region (e.g., variations that encode: an amino acid other than serine, such as alanine, at position 58 of the CFH protein; an amino acid other than arginine, such as histidine, at position 127 of the CFH protein; an amino acid other than glutamine, such as lysine, at position 400 of the CFH protein; an amino acid other than valine, such as isoleucine, at position 609 of the CFH protein; an amino acid other than serine, such as isoleucine, at position 890 of the CFH protein; an amino acid other than glutamic acid, such as aspartic acid, at position 936 of the CFH protein; an amino acid other than valine, such as leucine, at position 1007 of the CFH protein; an amino acid other than asparagine, such as tyrosine, at position 1050 of the CFH protein; an amino acid other than proline, such as glutamine, at position 1166 of the CFH protein; or an amino acid other than arginine, such as cysteine, at position 1210 of the CFH protein. See Tables 4, 5 and 7) can be detected using the methods and compositions described herein. Alternatively, variant genes in which the variation is in a noncoding region, such as those listed in Tables 4, 5 and 7, may detected using the methods and compositions described herein. As used herein, the term "variant CFH gene" refers to DNA that includes a variation in the CFH gene that is correlated with the occurrence of AMD. As used herein, the terms "wildtype CFH DNA" and "wildtype CFH gene" refer to DNA that does not include a variation in the CFH gene that is correlated with AMD.

The present disclosure also relates to a method of detecting, in a sample obtained from an individual, a variant CFH gene that is correlated with the occurrence of AMD in humans. Such a method may comprise: (a) combining the sample with a polynucleotide probe that hybridizes, under stringent conditions, to a variation in the CFH gene that is correlated with AMD in humans, but not to a wildtype CFH gene (wildtype CFH DNA is the term used above); and (b) determining whether hybridization occurs. The occurrence of hybridization indicates that a variant CFH gene that is correlated with age related macular degeneration is present in the sample. Samples used in the methods described herein may comprise cells from the eye, ear, nose, teeth, tongue, epidermis, epithelium, blood, tears, saliva, mucus, urinary tract, urine, muscle, cartilage, skin, or any other tissue or bodily fluid from which sufficient DNA or RNA can be obtained. Samples may be collected by a variety of means for collecting cells, such as for example, a buccal swab. The sample is processed, if necessary, to render the DNA or RNA that is present available for assaying in the methods described herein. For example, samples may be processed such that DNA from the sample is available for amplification or for hybridization to another polynucleotide. The processed samples may be crude lysates where available DNA or RNA is not purified from other cellular material, or may be purified to isolate available DNA or RNA. Samples may be processed by any means known in the art that renders DNA or RNA available for assaying in the methods described herein. Methods for processing samples include, but are not limited to, mechanical, chemical, or molecular means of lysing and/or purifying cells and cell lysates. Processing methods may include, for example, chromatographic methods such as ion exchange (e.g., cation and anion), size exclusion, gel filtration, affinity, and hydrophobic interaction chromatography, or ultrafiltration, electrophoresis, and immunoaffinity purification with antibodies specific for particular epitopes of the polypeptide.

The disclosure also provides a method of detecting, in a sample obtained from an individual, a variant CFH gene that is correlated with the occurrence of age related macular degeneration in humans, comprising: (a) combining the sample (referred to as a test sample) with a polynucleotide probe that hybridizes, under stringent conditions, to a variation in the CFH gene that is correlated with the occurrence of AMD in humans, thereby producing a combination; (b) maintaining the combination produced in step (a) under stringent hybridization conditions; and (c) comparing hybridization that occurs in the combination with hybridization in a control. The occurrence of hybridization in the combination but not in the control indicates that a variant CFH gene that correlates with AMD is present in the sample. In a further embodiment, the extent of hybridization is determined when comparing hybridization that occurs in the combination with hybridization in a control. The control is the same as the test sample and is treated the same as the test sample except that the polynucleotide probe is one that does not bind to a variation in the CFH gene that is correlated with the occurrence of AMD in humans. Alternatively, the polynucleotide probe is one that binds only to a wildtype CFH gene. The control can be assayed serially or simultaneously with the combination described above. Alternatively, results from a control may be established in a reference assay previously or subsequent to the combination described above. The sample used in the control is typically the same type of sample as the test sample and is treated the same as the test sample except that it is combined with a polynucleotide that does not hybridize to a variant CFH gene that is correlated with the occurrence of AMD in humans.

The disclosure also provides a method of detecting, in a sample obtained from an individual, a variant CFH gene that is correlated with the occurrence of AMD in humans, comprising: (a) combining a first portion of the sample with a polynucleotide probe that hybridizes, under stringent conditions, to a variation in the CFH gene that is correlated with the occurrence of AMD in humans; (b) combining a second portion of the sample with a polynucleotide probe that hybridizes, under stringent conditions, to a wildtype CFH gene; and (c) determining whether hybridization occurs. The occurrence of hybridization in the first portion, but not in the second portion, indicates that a variant CFH gene that is correlated with AMD is present in the sample.

In another embodiment, the invention provides a method of detecting, in a sample obtained from an individual, a variant CFH gene that is correlated with the occurrence of AMD in humans, comprising: (a) combining the sample with a pair of polynucleotide primers, wherein the first polynucleotide primer hybridizes to one side of DNA encoding amino acid 402 of the CFH protein and the second polynucleotide primer hybridizes to the other side of DNA encoding amino acid 402 of the CFH protein; (b) amplifying DNA in the sample, thereby producing amplified DNA; (c) sequencing amplified DNA; and (d) detecting in the DNA the presence of a variation that encodes an amino acid other than histidine at position 402 of the CFH protein. The presence of the variation indicates that a variant CFH gene that is correlated with the occurrence of AMD in humans is detected in the sample.

The disclosure also provides a method of detecting, in a sample obtained from an individual, a variant CFH gene that is correlated with the occurrence of AMD in humans, comprising: (a) combining the sample with a pair of polynucleotide primers, wherein the first polynucleotide primer hybridizes to one side of DNA encoding amino acid 62 of the CFH protein and the second polynucleotide primer hybridizes to the other side of DNA encoding amino acid 62 of the CFH protein; (b) amplifying DNA in the sample, thereby producing amplified DNA; (c) sequencing amplified DNA; and (d) detecting in the DNA the presence of a variation that encodes an amino acid other than histidine at position 62 of the CFH protein. The presence of the variation indicates that a variant CFH gene that is correlated with the occurrence of AMD in humans is detected in the sample.

Any method known in the art for amplifying nucleic acids may be used for the methods described herein. For example, DNA in a sample may be amplified using polymerase chain reaction (PCR), RT-PCR, quantitative PCR, real time PCR, Rapid Amplified Polymorphic DNA Analysis, Rapid Amplification of cDNA Ends (RACE), or rolling circle amplification.

The disclosure also provides methods of identifying or aiding in identifying an individual at risk for developing AMD. In one specific embodiment, such a method comprises assaying a sample obtained from the individual for the presence of a variant CFH gene that is correlated with the occurrence of AMD in humans. The presence of a variant CFH gene indicates that the individual is at risk for developing AMD.

In another embodiment, a method of identifying or aiding in identifying an individual at risk for developing AMD comprises: (a) combining a sample obtained from the individual with a polynucleotide probe that hybridizes, under stringent conditions, to a variation in the CFH gene that is correlated with AMD in humans, but does not hybridize to a wildtype CFH gene; and (b) determining whether hybridization occurs. The occurrence of hybridization indicates that the individual is at risk for developing AMD.

In another embodiment, a method of identifying or aiding in identifying an individual at risk for developing AMD, comprises: (a) obtaining DNA from an individual; (b) sequencing a region of the DNA that comprises the nucleotides that encode amino acid 402 of the CFH protein; and (c) determining whether a variation that encodes an amino acid other than histidine at position 402 of the CFH protein is present in the DNA. The presence of the variation indicates that the individual is at risk for developing AMD.

In another embodiment, a method of identifying or aiding in identifying an individual at risk for developing AMD, comprises: (a) obtaining DNA from an individual; (b) sequencing a region of the DNA that comprises the nucleotides that encode amino acid 62 of the CFH protein; and (c) determining whether a variation that encodes an amino acid other than valine at position 62 of the CFH protein is present in the DNA. The presence of the variation indicates that the individual is at risk for developing AMD.

The disclosure provides a method of detecting, in a sample obtained from an individual, a variant CFH polypeptide that is correlated with the occurrence of age related macular degeneration in humans. Such a method comprises: (a) combining the sample with an antibody that binds to a variant CFH polypeptide that is correlated with the occurrence of age related macular degeneration in humans; and (b) determining whether binding occurs. The occurrence of binding indicates that a variant CFH polypeptide that is correlated with the occurrence of age related macular degeneration is present in the sample.

The disclosure also provides diagnostic kits useful for detecting a variant CFH gene in a sample from an individual. A diagnostic kit may comprise, for example: (a) at least one container means having disposed therein a polynucleotide probe that hybridizes, under stringent conditions, to a variation in the CFH gene that is correlated with the occurrence of AMD in humans; and (b) a label and/or instructions for the use of the diagnostic kit in the detection of a variant CFH gene in a sample.

In another embodiment, a diagnostic kit useful for detecting a variant CFH gene in a sample from an individual may comprise, for example: (a) at least one container means having disposed therein a polynucleotide primer that hybridizes, under stringent conditions, adjacent to one side of a variation in the CFH gene that is correlated with the occurrence of age related macular degeneration in humans; and (b) a label and/or instructions for the use of the diagnostic kit in the detection of CFH in a sample. Optionally, the diagnostic kit additionally comprises a second polynucleotide primer that hybridizes, under stringent conditions, to the other side of the variation in the CFH gene that is correlated with the occurrence of age related macular degeneration in humans.

The disclosure also relates to compositions for treating a subject suffering from AMD. In a particular embodiment, a composition for treating a subject suffering from AMD comprises an effective amount of an isolated or recombinantly produced CFH polypeptide, or a fragment thereof, and a pharmaceutically acceptable carrier. In a particular embodiment, the CFH polypeptide, or the fragment thereof, inhibits the activation of C3. In another embodiment, the invention provides a method of treating a subject suffering from AMD, comprising administering to the subject an effective amount of an isolated or recombinantly produced CFH polypeptide, or a fragment thereof, and a pharmaceutically acceptable carrier.

The disclosure also provides a composition for treating a subject suffering from AMD, comprising an effective amount of an isolated or recombinantly produced nucleic acid molecule coding for a CFH polypeptide, or a fragment thereof, and a pharmaceutically acceptable carrier. As used herein, the term "effective amount" refers to the amount of an isolated or recombinantly produced CFH nucleic acid or polypeptide, or a composition comprising a CFH nucleic acid or polypeptide, that is in sufficient quantities to treat a subject or to treat the disorder itself. For example, an effective amount is sufficient to delay, slow, or prevent the onset or progression of AMD or related symptoms. The disclosure provides a method of treating a subject suffering from AMD, comprising administering to the subject an effective amount of an isolated or recombinantly produced nucleic acid molecule coding for a CFH polypeptide, or a fragment thereof, and a pharmaceutically acceptable carrier.

The disclosure also provides a composition for treating a subject suffering from or at risk for age related macular degeneration, comprising: (a) a nucleic acid molecule comprising an antisense sequence that hybridizes to a variant CFH gene or mRNA that is correlated with the occurrence of age related macular degeneration in humans; and (b) a pharmaceutically acceptable carrier. In certain embodiments, hybridization of the antisense sequence to the variant CFH gene reduces the amount of RNA transcribed from the variant CFH gene. In certain other embodiments, hybridization of the antisense sequence to the variant CFH mRNA reduces the amount of protein translated from the variant CFH mRNA, and/or alters the splicing of the variant CFH mRNA. A nucleic acid molecule comprising an antisense sequence that hybridizes to a variant CFH gene or mRNA may comprise one or more modified nucleotides or nucleosides that enhance in vivo stability, transport across the cell membrane, or hybridization to a variant CFH gene or mRNA. In other embodiments, the invention provides a method for treating a subject suffering from or at risk for age related macular degeneration, comprising administering to the subject an effective amount of a nucleic acid molecule comprising an antisense sequence that hybridizes to a variant CFH gene or mRNA that is correlated with the occurrence of age related macular degeneration in humans, and a pharmaceutically acceptable carrier.

The disclosure also provides a composition for treating a subject suffering from or at risk for age related macular degeneration, comprising: (a) a nucleic acid molecule comprising a siRNA or miRNA sequence, or a precursor thereof, that hybridizes to a variant CFH gene or mRNA that is correlated with the occurrence of age related macular degeneration in humans; and (b) a pharmaceutically acceptable carrier. In certain embodiments, hybridization of a nucleic acid molecule comprising a siRNA or miRNA sequence, or a precursor thereof to the variant CFH gene reduces the amount of RNA transcribed from the variant CFH gene. In other embodiments, hybridization of a nucleic acid molecule comprising a siRNA or miRNA sequence, or a precursor thereof to the variant CFH mRNA reduces the amount of protein translated from the variant CFH mRNA, and/or alters the splicing of the variant CFH mRNA. A nucleic acid molecule comprising an antisense sequence that hybridizes to a variant CFH gene or mRNA may comprise one or more modified nucleotides or nucleosides that enhance in vivo stability, transport across the cell membrane, or hybridization to a variant CFH gene or mRNA. In other embodiments, the invention provides a method for treating a subject suffering from or at risk for age related macular degeneration, comprising administering to the subject an effective amount of a nucleic acid molecule comprising a siRNA or miRNA sequence, or a precursor thereof, that hybridizes to a variant CFH gene or mRNA that is correlated with the occurrence of age related macular degeneration in humans and a pharmaceutically acceptable carrier

The disclosure also provides a composition for treating a subject suffering from or at risk for age related macular degeneration, comprising: (a) an aptamer that binds to a variant CFH polypeptide that is correlated with the occurrence of age related macular degeneration in humans; and (b) a pharmaceutically acceptable carrier, wherein binding of the aptamer to the variant CFH polypeptide reduces the activity of the variant CFH polypeptide. In other embodiments, the invention provides a method for treating a subject suffering from or at risk for age related macular degeneration, comprising administering to the subject an effective amount of an aptamer that binds to a variant CFH polypeptide that is correlated with the occurrence of age related macular degeneration in humans and a pharmaceutically acceptable carrier.

The disclosure also provides a composition for treating a subject suffering from or at risk for age related macular degeneration, comprising: (a) a small molecule that binds to a variant CFH polypeptide that is correlated with the occurrence of age related macular degeneration in humans; and (b) a pharmaceutically acceptable carrier. In certain embodiments, binding of the small molecule to the variant CFH polypeptide reduces the activity of the variant CFH polypeptide. In another embodiment, the invention provides a method for treating a subject suffering from or at risk for age related macular degeneration, comprising administering to the subject an effective amount of a small molecule that binds to a variant CFH polypeptide that is correlated with the occurrence of age related macular degeneration in humans and a pharmaceutically acceptable carrier.

The disclosure also provides a composition for treating a subject suffering from or at risk for age related macular degeneration, comprising: (a) an antibody that binds to a variant CFH polypeptide that is correlated with the occurrence of age related macular degeneration in humans; and (b) a pharmaceutically acceptable carrier. In certain embodiments, binding of the antibody to the variant CFH polypeptide reduces the activity of the variant CFH polypeptide. In another embodiment, the invention also provides a method for treating a subject suffering from or at risk for age related macular degeneration, comprising administering to the subject an effective amount of an antibody that binds to a variant CFH polypeptide that is correlated with the occurrence of age related macular degeneration in humans and a pharmaceutically acceptable carrier.

The methods and compositions described herein for treating a subject suffering from AMD may be used for the prophylactic treatment of individuals who have been diagnosed or predicted to be at risk for developing AMD. For instance, the composition is administered in an amount and dose that is sufficient to delay, slow, or prevent the onset of AMD or related symptoms. Alternatively, the methods and compositions described herein may be used for the therapeutic treatment of individuals who suffer from AMD. For example, the composition is administered in an amount and dose that is sufficient to delay or slow the progression of the condition, totally or partially, or in an amount and dose that is sufficient to reverse the condition.

As described herein for CFH, variations in CFH-like genes in humans (e.g., CFHL1, CFHL3, and CFHL4) are also useful for identifying or aiding in identifying individuals at risk for developing AMD. Variations in CFHL1, CFHL3, and CFHL4 may also be useful for diagnosing or aiding in the diagnosis of AMD, identifying or aiding in identifying individuals at risk for developing AMD, methods for diagnosing or aiding in the diagnosis of AMD, polynucleotides (e.g., probes, primers) useful in the methods, diagnostic kits containing probes or primers, methods of treating an individual at risk for or suffering from AMD and compositions useful for treating an individual at risk for or suffering from AMD. Examples of variations in CFHL1, CFHL3, and CFHL4 that may be correlated with the occurrence of AMD are found in Tables 8-10. Such variations, which can be in a coding or noncoding region of a CFHL gene (e.g., CFHL1, CFHL3, and CFHL4) can be useful in the methods and compositions described herein.

The disclosure also provides polynucleotides useful for the detection or aiding in the detection of a CFHL gene (e.g., CFHL1, CFHL3, or CFHL4) that is correlated with the occurrence of AMD in humans and, in specific embodiments, variations in a CFHL gene that are correlated with AMD in humans. The disclosure also provides diagnostic kits for detecting a variant CFHL gene in a sample from an individual. Such kits are useful in identifying or aiding in identifying individuals at risk for developing AMD, as well as for diagnosing or aiding in the diagnosis of AMD in an individual.

The disclosure also provides an isolated polynucleotide for the detection of a variant CFHL gene, such as CFHL1, CFHL3, or CFHL4, in a sample from an individual, comprising a nucleic acid molecule that specifically detects a variation in the CFHL gene that is correlated with the occurrence of age related macular degeneration in humans.

The disclosure also provides a polynucleotide primer that hybridizes, under stringent conditions, adjacent to a variation in a CFHL gene that is correlated with the occurrence of age related macular degeneration in humans. In certain embodiments, the invention provides a pair of polynucleotide primers that specifically detect a variation in a CFHL gene that is correlated with the occurrence of age related macular degeneration in humans, wherein the first polynucleotide primer hybridizes to one side of the variation and the second polynucleotide primer hybridizes to the other side of the variation. The pair of polynucleotide primers may hybridize to a region of a CFHL gene in such a manner that the ends of the hybridized primers proximal to the variation are from about 100 to about 10,000 nucleotides apart.

The present disclosure also relates to a method of detecting, in a sample obtained from an individual, a variant CFHL gene that is correlated with the occurrence of AMD in humans. Such a method may comprise: (a) combining the sample with a polynucleotide probe that hybridizes, under stringent conditions, to a variation in the CFHL gene that is correlated with AMD in humans, but not to a wildtype CFHL gene; and (b) determining whether hybridization occurs. The occurrence of hybridization indicates that a variant CFHL gene that is correlated with age related macular degeneration is present in the sample. A used herein, the term "wildtype CFHL gene" refers to a CFHL gene, such as CFHL1, CFHL3, or CFHL4, that is not correlated with the occurrence of AMD.

The disclosure also provides a method of detecting, in a sample obtained from an individual, a variant CFHL gene that is correlated with the occurrence of age related macular degeneration in humans, comprising: (a) combining the sample (referred to as a test sample) with a polynucleotide probe that hybridizes, under stringent conditions, to a variation in the CFHL gene that is correlated with the occurrence of AMD in humans, thereby producing a combination; (b) maintaining the combination produced in step (a) under stringent hybridization conditions; and (c) comparing hybridization that occurs in the combination with hybridization in a control. The occurrence of hybridization in the combination but not in the control indicates that a variant CFHL gene that correlates with AMD is present in the sample. In a further embodiment, the extent of hybridization is determined when comparing hybridization that occurs in the combination with hybridization in a control. The control is the same as the test sample and is treated the same as the test sample except that the polynucleotide probe is one that does not bind to a variation in the CFHL gene that is correlated with the occurrence of AMD in humans. Alternatively, the polynucleotide probe is one that binds only to a wildtype CFHL gene.

The disclosure also provides a method of detecting, in a sample obtained from an individual, a variant CFHL gene that is correlated with the occurrence of AMD in humans, comprising: (a) combining a first portion of the sample with a polynucleotide probe that hybridizes, under stringent conditions, to a variation in the CFHL gene that is correlated with the occurrence of AMD in humans; (b) combining a second portion of the sample with a polynucleotide probe that hybridizes, under stringent conditions, to a wildtype CFHL gene; and (c) determining whether hybridization occurs. The occurrence of hybridization in the first portion, but not in the second portion, indicates that a variant CFHL gene that is correlated with AMD is present in the sample.

The disclosure provides methods of identifying or aiding in identifying an individual at risk for developing AMD. In one specific embodiment, such a method comprises assaying DNA obtained from the individual for the presence of a variant CFHL gene that is correlated with the occurrence of AMD in humans. The presence of a variant CFHL gene indicates that the individual is at risk for developing AMD.

In another embodiment, a method of identifying or aiding in identifying an individual at risk for developing AMD comprises: (a) combining a sample obtained from the individual with a polynucleotide probe that hybridizes, under stringent conditions, to a variation in the CFHL gene that is correlated with AMD in humans, but does not hybridize to a wildtype CFHL gene; and (b) determining whether hybridization occurs. The occurrence of hybridization indicates that the individual is at risk for developing AMD.

### BRIEF DESCRIPTION OF THE DRAWINGS

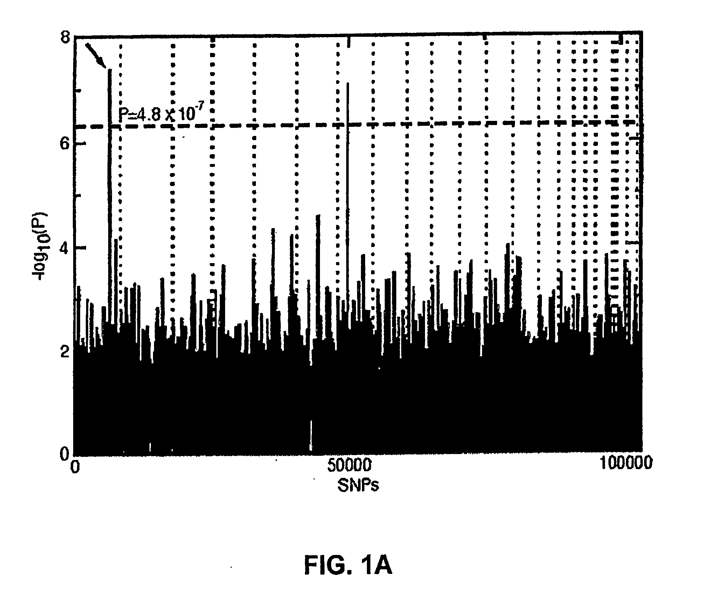

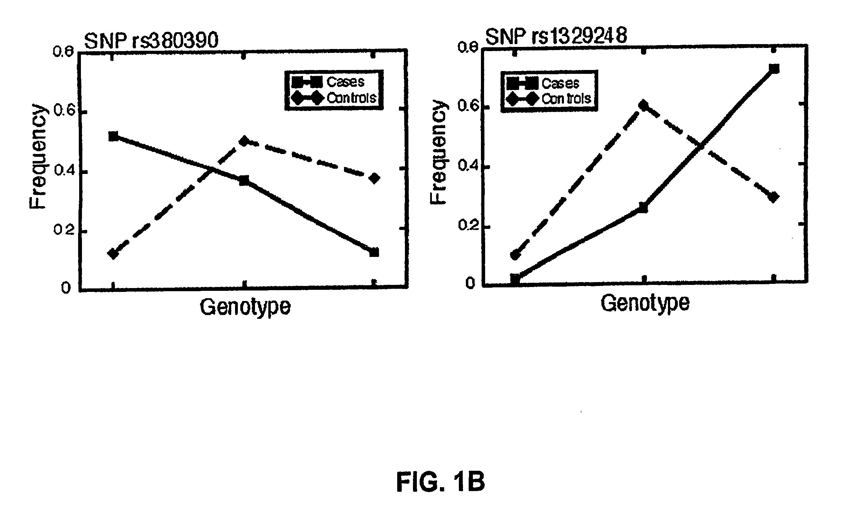

Figure 1A-1B are graphs showing statistical data of a genome-wide association study of genes associated with AMD. Figure 1A shows p-values of the genome-wide association scan.
   -log₁₀(p) is plotted for each SNP in chromosomal order. The spacing between SNPs on the plot is uniform and does not reflet distances between SNPs on the chromosomes, The dotted horizontal line shows the cutoff for p=0.05 after Honferroni correction. The vertical lines show chromosomal boundaries. Figure 1B shows variations in genotype frequencies between cases and controls.

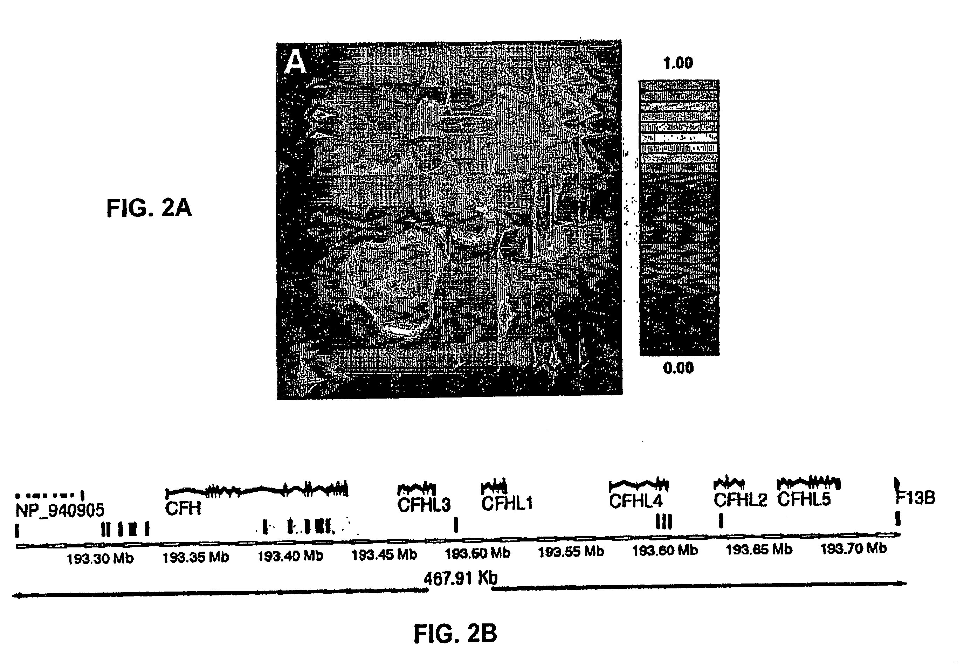

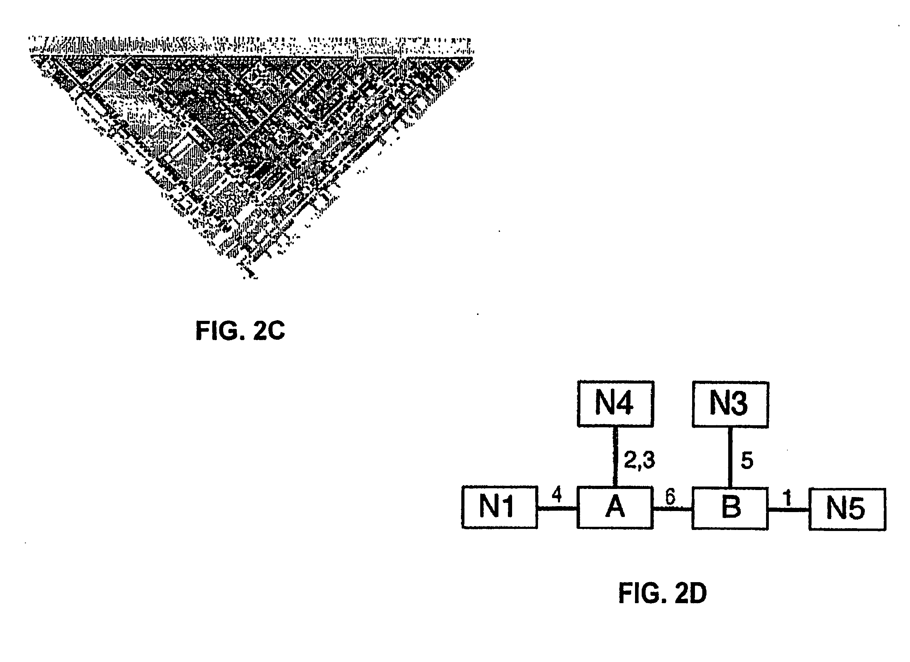

Figures 2A-2D show data on SNPs that are associated with AMD. Figure 2A shows linkage disequilibrium (LD) across the CFH region, plotted as pairwise D' values. Figure 2B shows a schematic of the region in strong LD with the two associated SNPs in the data. The vertical bars represent the approximate location of the SNPs available in the data set. The shaded region is the haplotype block found in the HapMap data. Figure 2C shows haplotype blocks in the HapMap CEU data cross the region. Darker shades indicate higher values of D'. Lighter shades indicate high D' with a low LOD score. The dark lines show the boundaries of haplotype blocks. Figure 2D shows a maximum parsimony cladogram derived from haplotypes across the 6-SNP region. The number by each line indicates which of the six SNPs varies along the branch. SNP 4 is rs380390 and SNP 6 is rs1329428, which are the two SNPs initially identified as associated with AMD.

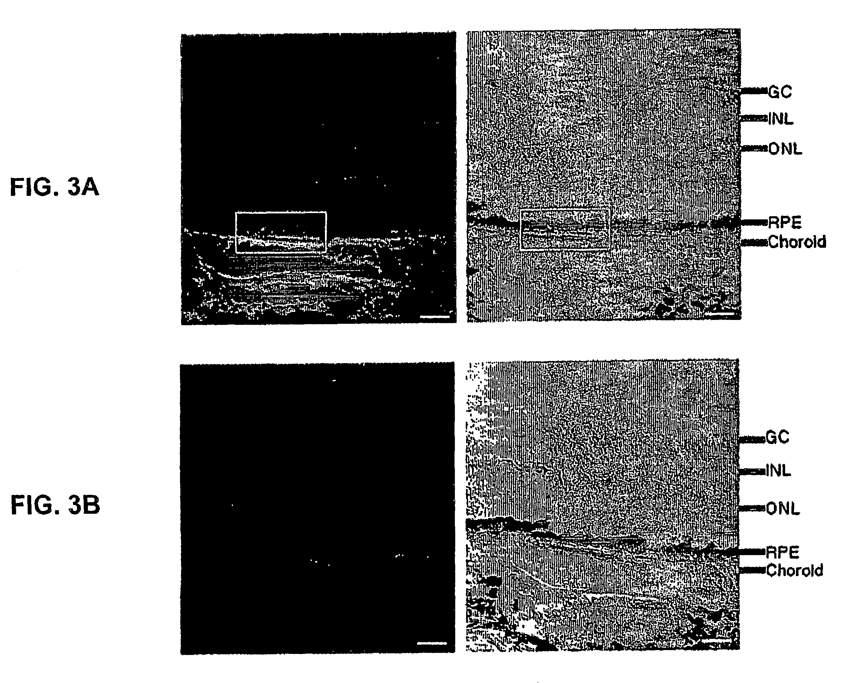

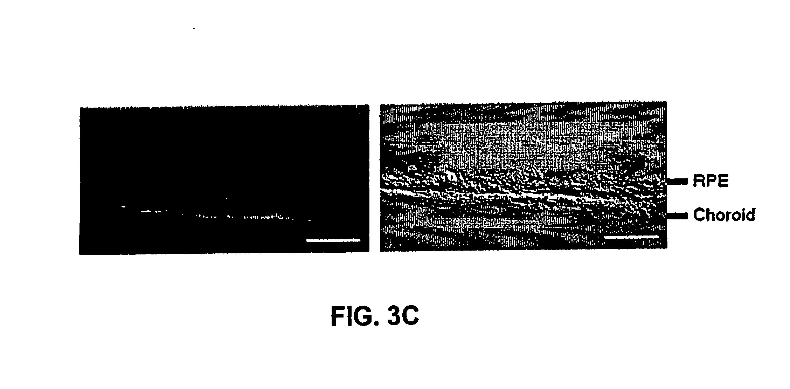

Figures 3A-3C show immunofluorescent localization of CFH protein in human retina. Figure 3A shows human retina sections stained with anti-human CFH antibody. Figure 3B shows human retina sections stained with anti-human CFH antibody preincubated with CFH protein as negative control. The nuclei are identified by DAPI staining. The magnified view of the boxed area in Figure 3A is shown in Figure 3C. The fluorescent and DIC channels are collected from each image and presented as the left and right pictures, respectively, in each panel. The fluorescent pictures in Figure 3A and Figure 3B are merged images from CFH labeling and DAPI stained nuclei. The DIC picture in Figure 3C is a merged image of CFH labeling and the DIC channel. The black spots in DIC images correspond to melanin granules in RPE and choroids. The anti-CFH antibody primarily stains the choroids (Figure 3A), especially strong in the wall of vessels lumen and in area close to RPE (Figure 3C), and the immunoreactivity can be competed away with purified human CFH protein (Figure 3B). The fluorescent signal from RPE arises from the autofluorescence of lipofusion which cannot be competed away by human factor H protein. GC: ganglion cells layer, INL: inner nuclear layer, ONL: outer nuclear layer, RPE: retinal pigment epithelium. Scale bar: 40 µm in Figures 3A and 3B, 20 µm in Figure 3C.

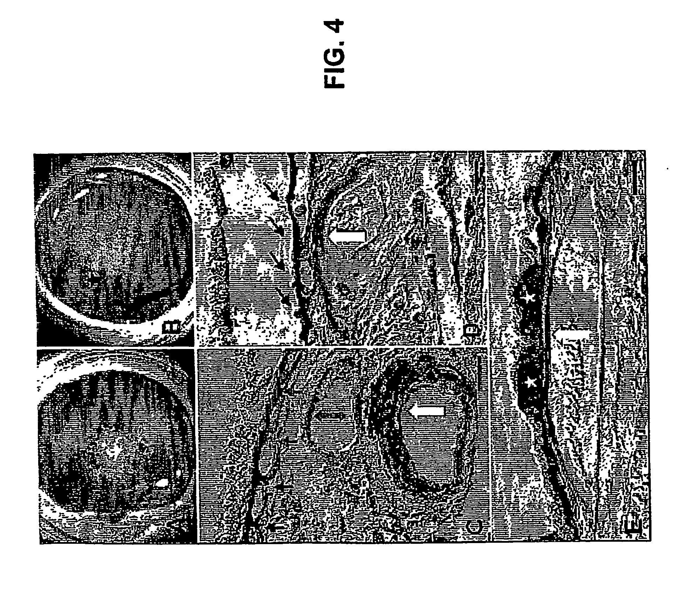

Figure 4A-4E show immunohistochemistry for activated complement C5b-9. Tissues from three patients are illustrated. Figures 4A and 4B show post-mortem fundus images from patients 1 and 2, respectively. The site illustrated histologically is indicated with an asterisk. Figure 4C shows tissue from patient 1 who is immunopositive for C5b-9 throughout Bruch's membrane and in intercapillary pillars (thin black arrows). Overlying retinal pigment epithelium is hypertrophic, and associated retina demonstrated market photoreceptor loss. Complement deposition is also present within the elastica of a choroidal artery (double headed black arrow), as well as within the walls of a choroidal vein (white arrow). Figure 4D shows C5b-9 deposition in Bruch's membrane, intercapillary pillars (arrows) and drusen (asterisk) in patient 2. The internal aspect of a choroidal vein is also immunopositive (white arrow). Figure 4E shows tissue from patient 3, an 86-year old with histologic evidence of early AMD. Activated complement deposition is noted throughout Bruch's membrane, in drusen (asterisks) and in the internal wall of a choroidal vein (white arrow). Scale bar: 20 µm in Figures 4C and 4D), 15 µm in Figure 4E.

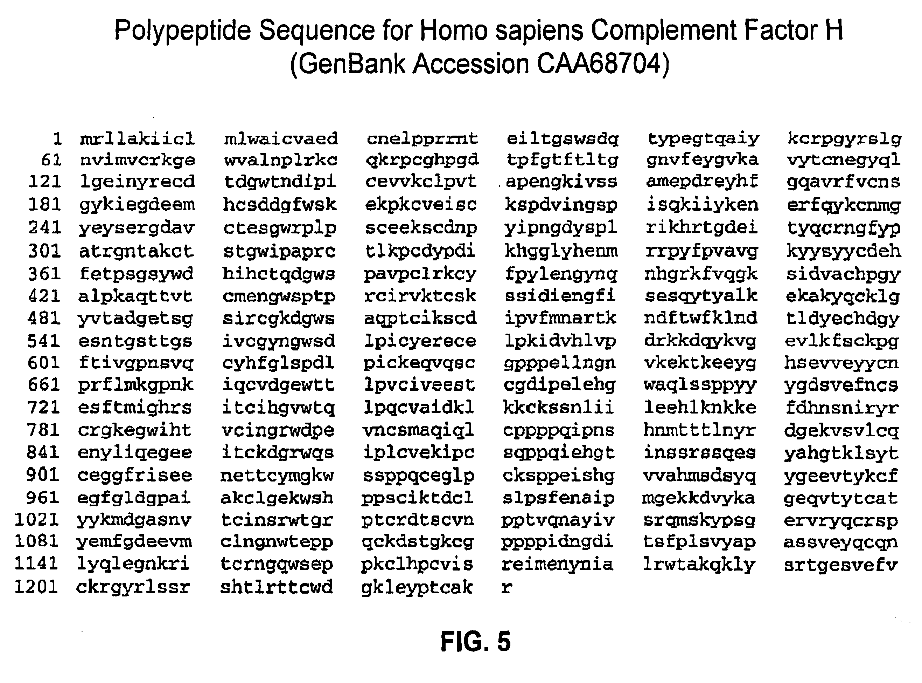

Figure 5 shows the polypeptide sequence for human Complement Factor H (GenBank Accession CAA68704).

### DETAILED DESCRIPTION OF THE INVENTION

To provide an overall understanding of the invention, certain illustrative embodiments will now be described.

### 1. Overview

The discovery that variations in the CFH gene are associated with AMD is useful for the early diagnosis and treatment of individuals predisposed to AMD. The determination of the genetic constitution of the CFH gene in an individual is useful in heating AMD at earlier stages, or even before an individual displays any symptoms of AMD. Furthermore, diagnostic tests to genotype CFH may allow individuals to alter their behavior to minimize environmental risks to AMD (e.g., smoking). Accordingly, the present invention relates to the identification of a variant CFH gene correlated with a predisposition to AMD. which is useful in identifying or aiding in identifying individuals at risk for developing AMI), as well as for diagnosing or aiding in the diagnosis of AMD. It also relates to methods for identifying or aiding in identifying individuals at risk for developing AMD, methods for diagnosing or aiding in the diagnosis of AMD, polynucleotides (e.g., probes, primers) useful in the methods, diagnostic kits containing probes or primers, methods of treating an individual at risk for or suffering from AMD and compositions useful for treating an individual at risk for or suffering from AMD.

In accordance with the present invention, a common variation in the CFH gene has been shown to be strongly associated with AMD. The present invention relates to methods and compositions for detecting such variations that predispose a human to AMD. A CFH gene can either be the cDNA or the genomic form of the gene, which may include upstream and downstream regulatory sequences. The CFH polypeptide can be encoded by a full length coding sequence or by any portion of the coding sequence so long as the desired activity or functional properties (e.g., enzymatic activity, ligand binding, signal transduction, etc.) of the full-length or fragment are retained. Examples of CFH nucleotide sequences include human nucleotide sequences (SEQ ID NOs: 1 or 2), a mouse nucleotide sequence (SEQ ID NO: 3), and a rat nucleotide sequence (SEQ ID NO: 4). Polynucleotide probes and primers of the invention may hybridize to any contiguous portion of a CFH gene, such as those shown in SEQ ID NOs 1-4. Examples of CFH polypeptide sequences include human polypeptide sequences (SEQ ID NOs: 5 or 6 and Figure 5), a mouse polypeptide sequence (SEQ ID NO: 7), and a rat polypeptide sequence (SEQ ID NO: 8). The CFH gene may further include sequences located adjacent to the coding region on both the 5' and 3' ends for a distance of about 1-2 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences.

The CFH gene is a member of the Regulator of Complement Activation (RCA) gene cluster and encodes a protein with twenty short consensus repeat (SCR) domains of 60 amino acids each. This protein is secreted into the bloodstream and has an essential role in the regulation of complement activation (Rodriguez de Cordoba et al., Mol Immunol. 41:355-67 (2004)). The complement system protects against infection and attacks diseased and dysplastic cells and normally spares healthy cells. Cells involved in immune surveillance and response to disease are recruited to augment the lytic action of activated complement components. When C3 convertase is activated, it leads to the production of C3a and C3b and then to the terminal C5b-9 complex. CFH on cells and in circulation regulates complement activity by inhibiting the activation of C3 to C3a and C3b, and by inactivating existing C3b. Variations in the CFH gone have previously been associated with homolytic-uremic syndrome (HUS) and chronic bypocomplementemic nephropathy. Alternate transcriptional splice variants, encoding different isoforms, have been characterized.

### 2. CFH polynucleotide probes and primers

Described herein are isolated and/or recombinant polynucleotides that specifically detect a variation in the CFH gone that is correlated with the occurrence of AMD. Polynucleotide probes hybridize to a variation (referred to as a variation of interest) in such a CFH gene, and the flanking sequence, in a specific manner and thus typically have a sequence which is fully or partially complementary to the sequence of the variation and the flanking region. Polynucleotide probes may hybridize to a segment of target DNA such that the variation aligns with a central position of the probe, or the variation may align with a terminal position of the probe. An isolated polynucleotide probe may hybridize, under stringent conditions, to a nucleic acid molecule comprising a variant CFH gene, or a portion or allelic variant thereof, that is correlated with the occurrence of AMD in humans, or to a nucleic acid molecule comprising at least 10 contiguous nucleotides of a CFH gene, or an allelic variant thereof, wherein the nucleic acid molecule comprises a variation that is correlated with the occurrence of AMD in humans.

The design and use of allole-specific probes for analyzing polymorphisms is described by e.g., Saiki et al., Nature 324: 163-166 (1986); Dattagupta, EP 235726; and Saiki WO 89/11548. Allele-apecific probes can be designed to hybridize to a segment of a target DNA from one individual but do not hybridize to the corresponding segment from another individual due to the presence of different polymorphic forms or variations in the respective segment from the two individuals, Hybridization conditions should be sufficiently stringent such that there is a significant difference in hybridization intensity between alleles. A probe may hybridize to only one of the alleles.

It is well known in the art how to perform hybridization experiments with nucleic acid molecules. The skilled artisan is familiar with the hybridization conditions required in the present invention and understands readily that appropriate stringency conditions which promote DNA hybridization can be varied. Such hybridization conditions are referred to in standard text books, such as Molecular Cloning: A laboratory Manual, Cold Spring Harbor Laboratory (2001); and Current Protocols in Molecular Biology, cds. Ausubel et al., John Wiley & Sons (1992). Particularly useful in methods of the present invention are polynucleotides which are capable of hybridizing to a variant CFH gene, or a region of a variant CFH gene, under stringent conditions. Under stringent conditions, a polynucleotide that hybridizes to a variant CFH gene does not hybridize to a wildtype CFH gene.

Nucleic acid hybridization is affected by such conditions as salt concentration, temperature, organic solvents, base composition, length of die complementary strands, and the number of nucleotide base mismatches between the hybridizing nucleic acids, as will readily be appreciated by those skilled in the art Stringent temperature conditions will generally include temperatures in excess of 30°C, or may be in excess of 37°C or 45°C. Stringency increases with temperature. For example, temperatures greater than 45°C are highly stringent conditions. Stringent salt conditions will ordinarily be less than 1000 mM, or may be less than 500 mM or 200 mM. For example, one could perform the Hybridization at 6.0x sodium chloride/sodium citrate (SSC) at about 45 °C, followed by a wash of 2.0x SSC at 50 °C. For example, the salt concentration in the wash step can be selected from a low stringency of about 2.0x SSC at 50 °C to a high stringency of about 0.2x SSC at 50 °C. In addition, tho temperature in the wash step can be increased from low stringency conditions at room temperature, about 22 °C, to high stringency conditions at about 65 °C. Both temperature and salt may be varied, or temperature or salt concentration may be held constant while the other variable is changed. Particularly useful in methods or the present invention are polynucleotides which are capable or hybridizing to a variant CFH gene, or a region of a variant CFH gene, under stringent conditions. It is understood, however, that the appropriate stringency conditions may be varied in the present invention to promote DNA hybridization. The combination of parameters, however, is much moro important than the measure of any single parameter. See, e.g., Wetmur and Davidson, 1968. Probe sequences may also hybridize specifically to duplex DHA under certain conditions to form triplex or higher order DNA complexes. The preparation of such probes and suitable hybridization conditions are well known in the art. One method for obtaining DNA encoding the biosynthetic constructs disclosed herein is by assembly of synthetic oligonucleotides produced in a conventional, automated, oligonucleotide synthesizer.

A polynucleotide probe or primer may be labeled so that it is detectable in a variety of detection systems, including, but not limited, to enzyme (e.g., ELISA, as well as enzyme-based histochemical assays), fluorescent, radioactive, chemical, and luminescent systems. A polynucleotide probe or primer may further include n quencher moiety that, when placed in proximity to a label (e.g., a fluorescent label), causes there to be little of no signal from the label. Detection of the label may be performed by direct or indirect means (e.g., via a biotin/avidin or a biotin/stretpavidin liukage). It is not intended that the present invention be limited to any particular detection system or label.

As used herein, the term "primer" refers to a polynucleotide that is capable of acting as a point of initiation of nucleic acid synthesis when placed under conditions in which synthesis of a primer extension product that is complementary to a nucleic acid strand occurs (for example, in the presence of nucleotides, an inducing agent such as DNA polymerase, and suitable temperature, pH, and electrolyte concentration). Alternatively, the primer may be capable of ligating to a proximal nucleic acid when placed under conditions in which ligation of two unlinked nucleic acids occurs (for example, in the presence of a proximal nucleic acid, an inducing agent such as DNA ligase, and suitable temperature, pH, and electrolyte concentration). A polynucleotide primer may be naturally occurring, as in a purified restriction digest, or may be produced synthetically. The primer is preferably single stranded for maximum efficiency in amplification, but may alternatively be double stranded. If double stranded, the primer is first treated to separate its strands before being used. Preferably, the primer is an oligodeoxyribonucleotide. The exact lengths of the primers will depend on many factors, including temperature, source of primer and the use of the method. In certain embodiments, the polynucleotide primer of the invention is at least 10 nucleotides long and hybridizes to one side or another of a variation in the CFH gene that is correlated with the occurrence of AMD in humans. The subject polynucleotides may contain alterations, such as one or more nucleotide substitutions, additions or deletions, provided they hybridize to their target variant CFH gene with the same degree of specificity.

### 3. Detection assays

The invention may be used in any assay that permits detection of a variation in the CFH gone that is correlated with the occurrence of AMD. Such methods may encompass, for example, DNA sequencing, hybridization, ligation, or primer extension methods. Furthermore, any combination of these methods may be utilized in the invention.

In one embodiment, the presence of a variation in the CFH gene that is correlated with the occurrence of AMD is detected and/or determined by DNA sequencing. DNA sequence determination may be performed by standard methods such as dideoxy chain termination technology and gel-electrophoresis, or by other methods such as by pyrosequencing (Biotage AB, Uppsala, Sweden). For example, DNA sequencing by dideoxy chain termination may be performed using unlabeled primers and labeled (e.g., fluorescent or radioactive) terminators. Alternatively, sequencing may be performed using labeled primers and unlabeled terminators. The nucleic acid sequence of the DNA In the sample can be compared to the nucleic acid sequence of wildtype DNA to identify whether a variation in the CPH gene that is correlated with the occurrence of AMD is present.

The presence of a variation in the CFH gene that is correlated with the occurrence of AMD may be detected and/or determined by hybridization. A polynucleotide probe may be hybridized to a variation in the CFH gene, and flanking nucleotides, that is correlated with AMD, but not to a wildtype CFH gene. The polynucleotide probe may comprise nucleotides that are fluorescently, radioactively, or chemically labeled to facilitate detection of hybridization. Hybridization may be performed and detected by standard methods known in the art, such as by Northern blotting, Southern blotting, fluorescent in situ hybridization (FISH), or by Hybridization to polynucleotides immobilized on a solid support, such as a DNA array or microarray. As used herein, the term "DNA array," and "microarray" refers to an ordered arrangement of hybridizable array elements. The array elements are arranged so that there are preferably at least oue or more different array elements immobilized on a substrate surface. The hybridization signal from each of the array clements is individually distinguishable. In a The array elements may comprise polynucleotides, although the present invention could also be used with cDNA or other types of nucleic acid array elements,

The polynucleotide probe may be used to hybridize genomic DNA by FISII. FISH can be used, for examples, in metaphase cells, to detect a deletion in genomic DNA. Genomic DNA is denatured to separate the complimentary strands within the DNA double helix structure. The polynucleotide probe is then added to the denatured genomic DNA. If a variation in the CFH gene that is correlated with the occurrence of AMD is present, the probe will hybridize to the genomic DNA. The probe signal (e.g., fluorescence) can then be detected through a fluorescent microscope for the presence of absence of signal. The absence of signal, therefore, indicates the absence of a variation in the CFH gene that is correlated with the occurrence of AMD. A labeled polynucleotide probe may be applied to immobilized polynucleotides on a DNA array. Hybridization may be detected, for example, by measuring the intensity of the labeled probe remaining on the DNA array after washing. The polynucleotides may also be used in commercial assays, such as the Taqman assay (Applied Biosystems, Foster City. CA).

The presence of a variation in the CFH gene that is correlated with the occurrence of AMD may be detected and/or determined by primer extension with DNA polymerase. Apolynucleotide primer may be hybridized immediately adjacent to the variation. A single base sequencing reaction using labeled dideoxynucleotide terminators may be used to detect the variation. The presence of a variation will result in the incorporation of the labeled teminator, whereas the absence of a variation will not result in the incorporation of the terminator. A polynucleotide primer may be hybridized to a variation in the CFH gene that is correlated with the occurrence of AMD. The primer, or a portion thereof will not hybridize to a wildtype CFH gene. The presence of a variation will result in primer extension, whereas the absence of a variation will not result in primer extension. The primers and/or nucleotides may further include fluorescent, radioactive, or chemical probes. A primer labeled by primer extension may be detected by measuring the intensity of the extension product, such as by gel electrophoresis, mass spectrometry, or any other method for detecting fluorescent, radioactive, or chemical labels.

The presence of a variation in the CFH gene that is corrected with the occurrence of AMD may be detected and/or determined by ligation. A polynucleotide primer of the invention may be hybridized to a variation in the CFH gene that is correlated with the occurrence of AMD. The primer, or a portion thereof will not hybridize to a wildtype CFH gene. A second polynucleotide that hybridizes to a region of the CFH gene immediately adjacent to the first primer is also provided. One, or both, of the polynucleotide primers may be fluorescently, radioactively, or chemically labeled. Ligation of the two polynucleotide primers will occur in the presence of DNA ligase if a variation in the CFH gene that is correlated with the occurrence of AMD is present. Ligation may be detected by gel electrophoresis, mass spectrometry, or by measuring the intensity of fluorescent, radioactive, or chemical labels.

The presence of a variation in the CFH gene that is correlated with the occurrence of AMD may be detected and/or determined by single-base extension (SBE). For example, a fluorescently-labeted primer that is coupled with fluorescence resonance energy transfer (FRET) between the label of the added base and the label of the primer may be used. Typically, the method, sach as that described by Chen et al., (PNAS 94:10756-61 (1997), uses a locus-specific polynucleotide primer labeled on the 5' terminus with 5-carboxyfluorescein (FAM). This labeled primer is designed so that the 3' end is immediately adjacent to the polymorphic site of interest. The labeled primer is hybridized to the locus, and single base extension of the labeled primer is performed with fluorescently labeled dideoxyribonucleotides (ddNTPs) in dye-terminator sequencing fashion, except that no deoxyribonucleotides are present. An increases in fluorescence of the added ddNTP in response to excitation at the wavelength of the labeled primer is used to infer the identity of the added nucleotide.

Methods of detecting a variation in the CFH gene that is correlated with the occurrence of AMD may include amplification of a region of DNA that comprises the variation. Any method of amplification may be used. A region of DNA comprising the variation may be amplified by using polymerase chain reaction (PCR). PCR was initially described by Mullis (See e.g., U.S. Pat. Nos. 4,683,195 4,683,202, and 4,965,188), which describes a method for increasing the concentration of a region of DNA, in a mixture of genomic DNA, without cloning or purification. Other PCR methods may also be used to nucleic acid amplification, including but not limited to RT-PCR, quantitative PCR, real time PCR, Rapid Amplified Polymorphic DNA Analysis, Rapid Amplification of cDNA Ends (RACB), or rolling circle amplification. For example, the polynucleotide primers may be combined with a DNA mixture (or any polynucleotide sequence that can be amplified with the polynucleotide primers of the invention), wherein the DNA comprises the CFH gene. The mixture also includes the necessary amplification reagents (o.g., deoxyribonucleotide triphosphates, buffer, etc.) necessary for the thermal cycling reaction. According to standard PCR methods, the mixture undergous a series of denaturation, primer annealing, and polymerase extension steps to amplify the region of DNA that comprises the variation in the CFH gene. The length of the amplified region of DNA is determined by the relative positions of the primers with respect to each other, and therefore, this length is a controllable parameter. For example, hybridization of the primers may occur such that the ends of the primers proximal to the variation are separated by 1 to 10,000 base pairs (e.g., 10 base pairs (bp) 50 bp, 200 bp, 500 bp, 1,000 bp, 2,500 bp, 5,000 bp, or 10,000 bp).

Standard instrumentation known to those skilled in the art are used for the amplification and detection of amplified DNA. For example, a wide variety of instrumentation has been developed for carrying out nucleic acid amplifications, particularly PCR, e.g. Johnson et al, U.S. Pat. No. 5,038,852 (computer-controlled thermal cycler); Wittwer et al, Nucleic Acids Research, 17: 4353-4357 (1989)(capillary tube PCR); Hallsby, U.S. Pat. No. 5,187,084 (air-based temperature control); Garner et al, Biotechniques, 14: 112-115 (1993)(high-throughput PCR in 864-well plates); Wilding et al, International application No. PCT/US93/04039 (PCR in micro-machined structures); Schnipelsky et al, European patent application No. 90301061.9 (publ. No. 0381501 A2)(disposable, single use PCR device), and the like. In certain embodiments, the invention described herein utilizes real-time PCR or other methods known in the art such as the Taqman assay.

In certain embodiments, a variant CFH gene that is correlated with the occurrence of AMD in humans may be detected using single-strand conformation polymorphism analysis, which identifies base differences by alteration in electrophoretic migration of single stranded PCR products, as described in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence differences between alleles of target sequences.

In one embodiment, the amplified DNA is analyzed in conjunction with one of the detection methods described herein, such as by DNA sequencing. The amplified DNA may alternatively be analyzed by hybridization with a labeled probe, hybridization to a DNA array or microarray, by incorporation of biotinylated primers followed by avidin-enzyme conjugate detection, or by incorporation of ³²P-labeled deoxynucleotide triphosphates, such as dCTP or dATP, into the amplified segment. In a specific embodiment, the amplified DNA is analyzed by determining the length of the amplified DNA by electrophoresis or chromatography. For example, the amplified DNA is analyzed by gel electrophoresis. Methods of gel electrophoresis are well known in the art See for example, Current Protoco/s in Molecular Biology, eds. Ausubel et al.. John Wiley & Sons: 1992. The amplified DNA can be visualized, for exemple, by fluorescent or radioactive means, or with other dyes or markers that intercalate DNA. The DNA may also be transferred to a solid support such as a nitrocellulose membrane and subjected to Southern Blotting following gel electrophoresis. In one embodiment, the DNA is exposed to ethidium bromide and visualized under ultra-violet light.

In other embodiments, nucleic acids of the disclosure also include nucleic acids that hybridize under stringent conditions to the nucleotide sequence designated in SEQ ID NO: 1 or 2, complement sequence of SEQ ID NO: 1 or 2, or fragments thereof. As discussed above, one of ordinary skill in the art will understand readily that appropriate stringency conditions which promote DNA hybridization can be varied. For example, one could perform the hybridization at 6.0 x sodium chloride/sodium citrate (SSC) at about 45 °C, followed by a wash of 2.0 x SSC at 50 °C. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In one embodiment, the invention provides nucleic acids which hybridize under low stringency conditions of 6 x SSC at room temperature followed by a wash at 2 x SSC at room temperature.

Isolated nucleic acids which differ from the nucleic acids as set forth in SEQ ID NO: 1 or 2 due to degeneracy in the genetic code are also within the scope of the invention. For example, a number of amino acids are designated by more than one triplet Codons that specify the same amino acid, or synonyms (for example, CAU and CAC are synonyms for histidine) may result in "silent" variations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this invention.

The nucleic acids and polypeptides of the disclosure may be produced using standard recombinant methods. For example, the recombinant nucleic acids of the invention may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the invention. The promoters may be either naturally occurring promoters, or hybrid promoters that combine elements of more than one promoter. An expression construct may be present in a cell on an episome, such as a plasmid, or the expression construct may be inserted in a chromosome. The expression vector may also contain a selectable marker gene to allow the selection of transformed host cells. Selectable marker genes are well known in the art and will vary with the host cell used.

In certain aspects of the disclosure, the subject nucleic acid is provided in an expression vector comprising a nucleotide sequence encoding a CFH polypeptide and operably linked to at least one regulatory sequence. Regulatory sequences are art-recognized and are selected to direct expression of the CFH polypeptide. Accordingly, the term regulatory sequence includes promoters, enhancers, termination sequences, preferred ribosome binding site sequences, preferred mRNA leader sequences, preferred protein processing sequences, preferred signal sequences for protein secretion, and other expression control elements. Examples of regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding a CFH polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the disclosure can be produced by ligating the cloned gene, or a portion thereof, into a vector suitable for expression in either prokaryotic cells, eukaryotic cells (yeast, avian, insect or mammalian), or both. Expression vehicles for production of recombinant CFH polypeptides include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning: A Laboratory Manual, Cold Spring Harbor Laboratory (2001). In some instances, it may be desirable to express the recombinant polypeptide by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

In one embodiment, a vector will be designed for production of a subject CFH polypeptide in CHO cells, such as a Pcmv-Script vector (Stratagene, La Jolla, Calif.), pcDNA4 vectors (Invitrogen, Carlsbad, Calif.) and pCI-neo vectors (Promega, Madison, Wisc.). In other embodiments, the vector is designed for production of a subject CFH polypeptide in prokaryotic host cells (e.g., *E. coli* and *B. subtilis*), eukaryotic host cells such as, for example, yeast cells, insect cells, myeloma cells, fibroblast 3T3 cells, monkey kidney or COS cells, mink-lung epithelial cells, human foreskin fibroblast cells, human glioblastoma cells, and teratocarcinoma cells. Alternatively, the genes may be expressed in a cell-free system such as the rabbit reticulocyte lysate system.

As will be apparent, the subject gene constructs can be used to express the subject CFH polypeptide in cells propagated in culture, e.g., to produce proteins, including fusion proteins or variant proteins, for purification.

This disclosure also pertains to a host cell transfected with a recombinant gene including a coding sequence (e.g., SEQ ID NO: 1 or 2) for one or more of the subject CFH polypeptides. The host cell may be any prokaryotic or eukaryotic cell. For example, a CFH polypeptide of the invention may be expressed in bacterial cells such as *E. coli*, insect cells (e.g., using a baculovirus expression system), yeast, or mammalian cells. Other suitable host cells are known to those skilled in the art.

Accordingly, the present disclosure further pertains to methods of producing the subject CFH polypeptides. For example, a host cell transfected with an expression vector encoding a CFH polypeptide can be cultured under appropriate conditions to allow expression of the CFH polypeptide to occur. CFH polypeptides may be secreted and isolated from a mixture of cells and medium containing the CFH polypeptides. Alternatively, the polypeptide may be retained cytoplasmically or in a membrane fraction and the cells harvested, lysed and the protein isolated. A cell culture includes host cells, media and other byproducts. Suitable media for cell culture are well known in the art The polypeptide can be isolated from cell culture medium, host cells, or both using techniques known in the art for purifying proteins, including ion-exchange chromatography, gel filtration chromatography, ultrafiltration, electrophoresis, and immunoaffinity purification with antibodies specific for particular epitopes of the polypeptide. In a particular embodiment, the CFH polypeptide is a fusion protein containing a domain which facilitates the purification of the CFH polypeptide.

In another embodiment, a fusion gene coding for a purification leader sequence, such as a poly-(His)/enterokinase cleavage site sequence at the N-terminus of the desired portion of the recombinant CFH polypeptide, can allow purification of the expressed fusion protein by affinity chromatography using a Ni²⁺ metal resin. The purification leader sequence can then be subsequently removed by treatment with enterokinase to provide the purified polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

### 5. Other therapeutic modalities

### Antisense polynucleotides

In certain embodiments, the disclosure provides polynucleotides that comprise an antisense sequence that acts through an antisense mechanism for inhibiting expression of a variant CFH gene. Antisense technologies have been widely utilized to regulate gene expression (Buskirk et al., Chem Biol 11, 1157-63 (2004); and Weiss et al., Cell Mol Life Sci 55, 334-58 (1999)). As used herein, "antisense" technology refers to administration or in situ generation of molecules or their derivatives which specifically hybridize (e.g., bind) under cellular conditions, with the target nucleic acid of interest (mRNA and/or genomic DNA) encoding one or more of the target proteins so as to inhibit expression of that protein, e.g., by inhibiting transcription and/or translation, such as by steric hinderance, altering splicing, or inducing cleavage or other enzymatic inactivation of the transcript. The binding may be by conventional base pair complementarity, or, for example, in the case of binding to DNA duplexes, through specific interactions in the major groove of the double helix. In general, "antisense" technology refers to the range of techniques generally employed in the art, and includes any therapy that relies on specific binding to nucleic acid sequences.

A polynucleotide that comprises an antisense sequence of the present disclosure can be delivered, for example, as a component of an expression plasmid which, when transcribed in the cell, produces a nucleic acid sequence that is complementary to at least a unique portion of the target nucleic acid. Alternatively, the polynucleotide that comprises an antisense sequence can be generated outside of the target cell, and which, when introduced into the target cell causes inhibition of expression by hybridizing with the target nucleic acid. Polynucleotides of the invention may be modified so that they are resistant to endogenous nucleases, e.g. exonucleases and/or endonucleases, and are therefore stable in vivo. Examples of nucleic acid molecules for use in polynucleotides of the invention are phosphoramidate, phosphothioate and methylphosphonate analogs of DNA (see also U.S. Pat. Nos. 5,176,996; 5,264,564; and 5,256,775). General approaches to constructing polynucleotides useful in antisense technology have been reviewed, for example, by van der krol et al. (1988) Biotechniques 6:958-976; and Stein et al. (1988) Cancer Res 48:2659-2668.

Antisense approaches involve the design of polynucleotides (either DNA or RNA) that are complementary to a target nucleic acid encoding a variant CFH gene. The antisense polynucleotide may bind to an mRNA transcript and prevent translation of a protein of interest. Absolute complementarity, although preferred, is not required. In the case of double-stranded antisense polynucleotides, a single strand of the duplex DNA may thus be tested, or triplex formation may be assayed. The ability to hybridize will depend on both the degree of complementarity and the length of the antisense sequence. Generally, the longer the hybridizing nucleic acid, the more base mismatches with a target nucleic acid it may contain and still form a stable duplex (or triplex, as the case may be). One skilled in the art can ascertain a tolerable degree of mismatch by use of standard procedures to determine the melting point of the hybridized complex.

Antisense polynucleotides that are complementary to the 5' end of an mRNA target, e.g., the 5' untranslated sequence up to and including the AUG initiation codon, should work most efficiently at inhibiting translation of the mRNA. However, sequences complementary to the 3' untranslated sequences of mRNAs have recently been shown to be effective at inhibiting translation of mRNAs as well (Wagner, R 1994. Nature 372:333). Therefore, antisense polynucleotides complementary to either the 5' or 3' untranslated, non-coding regions of a variant CFH gene could be used in an antisense approach to inhibit translation of a variant CFH mRNA. Antisense polynucleotides complementary to the 5' untranslated region of an mRNA should include the complement of the AUG start codon. Antisense polynucleotides complementary to mRNA coding regions are less efficient inhibitors of translation but could also be used in accordance with the invention. Whether designed to hybridize to the 5', 3', or coding region of mRNA, antisense polynucleotides should be at least six nucleotides in length, and are preferably less that about 100 and more preferably less than about 50, 25, 17 or 10 nucleotides in length.

Regardless of the choice of target sequence, it is preferred that in vitro studies are first performed to quantitate the ability of the antisense polynucleotide to inhibit expression of a variant CFH gene. It is preferred that these studies utilize controls that distinguish between antisense gene inhibition and nonspecific biological effects of antisense polynucleotide. It is also preferred that these studies compare levels of the target RNA or protein with that of an internal control RNA or protein. Additionally, it is envisioned that results obtained using the antisense polynucleotide are compared with those obtained using a control antisense polynucleotide. It is preferred that the control antisense polynucleotide is of approximately the same length as the test antisense polynucleotide and that the nucleotide sequence of the control antisense polynucleotide differs from the antisense sequence of interest no more than is necessary to prevent specific hybridization to the target sequence.

Polynucleotides of the disclosure including antisense polynucleotides, can be DNA or RNA or chimeric mixtures or derivatives or modified versions thereof, single-stranded or double-stranded. Polynucleotides of the invention can be modified at the base moiety, sugar moiety, or phosphate backbone, for example, to improve stability of the molecule, hybridization, etc. Polynucleotides of the invention may include other appended groups such as peptides (e.g., for targeting host cell receptors), or agents facilitating transport across the cell membrane (see, e.g., Letsinger et al., 1989, Proc Natl Acad Sci. USA 86:6553-6556; Lemaitre et al., 1987, Proc Natl Acad Sci. USA 84:648-652; PCT Publication No. W088/09810, published Dec. 15, 1988) or the blood- brain barrier (see, e.g., PCT Publication No. W089/10134, published Apr. 25, 1988), hybridization-triggered cleavage agents. (See, e.g., Krol et al., 1988, BioTechniques 6:958-976) or intercalating agents. (See, e.g., Zon, Pharm. Res. 5:539-549 (1988)). To this end, a polynucleotide of the invention may be conjugated to another molecule, e.g., a peptide, hybridization triggered cross-linking agent, transport agent, hybridization-triggered cleavage agent, etc.

Polynucleotides of the disclosure including antisense polynucleotides, may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxytriethyl) uracil, 5-carboxymethylaminomethyl-2-thiouridine, 5-carboxymethylaminomethyluracil, dihydrouracil, beta-D-galactosylqueosine, inosine, N6- isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil; beta-D-mannosylqueosine, 5-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methyl ester, uracil-5-oxyacetic acid (v), 5-methyl-2-thiouracil, 3-(3-amino-3-N-2-carboxypropyl) uracil, (acp3)w, and 2,6-diaminopurine.

Polynucleotides of the disclosure may also comprise at least one modified sugar moiety selected from the group including but not limited to arabinose, 2-fluoroarabinose, xylulose, and hexose.

A polynucleotide of the disclosure can also contain a neutral peptide-like backbone. Such molecules are termed peptide nucleic acid (PNA)-oligomers and are described, e.g., in Perry-O'Keefe et al. (1996) Proc. Natl. Acad. Sci. USA 93:14670 and in Eglom et al. (1993) Nature 365:566. One advantage of PNA oligomers is their capability to bind to complementary DNA essentially independently from the ionic strength of the medium due to the neutral backbone of the DNA. In yet another embodiment, a polynucleotide of the invention comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In a further embodiment, polynucleotides of the disclosure, including antisense polynucleotides are -anomeric oligonucleotides. An -anomeric oligonucleotide forms specific double-stranded hybrids with complementary RNA in which, contrary to the usual -units, the strands run parallel to each other (Gautier et al., 1987, Nucl. Acids Res. 15:6625-6641). The oligonucleotide is a 2'-O-methylribonucleotide (Inoue et al., 1987, Nucl. Acids Res. 15:6131-6148), or a chimeric RNA-DNA analogue (Inoue et al., 1987, FEBS Lett. 215:327-330).

Polynucleotides of the disclosure including antisense polynucleotides, may be synthesized by standard methods known in the art, e.g., by use of an automated DNA synthesizer (such as are commercially available from Biosearch, Applied Biosystems, etc.). As examples, phosphorothioate oligonucleotides may be synthesized by the method of Stein et al. Nucl. Acids Res. 16:3209 (1988)), methylphosphonate oligonucleotides can be prepared by use of controlled pore glass polymer supports (Sarin et al., Proc. Natl. Acad. Sci. USA 85:7448-7451 (1988)),.

While antisense sequences complementary to the coding region of an mRNA sequence can be used, those complementary to the transcribed untranslated region and to the region comprising the initiating methionine are most preferred.

Antisense polynucleotides can be delivered to cells that express target genes in vivo. A number of methods have been developed for delivering nucleic acids into cells; e.g., they can be injected directly into the tissue site, or modified nucleic acids, designed to target the desired cells (e.g., antisense polynucleotides linked to peptides or antibodies that specifically bind receptors or antigens expressed on the target cell surface) can be administered systematically.

However, it may be difficult to achieve intracellular concentrations of the antisense polynucleotides sufficient to attenuate the activity of a variant CFH gene or mRNA in certain instances. Therefore, another approach utilizes a recombinant DNA construct in which the antisense polynucleotide is placed under the control of a strong pol III or pol II promoter. The use of such a construct to transfect target cells in the patient will result in the transcription of sufficient amounts of antisense polynucleotides that will form complementary base pairs with the variant CFH gene or mRNA and thereby attenuate the activity of CFH protein. For example, a vector can be introduced in vivo such that it is taken up by a cell and directs the transcription of an antisense polynucleotide that targets a variant CFH gene or mRNA. Such a vector can remain episomal or become chromosomally integrated, as long as it can be transcribed to produce the desired antisense polynucleotide. Such vectors can be constructed by recombinant DNA technology methods standard in the art. Vectors can be plasmid, viral, or others know in the art, used for replication and expression in mammalian cells. A promoter may be operably linked to the sequence encoding the antisense polynucleotide. Expression of the sequence encoding the antisense polynucleotide can be by any promoter known in the art to act in mammalian, preferably human cells. Such promoters can be inducible or constitutive. Such promoters include but are not limited to: the SV40 early promoter region (Bemoist and Chambon, Nature 290:304-310 (1981)), the promoter contained in the 3' long terminal repeat of Rous sarcoma virus (Yamamoto et al., Cell 22:787-797 (1980)), the herpes thymidine kinase promoter (Wagner et al., Proc. Natl. Acad. Sci. USA 78:1441-1445 (1981)), the regulatory sequences of the metallothionine gene (Brinster et al, Nature 296:3942 (1982)). Any type of plasmid, cosmid, YAC or viral vector can be used to prepare the recombinant DNA construct that can be introduced directly into the tissue site. Alternatively, viral vectors can be used which selectively infect the desired tissue, in which case administration may be accomplished by another route (e.g., systematically).

### RNAi constructs - siRNAs and miRNAs

RNA interference (RNAi) is a phenomenon describing double-stranded (ds)RNA-dependent gene specific posttranscriptional silencing. Initial attempts to harness this phenomenon for experimental manipulation of mammalian cells were foiled by a robust and nonspecific antiviral defense mechanism activated in response to long dsRNA molecules. Gil et al. Apoptosis 2000, 5:107-114. The field was significantly advanced upon the demonstration that synthetic duplexes of 21 nucleotide RNAs could mediate gene specific RNAi in mammalian cells, without invoking generic antiviral defense mechanisms. Elbashir et al. Nature 2001, 411:494-498; Caplen et al. Proc Natl Acad Sci 2001, 98:9742-9747. As a result, small-interfering RNAs (siRNAs) and micro RNAs (miRNAs) have become powerful tools to dissect gene function. The chemical synthesis of small RNAs is one avenue that has produced promising results. Numerous groups have also sought the development of DNA-based vectors capable of generating such siRNA within cells. Several groups have recently attained this goal and published similar strategies that, in general, involve transcription of short hairpin (sh)RNAs that are efficiently processed to form siRNAs within cells. Paddison et al. PNAS 2002, 99:1443-1448; Paddison et al. Genes & Dev 2002, 16:948-958; Sui et al. PNAS 2002, 8:5515-5520; and Brummelkamp et al. Science 2002, 296:550-553. These reports describe methods to generate siRNAs capable of specifically targeting numerous endogenously and exogenously expressed genes.

Accordingly, the present disclosure provides a polynucleotide comprising an RNAi sequence that acts through an RNAi or miRNA mechanism to attenuate expression of a variant CFH gene. For instance, a polynucleotide of the invention may comprise a miRNA or siRNA sequence that attenuates or inhibits expression of a variant CFH gene. In one embodiment, the miRNA or siRNA sequence is between about 19 nucleotides and about 75 nucleotides in length, or preferably, between about 25 base pairs and about 35 base pairs in length. In certain embodiments, the polynucleotide is a hairpin loop or stem-loop that may be processed by RNAse enzymes (e.g., Drosha and Dicer).

An RNAi construct contains a nucleotide sequence that hybridizes under physiologic conditions of the cell to the nucleotide sequence of at least a portion of the mRNA transcript for a variant CFH gene. The double-stranded RNA need only be sufficiently similar to natural RNA that it has the ability to mediate RNAi. The number of tolerated nucleotide mismatches between the target sequence and the RNAi construct sequence is no more than 1 in 5 basepairs, or 1 in 10 basepairs, or 1 in 20 basepairs, or 1 in 50 basepairs. It is primarily important the that RNAi construct is able to specifically target a variant CFH gene. Mismatches in the center of the siRNA duplex are most critical and may essentially abolish cleavage of the target RNA. In contrast, nucleotides at the 3' end of the siRNA strand that is complementary to the target RNA do not significantly contribute to specificity of the target recognition.

Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript (e.g., 400 mM NaCl, 40mM PIPES pH 6.4, 1 mM EDTA, 50 °C or 70 °C hybridization for 12-16 hours; followed by washing).

Production of polynucleotides comprising RNAi sequences can be carried out by any of the methods for producing polynucleotides described herein. For example, polynucleotides comprising RNAi sequences can be produced by chemical synthetic methods or by recombinant nucleic acid techniques. Endogenous RNA polymerase of the treated cell may mediate transcription in vivo, or cloned RNA polymerase can be used for transcription in vitro. Polynucleotides of the invention, including wildtype or antisense polynucleotides, or those that modulate target gene activity by RNAi mechanisms, may include modifications to either the phosphate-sugar backbone or the nucleoside, e.g., to reduce susceptibility to cellular nucleases, improve bioavailability, improve formulation characteristics, and/or change other pharmacokinetic properties. For example, the phosphodiester linkages of natural RNA may be modified to include at least one of a nitrogen or sulfur heteroatom. Modifications in RNA structure may be tailored to allow specific genetic inhibition while avoiding a general response to dsRNA. Likewise, bases may be modified to block the activity of adenosine deaminase. Polynucleotides of the invention may be produced enzymatically or by partial/total organic synthesis, any modified ribonucleotide can be introduced by in vitro enzymatic or organic synthesis.

Methods of chemically modifying RNA molecules can be adapted for modifying RNAi constructs (see, for example, Heidenreich et al. (1997) Nucleic Acids Res, 25:776-780; Wilson et al. (1994) J Mol Recog 7:89-98; Chen et al. (1995) Nucleic Acids Res 23:2661-2668; Hirschbein et al. (1997) Antisense Nucleic Acid Drug Dev 7:55-61). Merely to illustrate, the backbone of an RNAi construct can be modified with phosphorothioates, phosphoramidate, phosphodithioates, chimeric methylphosphonate-phosphodiesters, peptide nucleic acids, 5-propynyl-pyrimidine containing oligomers or sugar modifications (e.g., 2'-substituted ribonucleosides, α-configuration).

The double-stranded structure may be formed by a single self-complementary RNA strand or two complementary RNA strands. RNA duplex formation may be initiated either inside or outside the cell. The RNA may be introduced in an amount which allows delivery of at least one copy per cell. Higher doses (e.g., at least 5, 10, 100, 500 or 1000 copies per cell) of double-stranded material may yield more effective inhibition, while lower doses may also be useful for specific applications. Inhibition is sequence-specific in that nucleotide sequences corresponding to the duplex region of the RNA are targeted for genetic inhibition.

In certain embodiments, the subject RNAi constructs are "siRNAs." These nucleic acids are between about 19-35 nucleotides in length, and even more preferably 21-23 nucleotides in length, e.g., corresponding in length to the fragments generated by nuclease "dicing" of longer double-stranded RNAs. The siRNAs are understood to recruit nuclease complexes and guide the complexes to the target mRNA by pairing to the specific sequences. As a result, the target mRNA is degraded by the nucleases in the protein complex or translation is inhibited. In a particular embodiment, the 21-23 nucleotides siRNA molecules comprise a 3' hydroxyl group.

In other embodiments, the subject RNAi constructs are "miRNAs." microRNAs (miRNAs) are small non-coding RNAs that direct post transcriptional regulation of gene expression through interaction with homologous mRNAs. miRNAs control the expression of genes by binding to complementary sites in target mRNAs from protein coding genes. miRNAs are similar to siRNAs. miRNAs are processed by nucleolytic cleavage from larger double-stranded precursor molecules. These precursor molecules are often hairpin structures of about 70 nucleotides in length, with 25 or more nucleotides that are base-paired in the hairpin. The RNAse III-like enzymes Drosha and Dicer (which may also be used in siRNA processing) cleave the miRNA precursor to produce an miRNA. The processed miRNA is single-stranded and incorporates into a protein complex, termed RISC or miRNP. This RNA-protein complex targets a complementary mRNA. miRNAs inhibit translation or direct cleavage of target mRNAs. (Brennecke et al., Genome Biology 4:228 (2003); Kim et al., Mol. Cells 19:1-15 (2005).

In certain embodiments, miRNA and siRNA constructs can be generated by processing of longer double-stranded RNAs, for example, in the presence of the enzymes Dicer or Drosha. Dicer and Drosha are RNAse III-like nucleases that specifically cleave dsRNA. Dicer has a distinctive structure which includes a helicase domain and dual RNAse III motifs. Dicer also contains a region of homology to the RDE1/QDE2/ARGONAUTE family, which have been genetically linked to RNAi in lower eukaryotes. Indeed, activation of, or overexpression of Dicer may be sufficient in many cases to permit RNA interference in otherwise non-receptive cells, such as cultured eukaryotic cells, or mammalian (non-oocytic) cells in culture or in whole organisms.

In one embodiment, the Drosophila in vitro system is used. In this embodiment, a polynucleotide comprising an RNAi sequence or an RNAi precursor is combined with a soluble extract derived from Drosophila embryo, thereby producing a combination. The combination is maintained under conditions in which the dsRNA is processed to RNA molecules of about 21 to about 23 nucleotides.

The miRNA and siRNA molecules can be purified using a number of techniques known to those of skill in the art. For example, gel electrophoresis can be used to purify such molecules. Alternatively, non-denaturing methods, such as non-denaturing column chromatography, can be used to purify the siRNA and miRNA molecules. In addition, chromatography (e.g., size exclusion chromatography), glycerol gradient centrifugation, affinity purification with antibody can be used to purify siRNAs and miRNAs.

In certain embodiments, at least one strand of the siRNA sequence of an effector domain has a 3' overhang from about 1 to about 6 nucleotides in length, or from 2 to 4 nucleotides in length. In other embodiments, the 3' overhangs are 1-3 nucleotides in length. In certain embodiments, one strand has a 3' overhang and the other strand is either blunt-ended or also has an overhang. The length of the overhangs may be the same or different for each strand. In order to further enhance the stability of the siRNA sequence, the 3' overhangs can be stabilized against degradation. In one embodiment, the RNA is stabilized by including purine nucleotides, such as adenosine or guanosine nucleotides. Alternatively, substitution of pyrimidine nucleotides by modified analogues, e.g., substitution of uridine nucleotide 3' overhangs by 2'-deoxythyinidine is tolerated and does not affect the efficiency of RNAi. The absence of a 2' hydroxyl significantly enhances the nuclease resistance of the overhang in tissue culture medium and may be beneficial in vivo.

In certain embodiments, a polynucleotide of the invention that comprises an RNAi sequence or an RNAi precursor is in the form of a hairpin structure (named as hairpin RNA). The hairpin RNAs can be synthesized exogenously or can be formed by transcribing from RNA polymerase III promoters in vivo. Examples of making and using such hairpin RNAs for gene silencing in mammalian cells are described in, for example, Paddison et al., Genes Dev, 2002, 16:948-58; McCaffrey et al., Nature, 2002, 418:38-9; McManus et al., RNA 2002, 8:842-50; Yu et al., Proc Natl Acad Sci USA, 2002, 99:6047-52). Preferably, such hairpin RNAs are engineered in cells or in an animal to ensure continuous and stable suppression of a desired gene. It is known in the art that miRNAs and siRNAs can be produced by processing a hairpin RNA in the cell.

In yet other embodiments, a plasmid is used to deliver the double-stranded RNA, e.g., as a transcriptional product. After the coding sequence is transcribed, the complementary RNA transcripts base-pair to form the double-stranded RNA.

### Aptamers and small molecules

The present disclosure also provides therapeutic aptamers that specifically bind to variant CFH polypeptides that are associated with AMD, thereby modulating activity of the variant CFH polypeptide. An "aptamer" may be a nucleic acid molecule, such as RNA or DNA that is capable of binding to a specific molecule with high affinity and specificity (Ellington et al., Nature 346, 818-22 (1990); and Tuerk et al., Science 249, 505-10 (1990)). An aptamer will most typically have been obtained by in vitro selection for binding of a target molecule. For example, an aptamer that specifically binds a variant CFH polypeptide can be obtained by in vitro selection for binding to a variant CFH polypeptide from a pool of polynucleotides. However, in vivo selection of an aptamer is also possible. Aptamers have specific binding regions which are capable of forming complexes with an intended target molecule in an environment wherein other substances in the same environment are not complexed to the nucleic acid. The specificity of the binding is defined in terms of the comparative dissociation constants (Kd) of the aptamer for its ligand (e.g., a variant CFH polypeptide) as compared to the dissociation constant of the aptamer for other materials in the environment or unrelated molecules in general. A ligand (e.g., a variant CFH polypeptide) is one which binds to the aptamer with greater affinity than to unrelated material. Typically, the Kd for the aptamer with respect to its ligand will be at least about 10-fold less than the Kd for the aptamer with unrelated material or accompanying material in the environment Even more preferably, the Kd will be at least about 50-fold less, more preferably at least about 100-fold less, and most preferably at least about 200-fold less. An aptamer will typically be between about 10 and about 300 nucleotides in length. More commonly, an aptamer will be between about 30 and about 100 nucleotides in length.

Methods for selecting aptamers specific for a target of interest are known in the art. For example, organic molecules, nucleotides, amino acids, polypeptides, target features on cell surfaces, ions, metals, salts, saccharides, have all been shown to be suitable for isolating aptamers that can specifically bind to the respective ligand. For instance, organic dyes such as Hoechst 33258 have been successfully used as target ligands for in vitro aptamer selections (Werstuck and Green, Science 282:296-298 (1998)). Other small organic molecules like dopamine, theophylline, sulforhodamine B, and cellobiose have also been used as ligands in the isolation of aptamers. Aptamers have also been isolated for antibiotics such as kanamycin A, lividomycin, tobramycin, neomycin B, viomycin, chloramphenicol and streptomycin. For a review of aptamers that recognize small molecules, see Famulok, Science 9:324-9 (1999).

An aptamer of the disclosure can be comprised entirely of RNA. In other embodiments of the invention, however, the aptamer can instead be comprised entirely of DNA, or partially of DNA, or partially of other nucleotide analogs. To specifically inhibit translation in vivo, RNA aptamers are preferred. Such RNA aptamers are preferably introduced into a cell as DNA that is transcribed into the RNA aptamer. Alternatively, an RNA aptamer itself can be introduced into a cell.

Aptamers are typically developed to bind particular ligands by employing known in vivo or in vitro (most typically, in vitro) selection techniques known as SELEX (Ellington et al., Nature 346, 818-22 (1990); and Tuerk et al., Science 249, 505-10 (1990)). Methods of making aptamers are also described in, for example, U.S. Pat No. 5,582,981, PCT Publication No. WO 00/20040, U.S. Pat No. 5,270,163, Lorsch and Szostak, Biochemistry, 33:973 (1994), Mannironi et al., Biochemistry 36:9726 (1997), Blind, Proc. Nat'l. Acad. Sci. USA 96:3606-3610 (1999), Huizenga and Szostak, Biochemistry, 34:656-665 (1995), PCT Publication Nos. WO 99/54506, WO 99/27133, WO 97/42317 and U.S. Pat. No. 5,756,291.

Generally, in their most basic form, in vitro selection techniques for identifying aptamers involve first preparing a large pool of DNA molecules of the desired length that contain at least some region that is randomized or mutagenized. For instance, a common oligonucleotide pool for aptamer selection might contain a region of 20-100 randomized nucleotides flanked on both ends by an about 15-25 nucleotide long region of defined sequence useful for the binding of PCR primers. The oligonucleotide pool is amplified using standard PCR techniques, although any means that will allow faithful, efficient amplification of selected nucleic acid sequences can be employed. The DNA pool is then in vitro transcribed to produce RNA transcripts. The RNA transcripts may then be subjected to affinity chromatography, although any protocol which will allow selection of nucleic acids based on their ability to bind specifically to another molecule (e.g., a protein or any target molecule) may be used. In the case of affinity chromatography, the transcripts are most typically passed through a column or contacted with magnetic beads or the like on which the target ligand has been immobilized. RNA molecules in the pool which bind to the ligand are retained on the column or bead, while nonbinding sequences are washed away. The RNA molecules which bind the ligand are then reverse transcribed and amplified again by PCR (usually after elution). The selected pool sequences are then put through another round of the same type of selection. Typically, the pool sequences are put through a total of about three to ten iterative rounds of the selection procedure. The cDNA is then amplified, cloned, and sequenced using standard procedures to identify the sequence of the RNA molecules which are capable of acting as aptamers for the target ligand. Once an aptamer sequence has been successfully identified, the aptamer may be further optimized by performing additional rounds of selection starting from a pool of oligonucleotides comprising the mutagenized aptamer sequence. For use in the present invention, the aptamer is preferably selected for ligand binding in the presence of salt concentrations and temperatures which mimic normal physiological conditions.

The unique nature of the in vitro selection process allows for the isolation of a suitable aptamer that binds a desired ligand despite a complete dearth of prior knowledge as to what type of structure might bind the desired ligand.

The association constant for the aptamer and associated ligand is preferably such that the ligand functions to bind to the aptamer and have the desired effect at the concentration of ligand obtained upon administration of the ligand. For in vivo use, for example, the association constant should be such that binding occurs well below the concentration of ligand that can be achieved in the serum or other tissue. Preferably, the required ligand concentration for in vivo use is also below that which could have undesired effects on the organism.

The present disclosure also provides small molecules and antibodies that specifically bind to a variant CFH polypeptide that is associated with AMD, thereby inhibiting the activity of a variant CFH polypeptide. Examples of small molecules include, without limitation, drugs, metabolites, intermediates, cofactors, transition state analogs, ions, metals, toxins and natural and synthetic polymers (e.g., proteins, peptides, nucleic acids, polysaccharides, glycoproteins, hormones, receptors and cell surfaces such as cell walls and cell membranes).

### Antibodies

Another aspect of the disclosure pertains to antibodies. In one embodiment, an antibody that is specifically reactive with a variant CFH polypeptide may be used to detect the presence of a variant CFH polypeptide or to inhibit activity of a variant CFH polypeptide. For example, by using immunogens derived from a variant CFH peptide, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of the variant CFH peptide, an antigenic fragment which is capable of eliciting an antibody response, or a fusion protein. In a particular embodiment, the inoculated mouse does not express endogenous CFH, thus facilitating the isolation of antibodies that would otherwise be eliminated as anti-self antibodies. Techniques for conferring immunogenicity on a protein or peptide include conjugation to carriers or other techniques well known in the art. An immunogenic portion of a variant CFH peptide can be administered in the presence of adjuvant The progress of immunization can be monitored by detection of antibody titers in plasma or serum. Standard ELISA or other immunoassays can be used with the immunogen as antigen to assess the levels of antibodies.

Following immunization of an animal with an antigenic preparation of a variant CFH polypeptide, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a variant CFH polypeptide and monoclonal antibodies isolated from a culture comprising such hybridoma cells.

The term "antibody" as used herein is intended to include fragments thereof which are also specifically reactive with a variant CFH polypeptide. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific, single-chain, and chimeric and humanized molecules having affinity for a variant CFH polypeptide conferred by at least one CDR region of the antibody. In preferred embodiments, the antibody further comprises a label attached thereto and able to be detected (e.g., the label can be a radioisotope, fluorescent compound, enzyme or enzyme co-factor).

In certain embodiments, an antibody of the disclosure is a monoclonal antibody, and in certain embodiments, the invention makes available methods for generating novel antibodies that bind specifically to variant CFH polypeptides. For example, a method for generating a monoclonal antibody that binds specifically to a variant CFH polypeptide may comprise administering to a mouse an amount of an immunogenic composition comprising the CFH polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to the variant CFH polypeptide. Once obtained, a hybridoma can be propagated in a cell culture, optionally in culture conditions where the hybridoma-derived cells produce the monoclonal antibody that binds specifically to the CFH polypeptide. The monoclonal antibody may be purified from the cell culture.

The term "specifically reactive with" as used in reference to an antibody is intended to mean, as is generally understood in the art, that the antibody is sufficiently selective between the antigen of interest (e.g., a variant CFH polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody:antigen interaction is the affinity of the antibody for the antigen. Although the desired specificity may be reached with a range of different affinities, generally preferred antibodies will have an affinity (a dissociation constant) of about 10⁻⁶, 10⁻⁷, 10⁻⁸, 10⁻⁹ or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the Biacore binding assay, Bia-core AB, Uppsala, Sweden), sandwich assays (e.g., the paramagnetic bead system of IGEN International, Inc., Gaithersburg, Maryland), western blots, immunoprecipitation assays, and immunohistochemistry.

### 6. Screening Assays

In certain aspects, the present disclosure relates to the use of CFH polypeptides to identify compounds (agents) which are agonist or antagonists of CFH polypeptides. Compounds identified through this screening can be tested in cells of the eye, (e.g., epithelial and endothelial cells) as well as other tissues (e.g., muscle and/or neurons) to assess their ability to modulate CFH activity in vivo or in vitro. In certain aspects, compounds identified through this screening modulate the formation of drusen deposits. Optionally, these compounds can further be tested in animal models to assess their ability to modulate CFH activity in vivo.

There are numerous approaches to screening for therapeutic agents that target CFH polypeptides. In certain embodiments, high-throughput screening of compounds can be carried out to identify agents that affect activity of CFH polypeptides. A variety of assay formats will suffice and, in light of the present disclosure, those not expressly described herein will nevertheless be comprehended by one of ordinary skill in the art. As described herein, the test compounds (agents) of the invention may be created by any combinatorial chemical method. Alternatively, the subject compounds may be naturally occurring biomolecules synthesized in vivo or in vitro. Compounds (agents) to be tested for their ability to act as modulators of CFH activity can be produced, for example, by bacteria, yeast, plants or other organisms (e.g., natural products), produced chemically (e.g., small molecules, including peptidomimetics), or produced recombinantly. Test compounds contemplated by the present invention include non-peptidyl organic molecules, peptides, polypeptides, peptidomimetics, sugars, hormones, and nucleic acid molecules.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be optionally derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S transferase (GST), photoactivatible crosslinkers or any combinations thereof.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the in vitro system, the assay instead being focused primarily on the effect of the drug on the molecular target.

In certain embodiments, the subject compounds are identified by their ability to interact with a CFH polypeptide of the invention. The interaction between the compound and the CFH polypeptide may be covalent or non-covalent. For example, such interaction can be identified at the protein level using in vitro biochemical methods, including photocrosslinking, radiolabeled ligand binding, and affinity chromatography (Jakoby WB et al., 1974, Methods in Enzymology 46:1). In certain cases, the compounds may be screened in a mechanism based assay, such as an assay to detect compounds which bind to a CFH polypeptide. This may include a solid phase or fluid phase binding event. Alternatively, the gene encoding a CFH polypeptide can be transfected with a reporter system (e.g., β-galactosidase, luciferase, or green fluorescent protein) into a cell and screened against the library preferably by a high throughput screening or with individual members of the library. Other mechanism based binding assays may be used, for example, binding assays which detect changes in free energy. Binding assays can be performed with the target fixed to a well, bead or chip or captured by an immobilized antibody or resolved by capillary electrophoresis. The bound compounds may be detected usually using colorimetric or fluorescence or surface plasmon resonance.

### 7. Pharmaceutical Compositions

The methods and compositions described herein for treating a subject suffering from AMD may be used for the prophylactic treatment of individuals who have been diagnosed or predicted to be at risk for developing AMD. In this case, the composition is administered in an amount and dose that is sufficient to delay, slow, or prevent the onset of AMD or related symptoms. Alternatively, the methods and compositions described herein may be used for the therapeutic treatment of individuals who suffer from AMD. In this case, the composition is administered in an amount and dose that is sufficient to delay or slow the progression of the condition, totally or partially, or in an amount and dose that is sufficient to reverse the condition to the point of eliminating the disorder. It is understood that an effective amount of a composition for treating a subject who has been diagnosed or predicted to be at risk for developing AMD is a dose or amount that is in sufficient quantities to treat a subject or to treat the disorder itself.

In certain embodiments, compounds of the present disclosure (e.g., an isolated or recombinantly produced nucleic acid molecule coding for a CFH polypeptide or an isolated or recombinantly produced CFH polypeptide) are formulated with a pharmaceutically acceptable carrier. For example, a CFH polypeptide or a nucleic acid molecule coding for a CFH polypeptide can be administered alone or as a component of a pharmaceutical formulation (therapeutic composition). The subject compounds may be formulated for administration in any convenient way for use in human medicine.

The therapeutic methods of the disclosure also include administering the composition topically, systemically, or locally. For example, therapeutic compositions of the invention may be formulated for administration by, for example, injection (e.g., intravenously, subcutaneously, or intramuscularly), inhalation or insufflation (either through the mouth or the nose) or oral, buccal, sublingual, transdermal, nasal, or parenteral administration. The compositions described herein may be formulated as part of an implant or device. When administered, the therapeutic composition for use in this invention is in a pyrogen-free, physiologically acceptable form. Further, the composition may be encapsulated or injected in a viscous form for delivery to the site where the target cells are present, such as to the cells of the eye. Techniques and formulations generally may be found in Remington's Pharmaceutical Sciences, Meade Publishing Co., Easton, PA. In addition to CFH polypeptides or nucleic acid molecules coding for CFH polypeptides, therapeutically useful agents may optionally be included in any of the compositions as described above. Furthermore, therapeutically useful agents may, alternatively or additionally, be administered simultaneously or sequentially with CFH polypeptides or nucleic acid molecules coding for CFH polypeptides according to the methods of the invention.

Compositions of the disclosure can also be administered orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds of the present invention may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, com, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

Certain compositions disclosed herein may be administered topically, either to skin or to mucosal membranes. The topical formulations may further include one or more of the wide variety of agents known to be effective as skin or stratum corneum penetration enhancers. Examples of these are 2-pyrrolidone, N-methyl-2-pyrrolidone, dimethylacetamide, dimethylformamide, propylene glycol, methyl or isopropyl alcohol, dimethyl sulfoxide, and azone. Additional agents may further be included to make the formulation cosmetically acceptable. Examples of these are fats, waxes, oils, dyes, fragrances, preservatives, stabilizers, and surface active agents. Keratolytic agents such as those known in the art may also be included. Examples are salicylic acid and sulfur.

Dosage forms for the topical or transdermal administration include powders, sprays, ointments, pastes, creams, lotions, gels, solutions, patches, and inhalants. The active compound may be mixed under sterile conditions with a pharmaceutically acceptable carrier, and with any preservatives, buffers, or propellants which may be required. The ointments, pastes, creams and gels may contain, in addition to a subject compound of the invention (e.g., an isolated or recombinantly produced nucleic acid molecule coding for a CFH polypeptide or an isolated or recombinantly produced CFH polypeptide), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a subject compound, excipients such as lactose, talc, silicic acid, aluminum hydroxide, calcium silicates, and polyamide powder, or mixtures of these substances. Sprays can additionally contain customary propellants, such as chlorofluorohydrocarbons and volatile unsubstituted hydrocarbons, such as butane and propane.

It is understood that the dosage regimen will be determined for an individual, taking into consideration, for example, various factors which modify the action of the subject compounds of the invention (e.g., an isolated or recombinantly produced nucleic acid molecule coding for a CFH polypeptide or an isolated or recombinantly produced CFH polypeptide), the severity or stage of AMD, route of administration, and characteristics unique to the individual, such as age, weight, and size. A person of ordinary skill in the art is able to determine the required dosage to treat the subject. In one embodiment, the dosage can range from about 1.0 ng/kg to about 100 mg/kg body weight of the subject Based upon the composition, the dose can be delivered continuously, or at periodic intervals. For example, on one or more separate occasions. Desired time intervals of multiple doses of a particular composition can be determined without undue experimentation by one skilled in the art. For example, the compound may be delivered hourly, daily, weekly, monthly, yearly (e.g. in a time release form) or as a one time delivery.

In certain embodiments, pharmaceutical compositions suitable for parenteral administration may comprise a CFH polypeptide or a nucleic acid molecule coding for a CFH polypeptide in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The compositions of the disclosure may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

The present disclosure also provides gene therapy for the in vivo production of CFH polypeptides. Such therapy would achieve its therapeutic effect by introduction of CFH polynucleotide sequences into cells or tissues that are deficient for normal CFH function. Delivery of CFH polynucleotide sequences can be achieved using a recombinant expression vector such as a chimeric virus or a colloidal dispersion system. Targeted liposomes may also be used for the therapeutic delivery of CFH polynucleotide sequences.

Various viral vectors which can be utilized for gene therapy as taught herein include adenovirus, herpes virus, vaccinia, or an RNA virus such as a retrovirus. A retroviral vector may be a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing the CFH polynucleotide. In one preferred embodiment, the vector is targeted to cells or tissues of the eye.

Alternatively, tissue culture cells can be directly transfected with plasmids encoding the retroviral structural genes gag, pol and env, by conventional calcium phosphate transfection. These cells are then transfected with the vector plasmid containing the genes of interest The resulting cells release the retroviral vector into the culture medium.

Another targeted delivery system for CFH polynucleotides is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome. Liposomes are artificial membrane vesicles which are useful as delivery vehicles in vitro and in vivo. RNA, DNA and intact virions can be encapsulated within the aqueous interior and be delivered to cells in a biologically active form (see e.g., Fraley, et al., Trends Biochem. Sci., 6:77, 1981). Methods for efficient gene transfer using a liposome vehicle, are known in the art, see e.g., Mannino, et al., Biotechniques, 6:682, 1988. The composition of the liposome is usually a combination of phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

Moreover, the pharmaceutical preparation can consist essentially of the gene delivery system in an acceptable diluent, or can comprise a slow release matrix in which the gene delivery vehicle is imbedded. Alternatively, where the complete gene delivery system can be produced intact from recombinant cells, e.g. retroviral packages, the pharmaceutical preparation can comprise one or more cells which produce the gene delivery system. In the case of the latter, methods of introducing the viral packaging cells may be provided by, for example, rechargeable or biodegradable devices. Various slow release polymeric devices have been developed and tested in vivo in recent years for the controlled delivery of drugs, including proteinacious biopharmaceuticals, and can be adapted for release of viral particles through the manipulation of the polymer composition and form. A variety of biocompatible polymers (including hydrogels), including both biodegradable and non-degradable polymers, can be used to form an implant for the sustained release of the viral particles by cells implanted at a particular target site. Such embodiments of the present invention can be used for the delivery of an exogenously purified virus, which has been incorporated in the polymeric device, or for the delivery of viral particles produced by a cell encapsulated in the polymeric device.

A person of ordinary skill in the art is able to determine the required amount to treat the subject. It is understood that the dosage regimen will be determined for an individual, taking into consideration, for example, various factors which modify the action of the subject compounds of the invention, the severity or stage of AMD, route of administration, and characteristics unique to the individual, such as age, weight, and size. A person of ordinary skill in the art is able to determine the required dosage to treat the subject. In one embodiment, the dosage can range from about 1.0 ng/kg to about 100 mg/kg body weight of the subject. The dose can be delivered continuously, or at periodic intervals. For example, on one or more separate occasions. Desired time intervals of multiple doses of a

### 8. Kits The methods of the invention may be carried out with various kits.

Described herein are kits, e.g., kits for detecting a variant CFH gene in a sample from an individual. A kit may comprise at least one container means having disposed therein a premeasured dose of a polynucleotide probe that hybridizes, under stringent conditions, to a variation in the CFH gene that is correlated with the occurrence of AMD in humans. A kit comprises at least one container means having disposed therein a premeasured dose of a polynucleotide primer that hybridizes, under stringent conditions, adjacent to one side of a variation in the CFH gene that is correlated with the occurrence of AMD in humans. In a further embodiment, a second polynucleotide primer that hybridizes, under stringent conditions, to the other side of a variation in the CFH gene that is correlated with the occurrence of AMD in humans is provided in a premeasured dose. Kits further comprise a label and/or instructions for the use of the therapeutic or diagnostic kit in the detection of CFH in a sample. Kits may also include packaging material such as, but not limited to, ice, dry ice, styrofoam, foam, plastic, cellophane, shrink wrap, bubble wrap, paper, cardboard, starch peanuts, twist ties, metal clips, metal cans, drierite, glass, and rubber (see products available from www.papermart.com. for examples of packaging material).

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning: A Laboratory Manual, Cold Spring Harbor Laboratory (2001); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXAMPLES

The following examples are for illustrative purposes and are not intended to be limiting in any way.

### EXAMPLE 1: Whole genome SNP association for genes correlated with AMD.

Described herein is a whole-genome case-control association study for genes involved in AMD. Two crucial factors were used in designing this experiment; clearly defined phenotypes were chosen for cases and controls. The definition of a case was based on both a quantitative photographic assessment of the presence of at least some large drusen combined with photographic evidence of sight-threatening AMD (geographic atrophy or neovascular AMD). The definition of a control was based on the study participant having either no drusen or only a few small drusen. Data was analyzed using a statistically conservative approach to correct for the large number of SNPs tested, thereby guaranteeing that the actual probability of a false positive is no greater than the reported p-values.

A subset of individuals who participated in the Age-Related Eye Disease Study (AREDS) (AREDS Research Group, Arch Ophthamol 119, 1417, (2001)) were used in the association study. From the AREDS sample, 96 case subjects were identified who, at their most recent study visit, had either uniocular choroidal neovascularization (50 cases) or geographic atrophy either central or non-central to the macula (46 cases). The fellow eye of these case subjects was required to have at least one large drusen (>125 µm in diameter), and total drusen area equivalent to a circle of at least 1061 µm in diameter. The group of study participants who had both large drusen and sight-threatening AMD was selected because there can be many precursors to the development of either choroidal neovascularization or geographic atrophy. Controls were 50 individuals from the AREDS sample who had few or no drusen (<63 µm in diameter in each eye) for the duration of their participation in AREDS. All individuals identified themselves as "White, not of Hispanic origin." To the extent possible, the proportions of gender and smoking status were kept the same in cases and controls. Controls were purposely chosen to be older than the cases to increase the probability that they will remain without AMD (Table 1).

**Table 1. Summary of sample phenotypes.**

| | Cases (n=96) | Controls (n=50) |
|---|---|---|
| Males (%) | 44 | 54 |
| Never smoked (%) | 36 | 52 |
| Formerly smoked (%) | 58 | 48 |
| Currently smoke (%) | 5 | 0 |
| Mean age (± s.d.) (years) | 79±5.2 | 82±2.2 |
| Age range (years) | 65-89 | 78-87 |
| One eye with neovascular AMD, other eye with at least one large drusen (%) | 52 | 0 |
| One eye with geographic atrophy AMD, other eye with at least one large drusen (%) | 48 | 0 |
| Both eyes with few or no drusen (%) | 0 | 100 |

### EXAMPLE 2: Genotyping and SNP identification of individuals in study population

Each individual was genotyped using the Affymetrix GeneChip Mapping 100K Set of microarrays (H. Matsuzaki et al., Nat Methods 1, 109 (2004)). This mapping assay consists of two chips (XbaI and HindIII) with approximately 50,000 SNPs each that are used for each individual. About 250 ng of genomic DNA was digested with two restriction enzymes XbaI and HindIII and processed according to the Affymetrix protocol (H. Matsuzaki et al., Nat Methods 1, 109 (2004)). The images were analyzed using GDAS software (Affymetrix). For the data obtained from each chip, two internal quality control measures were used: the call rate always exceeded 95% and heterozygosity on the X chromosome correctly identified the gender of the individual. Thirty-one identical SNPs were placed on both chips and checked that they yielded the same genotype for the same individual to ensure that no samples were confused.

Three experiments were performed to test the reproducibility of this system. First, 4 samples were processed twice with the Xba chips. Next, 2 replicates of a reference DNA positive control provided by Affymetrix were run on Xba chips alongside the samples. Finally, results for 3 individuals were compared with genotyping using the Affymetrix 10K SNP platform to test the accuracy of this assay (H. Matsuzaki et al., Genome Res 14, 414 (2004)).

An assessment of the percentage of the individuals producing a genotype call for each SNP was made in order to examine the genotyping quality for each individual SNP. A SNP with a call rate of 100% means every individual is successfully assigned a genotype for this SNP and there is no missing data. Call rates were required to be at least 85% to remove SNPs for which genotyping was consistently problematic. SNPs which are monomorphic in the data were also removed, since these SNPs are uninformative. SNPs in which genotype frequencies deviated from the Hardy-Weinberg equilibrium expectation (HWD χ² > 25, *P* = 0.05, 1 df, after Bonferroni correction) were removed as being likely to contain genotyping errors rather than real disequilibrium. SNPs for which no homozygotes were observed in the entire sample were also likely due to errors and removed. Altogether, of the 116,204 SNPs genotyped, 105,980 SNPs with a call rate of at least 85%, both alleles observed, at least one homozygote observed, and a HWD χ² ≤ 25 were found and considered. Of these, the 103,611 SNPs that lie on the 22 autosomal chromosomes were analyzed. A summary of genotyping quality can be found in Table 2.

**Table 2. Genotyping quality control and informativeness.**

| **Per-chip data quality** | |
|---|---|
| Median call rate per chip | 99.1 % |
| Minimum call rate per chip | 95.6% |
| Chips for which gender matches | 292 (100%) |

| **Per-individual data quality** | |
|---|---|
| Median call rate per individual | 99.1% |
| Minimum call rate per individual | 96.7% |
| Average number of matches for common SNPs between two chips | 30.7/31 |
| Minimum number of matches for common SNPs between two chips* | 28/31 |

| **Reproducibility** | |
|---|---|
| Xba Repeat concordance (4 replicates) | 99.886% |
| Xba Positive control concordance (2 replicates) | 99.870% |
| 10K concordance (3 replicates) | 99.767% |

| **Call rate (per-SNP)** | |
|---|---|
| Total number of SNPs | 116204 |
| SNPs with 100% call rate | 81456 |
| SNPs with call rate between 85% and 100% | 33262 |
| SNPs with call rate less than 85% | 1486 |

| **Locus Polymorphism** | |
|---|---|
| Number of SNPs with no polymorphism observed | 8538 |
| Number of SNPs with minor allele frequency < 0.01 | 3604 |
| Number of SNPs with only heterozygotes observed | 19 |
| Number of polymorphic SNPs with no heterozygotes observed | 71 |

| **Hardy-Weinberg Equilibrium** | |
|---|---|
| Number of SNPs significantly out of equilibrium | 231 |

| | |
|---|---|
| *For the most part, when SNPs do not match it is due to one of the SNPs not being called. In only 3 out of 4485 comparisons is a mismatch observed, which is equivalent to 99.93% concordance. | |

### EXAMPLE 3: Statistical analysis of SNP association with disease status

Allelic association with disease status was tested for each SNP. A 2x2 contingency table of allele frequencies was constructed. The Person χ² value and a P-value were calculated, based on the central χ² distribution under the null hypothesis of no association with 1 degree of freedom. This nominal P-value was corrected for multiple testing by applying the Bonferroni correction, in which only SNPs with a p-value less than 0.05/103,611 = 4.8x10⁻⁷ were considered. This produced a Bonferroni-corrected P-value This correction is known to be conservative and thus may "overcorrect" the raw p-values (L. M. McIntyre, E. R Martin, K. L. Simonsen, N. L. Kaplan, Genet Epidemiol 19, 18 (2000)). While this technique may overlook real associations, it adjusts for the large number of multiple comparisons and yields p-values that do not underestimate the false positive rate.

Two methods of genomic control were used to look for population stratification, GC and GCF (B. Devlin, S. A. Bacanu, K. Roeder, Nat Genet 36, 1129 (2004)). In the first method, the median χ² value was taken for allelic association with a number of SNPs assumed to be unassociated with the disease (null SNPs). The test statistic χ²₍₁₎ values were divided by this median, and tested for significance using the χ² distribution. Alternatively, for the GCF method, the mean rather the median of the null χ² statistics was used; significance of the quotient was tested using the F(1,L) distribution, where L is the number of null SNPs used (B. Devlin, S. A. Bacanu, K. Roeder, Nat Genet 36, 1129 (2004)). Two different sets of unassociated SNPs were used: all the SNPs successfully genotyped except the two significant (see below) ones, and the set of 31 SNPs that are in common between the two chips used in the assay (see Example 2 above).

The candidate region was defined by looking for adjacent SNPs in which all four gametes were observed (R. R. Hudson, N. L. Kaplan, Genetics 111, 147 (1985)) and bounding the region there. To look at linkage disequilibrium between SNPs in the candidate region, haplotype frequencies in the region were inferred using PHASE version 2.1 (M. Stephens, P. Donnelly, Am J Hum Genet 73, 1162 (2003); M. Stephens, N. J. Smith, P. Donnelly, Am J Hum Genet 68, 978 (2001)). Based on the inferred haplotype frequencies across the entire region, pairwise linkage disequilibrium was calculated by first computing the two-locus haplotype frequencies implied by the overall haplotype frequencies. The measure of linkage disequilibrium, D', was then calculated using standard equations (D. L. Hartl, A. G. Clark, Principles of Population Genetics (Sinauer Associates, Sunderland, MA, ed. Third, 1997)) and plotted using the program GOLD (G. R. Abecasis, W. O. Cookson, Bioinformatics 16, 182 (2000)).

To define the smaller haplotype blocks within the 4-gamete region, the HapMap data website was used (http://www.hapmap.org). Genotypes for SNPs in the region between SNPs rs 10494744 and rs 10484502 were downloaded. Genotypes for the CEU population (CEPH Utah population of northern and western European ancestry) were downloaded and visualized using Haploview version 3.0. Haplotype blocks were then defined using the method and parameters of Gabriel et al (S. B. Gabriel et al., Science 296, 2225 (2002)).

Haplotypes across the narrower region defined by the HapMap block were also inferred using PHASE version 2.1. Haplotypes with an estimated frequency of at least 1% were considered for further analysis. Phylogenetic trees were built using the maximum parsimony of PHYLIP 3.62 ("dnapars" program). The odds ratio in a nested cladistic framework was calculated for the haplotypes (P. Armitage, G. Berry, Statistical Methods in Medical Research (Blackwell Scientific Publications, Oxford, ed. Second, 1987); A. R. Templeton, E. Boerwinkle, C. F. Sing, Genetics 117, 343 (1987)).

Odds ratios, confidence intervals, and population attributable risk were calculated as described in P. Armitage, G. Berry, Statistical Methods in Medical Research (Blackwell Scientific Publications, Oxford, ed. Second, 1987). The population frequency of the alleles of interest (see Example 4 below) is relatively high, 23% for and 41 % for homozygous rs380390 and rs1329428, respectively. Therefore, the odds ratios necessarily calculated from the case control design study used here will over-estimate (without changing the significance levels) the equivalent relative risk estimate needed to calculate lifetime risk. A prospective cohort study design will provide valid estimates of lifetime risk in persons who have and have not inherited the alleles.

### EXAMPLE 4: Polymorphisms in the Complement Factor H gene are associated with AMD

Of the autosomal SNPs, only two, rs380390 and rs10272438, are significantly associated with disease status (Bonferroni corrected p=0.0043 and p=0.0080, respectively; Figure 1A). One criticism of case-control association studies such as this one is that population stratification can result in false positive results. If the cases and controls are drawn from populations of different ancestry, with different allele frequencies, it is possible to detect these population differences instead of loci associated with the disease. All individuals in this study self-identify their ethnicity as non-Hispanic white and all of the case and control individuals are drawn from the same AREDS population. There was some differential recruiting of cases front office practices and recruiting of controls from radio and newspaper advertising (AREDS Research Group, Ophthamology 107, 2224 (2000)). Finding two SNPs out of >100,000 implied no genetic Stratification, but genomic control methods were used to control for this possibility (B. Devlin, S. A. Bacanu, K. Roeder, Nat Genet 36, 1129 (2004)). It was consistently found that the significance of the tests was not inflated and that, therefore, these two SNPs are significantly associated with disease.

SNP rs38030 was successfully genotyped in all individuals. In 21 individuals, no genotype was determined for SNP rs 10272438, and it appears to be excessively out of Hardy-Weinberg equilibrium (IIWE χ²=36), indicating possible genotyping citors. Missing genotypes were determined by resequencing. After including these additional genotypes, the association was no longer significant after Bonferroni correction. Futhermore, the SNP with the third lowest p-value, rs1329428 (Bonferroni corrected p=0.14), is located 1.8 kb away from rs380390 on the same chromosome. The genotype frequencies at these two neighboring loci clearly vary between the case and control populations (Figure 1B). Homozygotes for the C allele of rs330390 and the C allele at rs1329428 clearly have an increased risk of developing AMD (Table1). The risk conferred by these genotypes accounts for approximately 45% (rs380390) to 61% (rs1329428) of the cases observed in the population (Table 3). Therefore, we decided to focus on these two sNPs as marking our most promising locus.

**Table 3. Risk ratios and population attributable risks for various genotypes and haplotypes.**

| | rs380390 (C/G) | rs1329428 (C/T) |
|---|---|---|
| Risk allele | C | C |
| Allelic Association χ² nominal p-value | 4.1e-08 | 1.4e-06 |
| Odds ratio (dominant) (95% CI) | 4.6 (2.0-11) | 4.7 (1.0-22) |
| PAR (95% CI) | 70% (42%-84%) | 80%(0%-96%) |
| Frequency in HapMap CEU | 0.70 | 0.82 |
| Odds ratio (recessive) (95% CI) | 7.4 (2.9-19) | 6.2 (2.9-13) |
| PAR (95% CI) | 46% (31%-57%) | 61% (43%-73%) |
| Frequency in HapMap CEU | 0.23 | 0.41 |

| | | |
|---|---|---|
| Dominant and recessive refer to the risk factor consisting of having at least one copy (dominant) or two copies (recessive) of the risk allele. PAR is the population atributable risk. The dominant odds ratio and PAR compare likelihood of AMD in individuals with one copy of the risk allele versus individuals with no copy of the risk allele. The recessive odds ratio and PAR compare likelihood of AMD. in individuals with two copies of the risk allele versus individuals with no more than one copy of the risk allele. The population frequencies for the risk genotypes are taken from the CEU HapMap population (CBPH collection of Utah residents of northern and western European ancestry). | | |

rs380390 and rs1329428 lie in an intron of the gene for complement factor H (CFH). As both of these SNPs are noncoding and neither appears to alter a conserved sequence, these two SNPs may be in linkage disequilibrum with a corresponding functional mutation. To circumscribe the region in which the functional mutation may lie, the linkage disequilibrium throughout this region was analyzed (Figure 2A). The two associated SNIPS lie in a region of high linkage disequilibrium that is around 500 kb long. As this region is longer than other typically observed blocks of high linkage disequilibrium (S. B Gabriel et al., Science 296, 2225 (2002)) and there are long stretches in this region where there are no SNIPS in our dataset (Figure 2B), other data sources with denser SNP coverage were used to narrow down the region.

Data from the International HapMap project was used to analyze patterns of linkage disequilibrium in a population of residents of Utah with ancestry from northern and western Europe (the CEPH sample) (The International HapMap Consortium, Nature 426, 789 (2003)). In the 500 kb region of interest, there are 152 SNPs in the HapMap data set. Using a standard definition of linkage disequilibrium blocks (S. B Gabriel et al., Science 296, 2225 (2002)), it was found that the two associated SNPs lie in a block that is 41 kb long and entirely contained within the CFH gene (Figure 2C).

There are six SNPs from the present study's data set in this 41 kb region. These SNPs form four predominant haplotypes with a frequency greater than 1% (Table 4). Combined, these four haplotypes represent 99% of the chromosomes in this study. Reconstructing inferred haplotypes and building a phylogenetic tree allowed assessment of the evolutionary relationship between haplotypes (Figure 2D). Using inferred haplotypes for each individual, the odds ratio of disease in a nested cladistic framework under both dominant and recessive models were computed (A. R Templeton, E. Boerwinkle, C. F. Sing, Genetics 117, 343 (1987)). The highest risk is conferred by haplotype N1, which is the only haplotype containing the risk allele at SNP rs380390.

**Table 4. Haplotypes in the haplotype block that harbors the putative disease variant**

| Name | rs2019727 | rs10489456 | rs3753396 | **rs380390** | rs2284664 | **rs1329428** | Frequency |
|---|---|---|---|---|---|---|---|
| N1 | A | C | T | C | C | G | 0.59 |
| N2 | A | C | T | G | C | G | 0.0068 |
| N3 | A | C | T | G | T | A | 0.12 |
| N4 | A | T | C | G | C | G | 0.15 |
| N5 | T | C | T | G | C | A | 0.12 |
| N6 | T | C | T | G | C | G | 0.0071 |

| | | | | | | | |
|---|---|---|---|---|---|---|---|
| Haplotype frequencies are estimated using the program PHASE (M. Stephens, P. Donnelly, Am J Hum Genet 73, 1162 (2003); M. Stephens, N. J. Smith, P. Donnelly, Am J Hum Genet 68, 978 (2001)). The SNPs used to construct the haplotypes are the SNPs from the mapping microarrays found in the 41 kb haplotype block defined by the HapMap data. Frequencies are the estimated frequency of each haplotype in the combined case and control population. The two SNPs that show association in the initial analysis are indicated in boldface. | | | | | | | |

Having at least one copy of this haplotype increases the risk for AMD 4.6-fold (95% CI 2.0-11). Having two copies of this haplotype increases the risk for AMD 7.4-fold (95% CI 3.0-19). Therefore, functionally relevant mutation should be found in the context of haplotype N1. This mutation will occur somewhere in the CFH gene, as the 41kb haplotype block is entirely within CFH.

### EXAMPLE 5: Resequencing confirms that variations in CFH are correlated with AMD

To identify the functional mutation underlying susceptibility to AMD, 96 individuals (66 cases and 30 controls) were chosen for exonic resequencing, including the exon/intron junctions. Most of these individuals were selected either because SNP rs380390 was homozygous (representing opposite risk groups) or SNP rs10272438 was not successfully genotyped (the same plates were used to re-sequence this SNP for genotyping). Three additional individuals were randomly selected to get a total of 96 for a full plate. Primer design, PCR amplification, bi-directional sequencing of PCR products, and mutation analyses were performed by Genaissance (New Haven, CT).

All CFH exons were sequenced, including those outside of the 41kb block, as well as the region of SNP rs380390 as a control. Priority was given to sequencing homozygotes at SNP rs380390 to make it easier to determine haplotypes. SNP rs380390 was successfully resequenced in 93 individuals; the genotype derived from resequencing matched the original genotype in all cases. A total of 50 polymorphisms were identified; 17 of these have a minor allele frequency of at least 5% (**Table 5**). Of these 17, three represent non-synonymous mutations. If these SNPs are ranked based on the allelic association χ² measure, SNP rs1061170 is the most associated among the non-synonymous SNPs. This SNP represents a mutation between tyrosine and histidine. This SNP is located in exon 9 of CFH, only 2 kb upstream of the 41 kb haplotype block. Adding this SNP to the haplotype analysis reveals that 97% of the chromosomes with the highest-risk haplotype (N1) also have the risk allele (His).

**Table 5. New polymorphisms identified through resequencing.**

| Region | Position | Change | Type | MAF | AA Change | rs Number |
|---|---|---|---|---|---|---|
| promoter | 120992 | A/G | noncoding | 0.005263 | | |
| promoter | 120865 | A/G | noncoding | 0.010526 | | |
| promoter | 120546 | C/T | noncoding | 0.242105 | | rs3753394 |
| promoter | 120410 | T/C | noncoding | 0.005263 | | |
| promoter | 120294 | A/G | noncoding | 0.005263 | | |
| intron 1 | 99391 | C/T | noncoding | 0.117021 | | rs511397 |
| exon 2 | 99242 | T/G | nonsynonomous | 0.005319 | Ser 58 Ala | |
| exon 2 | 99230 | G/A | nonsynonomous | 0.117021 | Val 62 Ile | rs800292 |
| intron 2 | 99114 | G/A | noncoding | 0.005319 | | |
| intron | 98283 | T/C | noncoding | 0.005263 | | |
| intron 3 | 98188 | T/G | noncoding | 0.005263 | | |
| exon 4 | 96315 | G/A | nonsynonomous | 0.005263 | Arg 127 His | |
| exon 7 | 87139 | A/C | synonomous | 0.415789 | | rs1061147 |
| intron 7 | 83059 | T/C | noncoding | 0.005263 | | |
| intron 7 | 82966 | G/T | noncoding | 0.410526 | | rs482934 |
| intron 7 | 82957 | A/G | noncoding | 0.005263 | | |
| exon 9 | 82232 | C/A | nonsynonomous | 0.005208 | Gln 400 Lys | |
| exon 9 | 82226 | C/T | nonsynonomous | 0.414894 | His 402 Tyr | rs1061170 |
| intron 9 | 58652 | T/C | noncoding | 0.005319 | | |
| exon 10 | 58516 | G/A | synonomous | 0.22043 | | rs2274700 |
| intron 10 | 58319 | A/G | noncoding | 0.005319 | | rs203678 |
| intron 10 | 58260 | C/G | noncoding | 0.005319 | | |
| intron 10 | 56838 | G/T | noncoding | 0.367021 | | rs203674 |
| exon 12 | 47084 | G/A | nonsynonomous | 0.005263 | Val 609 Ile | |
| intron 12 | 46992 | T/G | noncoding | 0.005208 | | |
| exon 13 | 45721 | A/G | synonomous | 0.143617 | | rs3753396 |
| exon 15 | 43875 | A/G | synonomous | 0.005376 | | |
| intron 15 | 40549 | A/G | noncoding | 0.215054 | | rs7514261 |
| intron 15 | 40445 | C/T | noncoding | 0.021277 | | |
| intron 15 | 40412 | G/C | noncoding | 0.365591 | | rs380390 |
| intron 15 | 40335 | G/C | noncoding | 0.005319 | | rs380060 |
| intron 15 | 40179 | C/T | noncoding | 0.215054 | | rs7540032 |
| intron 15 | 35577 | T/G | noncoding | 0.005208 | | rs435628 |
| intron 15 | 35537 | C/A | noncoding | 0.357895 | | rs375046 |
| intron 16 | 35263 | C/T | noncoding | 0.005263 | | rs428060 |
| exon 17 | 34821 | C/T | synonomous | 0.026316 | | |
| exon 17 | 34786 | G/T | nonsynonomous | 0.005263 | Ser 890 Ile | rs515299 |
| intron 17 | 31825 | A/C | noncoding | 0.005319 | | |
| exon 18 | 31689 | G/T | nonsynonomous | 0.154255 | Glu 936 Asp | rs1065489 |
| intron 18 | 30673 | T/G | noncoding | 0.005556 | | rs385892 |
| intron 18 | 30547 | T/C | noncoding | 0.111702 | | rs16840522 |
| intron 18 | 30546 | A/G | noncoding | 0.005319 | | rs385543 |
| exon 19 | 30396 | G/T | nonsynonomous | 0.005319 | Val 1007 Leu | rs534399 |
| intron 19 | 28886 | T/C | noncoding | 0.154255 | | rs513699 |
| exon 20 | 28877 | C/T | synonomous | 0.154255 | | rs513729 |
| exon 20 | 28867 | A/T | nonsynonomous | 0.015957 | Asn 1050 Tyr | |
| intron 20 | 28592 | A/G | noncoding | 0.012987 | | |
| intron 20 | 26589 | G/C | noncoding | 0.005618 | | |
| exon 22 | 25219 | C/A | nonsynonomous | 0.005556 | Pro 1166 Gln | |
| exon 22 | 25088 | C/T | nonsynonomous | 0.005618 | Arg 1210 Cys | |

| | | | | | | |
|---|---|---|---|---|---|---|
| Location of each polymorphism refers to the position on GenBank accession AL049744.8 (SEQ ID NO: 9), or the complementary DNA strand of GenBank accession AL049744.8. MAF is minor allele frequency. | | | | | | |

Other data support the finding that mutations in CFH are correlated with AMD. The gene for CFH is located on chromosome 1q31, a region that had been previously identified by six independent linkage scans to be involved in AMD (J. Majewski et al., Am J Hum Genet 73, 540 (2003); J. M. Seddon, S. L. Santangelo, K. Book, S. Chong, J. Cote, Am J Hum Genet 73, 780 (2003); D. E. Weeks et al., Am J Hum Genet 75, 174 (2004); G. R. Abecasis et al., Am J Hum Genet 74, 482 (2004); S. K. Iyengar et al., Am J Hum Genet 74, 20 (2004);and D. W. Schultz et al., Hum Mol Genet 12, 3315 (2003)). In one of these linkage studies, using a single large pedigree the authors concluded that mutations in a different gene in this region (HEMICENTTN-1), was responsible for AMD (D. W. Schultz et al., Hum Mol Genet 12, 3315 (2003)). This conclusion was based on the observation that of all the polymorphisms tested, only the HEMICENTIN-1 mutation perfectly cosegregated with disease status. Mutations in HEMICENIZN-1, however, have not been found to be generally associated with AMD in three separate, independent population-based association studies (G. R. Abecasis et al., Am J Hum Genet 74, 482 (2004); M. Hayashi et al., Ophthalmic Genet 25, 111 (2004); and G. J. McKay et al., Mol Vis 10, 682 (2004)). Mutations in CFH, as disclosed herein, are therefore more likely to be the cause of linkage signals observed at chromosome 1q31.

### Example 6: Immunolocalization of C5b-9 complex in the eyes of patients suffering from AMD

Various components of the complement cascade, including the terminal C5b-9 complex, have been identified as components of drusen in the eyes of patients with AMD (L. V. Johnson, W. P. Leitner, M. K. Staples, D. H. Anderson, Exp Eye Res 73, 887 (2001); R F. Mullins, S. R. Russell, D. H. Anderson, G. S. Hageman, FASEB J 14, 835 (2000)). The eyes of four patients with AMD were examined to look for the presence of C5b-9 (Figure 4). Post-mortem retinas from four donors were examined. Three were obtained through the Foundation Fighting Blindness (FFB) eye donor program. All of these had a clinical diagnosis of dry AMD. One pair of eyes embedded in paraffin was obtained from an 86 year old Caucasian female through the autopsy service of the Yale School of Medicine. No clinical history was available. Histologically, these retinas have multiple large or coalescing drusen with minimal RPE and photoreceptor loss consistent with a diagnosis of early AMD. Approval for research on human post mortem donor eyes was obtained from the Yale School of Medicine.

Upon enucleation, eyes were fixed in 4% paraformaldehyde, 0.5% glutaraldehyde in 0.1 M phosphate buffer for several days. The fixed eyes were transferred to 2% paraformaldehyde for storage. Six 0.5 cm circular punches were taken from each of the AMD donor eyes. Three of these were selected from the central retina at the junction of atrophic and more normal retina, and the remaining three from peripheral retina. Retinal plugs were embedded in paraffin and sections cut at 5 µm.

Following deparaffinization and rehydration, antigen retrieval was performed by boiling sections in a microwave oven for 10 minutes in 10 mM sodium citrate (pH 6.0). Sections were allowed to cool for 20 minutes, prior to a 5-minute endogenous peroxidase block in 5% H₂O₂. Immunohistochemistry was performed using a mouse monoclonal antibody against human activated complement C5b-9 (Quidel Corporation, San Diego, CA, catalogue #A239). Primary antibody was applied at a concentration of 1:250 in 1X PBS. Biotinylated goat anti-mouse (cat # BA9200) secondary antibody (Vector, Burlingame, CA) was used at a concentration of 1:200. Nickel enhanced diaminobenzidine (DAB; cat # SK4100; Vector) was used to visualize bound antibody. Negative controls were obtained by omission of the primary antibody. Images were obtained with a Zeiss Axioplan microscope equipped with differential interference contrast lenses and a Zeiss Axiocam digital camera.

### Immunofluorescence Microscopy for CFH

Donor eyes were embedded in optimal cutting temperature compound (OCT; Miles Laboratory, Elkhart, IN), snap frozen, and stored at -70°C. Frozen retina sections were cut at 8 to 10 µm and placed on slides (Superfrost/Plus; Fisher Scientific, Fair Lawn, NJ). All human eyes were obtained with the informed consent of the donors, and the research with human eyes was performed in accordance with the tenets of the Declaration of Helsinki and the Institutional Review Board (IRB).

For immunofluorescence labeling, frozen sections of human retina were fixed in 4% paraformaldehyde in phosphate buffer saline (PBS) for 10 minutes. The tissue sections were blocked for 30 minutes with 5% normal donkey serum (Jackson Immunoresearch, West Grove, PA), diluted in IC buffer (PBS, containing 0.2% Tween-20, 0.1% sodium azide), and incubated for 1 hour at room temperature with a goat anti-human Factor H antibody (Quidel, Santa Clara, CA) diluted 1:200 in staining buffer (IC buffer plus 1% normal donkey serum). Sections were washed repeatedly in IC buffer and incubated for 1 hour with the nuclear dye 4',6'-diamino-2 phenylindole (DAPI; 1 µg/mL) and Alexa-488 Donkey anti-goat antibodies (Molecular Probes, Eugene, OR) diluted 1:250 in staining buffer. After repeated washing with IC buffer, sections were covered in mounting medium (Gel Mount; Biomeda, Foster City, CA) and coverslipped. For the control, the same concentration of anti-human factor H antibody was preincubated for 1 hour with purified human factor H protein (Calbiochem, La Jolla, CA) at the ratio of 3 µg for 1 µl of antibodies. The pretreated antibodies were then used to stain tissue sections as just described. Specimens were analyzed on a laser scanning confocal microscope (model SP2; Leica Microsystems, Exton, PA) equipped with Nomarski optics. Immunolabeled and negative control sections were imaged under identical scanning conditions. Images were processed with Photoshop (Adobe Systems, San Jose, CA).

In all patients, deposition of activated complement C5b-9 was noted in Bruch's membrane. Immunostaining frequently extended to include the intercapillary pillars, and was strongly present within drusen. Staining was rarely noted in the stroma vascularis. However, when it was present, it was invariably located in the inner (toward the retina) walls of choroidal veins, and in severe cases, arteries. No immunostaining for C5b-9 was noted in the retina or elsewhere in sections. The negative control failed to exhibit any staining. These and other biochemical analyses of the composition of drusen may indicate that AMD results from an aberrant inflammatory process in which inappropriate complement activation plays a role (G. S. Hageman et al., Prog Retin Eye Res 20, 705 (2001)). This is supported by a mouse model of AMD in which complement components are found in the drusen (J. Ambati et al., Nat Med 9, 1390 (2003)).

Moreover, both age and smoking, two significant risk factors for AMD, influence plasma levels of complement factor H (J. Esparza-Gordillo et al., Immunogenetics 56, 77 (2004)). CFH sequences have also been observed in an EST library derived from human RPE and choroid (G. Wistow et al., Mol Vis 8, 205 (2002)). Immunofluorescence experiments confirm that CFH is present in this region of the eye (Figure 3). The fluorescent images and their corresponding DIC images obtained from two different areas of human retina sections show strong staining in choroid vessels and area close to RPE (likely to be underneath the Bruch's membrane) (Figure 3). This finding is consistent with the observation that the RPE and choroid produce mRNA for several complement components found in drusen (R. F. Mullins, S. R. Russell, D. H. Anderson, G. S. Hageman, FASEB J 14, 835 (2000)). Drusen of similar composition to that found in AMD are found in the eyes of patients with membranoproliferative glomerulonephritis type II (MPGNII), a kidney disease (R. F. Mullins, N. Aptsiauri, G. S. Hageman, Eye 15, 390 (2001)); factor H deficiency can cause MPGNII (S. R. D Cordoba, J. Esparza-Gordillo, E. G. d. Jorge, M. Lopez-Trascasa, P. Sanchez-Corral, Mol Immunol 41, 355 (2004)). Our immunostaining experiments (Figures 3 and 4) suggest a pathogenesis of AMD in which loss, impairment, or deficiency of factor H results in complement deposition in choroidal capillaries (more severe) and choroidal vessels (less severe) with subsequent leakage of plasma proteins in to Bruch's membrane. Finally, nutritional supplementation with zinc at 80 mg/day decreases the risk of AMD; biochemical studies have shown that factor H function in sensitive to zinc concentration (AREDS Research Group, Arch Ophthamol 119, 1417, (2001); A. M. Blom, L. Kask, B. Ramesh, A. Hillarp, Arch Biochem Biophys 418, 108 (2003)).

The present invention provides among other things polynucleotides useful for identifying or aiding in identifying individuals at risk for developing AMD, as well as for diagnosing or aiding in the diagnosis of AMD. While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations. ,

**Table 6. Primer sequences used in resequencing.**

| **Region** | **Forward primer sequence** | **Reverse primer sequence** |
|---|---|---|
| promoter | AGAATCGTGGTCTCTGTGTGTGG | AGCAGCTGGTGATATCCTCTGG |
| promoter | TCAAATGAGAGTGAGCCAGTTGC | CTGTTCACAACGTCCAGTTCTCC |
| exon 1 | GTGGGAGTGCAGTGAGAATTGG | AACTCAACAATGTCAAAAGCC |
| exon 2 | GATAGACCTGTGACTGTCTAGGC | GGCAATAGTGATATAATTCAGGC |
| exon 3 | ACCTCAGCCTCCCAAAGTGC | TGCATACTGTTTTCCCACTCTCC |
| exon 4 | AAGGAGGAGGAGAAGGAGGAAGG | CAGGCTGCATTCGTTTTTGG |
| exon 5 | CCACTCCCATAGAAAAGAATCAGG | ACTTCTTTGCACCAGTCTCTTCC |
| exon 6 | GATAAATCATTTATTAAGCGG | GAACCTTGAACACAGAAAATGC |
| exon 7 | GGATGAGTITGGAGAAGAAGG | TATGAGTTTCGGCAACTTCG |
| exon 8 | TCATCTTCATTAACAAAGACC | AGATCTATTTTGGTCACTTTGC |
| exon 9 | C1TTGTTAGTAACTTTAGTTCG | TTATACACAGTRGAAAAACC |
| exon 10 | GGCAACTCTGAGCTTATTTTCC | AGAGTAGGAAAAGCCTGAATGG |
| exon 11 | CATAGATTATTTTTGTACGG | CAAAACTCCCTTCTTTTCCC |
| exon 12 | ATCTGATGCCCCTCTGTATGACC | ATTCAGTACTCAATACATGTCC |
| exon 13 | CACCATTCTTGATTGTTTAGG | GAATCTCCATAGTAATAAGG |
| exon 14 | CAATGTGTTGATGGAGAGTGG | ATTGAATTATAAGCAATATGC |
| exon 15 | CATTTCAGCGACAGAATACAGG | GTGTGTGTGTGTGTGTGTGC |
| intron 15 | AAGGCAGGAAAGTGTCCTTATGC | GTCAAATTACTGAAAATCACC |
| exon 16 | AACTGTTACACAGCTGAAAAG | GTGGTGATTGATTAATGTGC |
| exon 17 | GGTGGAGGAATATATCTTTGC | ATAGAATAGATTCAATCATGC |
| exon 18 | CGATAGACAGACAGACACCAGAAGG | CAGCTATAATITCCCACAGCAGTCC |
| exon 19 | GTGTAATCTCAATTGCTACGGCTACC | CAAGTAGCTGGGACTTCAGATGC |
| exon 20 | TAGTTTCATGTCTTTTCCTC | GAATTTTAAGCACCATCAGTC |
| exon 21 | CCAGGACTCATTTCTTTCACC | CTTTCTGACAGAAATATTTGG |
| exon 22 | TGATGTTTCTACATAGTTGG | GGAGTAAAACAATACATAAAAAATG |

**Table 7. Variations in CFH identified through resequencing that may be correlated with the occurrence of AMD. Each variation is shown in the context of its surrounding DNA sequence. Location of each variation refers to the position on GenBank accession AL049744.8, or the complementary DNA strand of GenBank Accession AL049744.8.**

| **Region** | **Position** | **Common/Common** | **Common/Rare** | **Rare/Rare** | **Log HW P-value** | **Change** | **Sequence Context** |
|---|---|---|---|---|---|---|---|
| promoter | 120992 | 94 | 1 | 0 | 0 | A/G | GTACTGGGGTTTTCTGGGATGTAAT [A/G] ATGTTCAGTGTTTTGACCTTGGTGG |
| promoter | 120865 | 94 | 0 | 1 | -2.2764618 | A/G | ACAAAGTTTTAAAAATCAGCATTTC [A/G] ATTTOTTGATTTTTGGATTATTAAA |
| promoter | 120546 | 57 | 30 | 8 | -0.7719879 | C/T | AGOOTTTATGAAATCCAGAGGATAT [C/T] ACCAGCTGCTGATTTGCACATACCA |
| promoter | 120410 | 94 | 1 | 0 | 0 | T/C | GAGTGCAGTGAGAATTGGGTTTAAC [T/C] TCTGGCATTTCTGGGCTTGTGGCTT |
| promoter | 120294 | 94 | 1 | 0 | 0 | A/G | TTTGCAGCAAGTTCTTTCCTGCACT [A/G] ATCACAATTCTTGGAAGAGGAGAAC |
| intron 1 | 99391 | 72 | 22 | 0 | 0.4512837 | C/T | TAAATATACTGTACATTTAAATAGA [C/T] ACTTTATGCACTTATTTTGTTTTTA |
| exon 2 | 99242 | 93 | 1 | 0 | 0 | T/G Ser 58 Ala | CTATAAATGCCGCCCTGGATATAGA [T/G Ser 58 Ala] CTCTTGGAAATGTAATAATGGTATG |
| exon 2 | 99230 | 72 | 22 | 0 | 0.4512837 | G/A Val 62 Ile | CCCTGGATATAGATCTCTTGGAAAT [G/A Val 62 Ile] TAATAATGGTATGCAGGAAGGGAGA |
| intron 2 | 99114 | 93 | 1 | 0 | 0 | G/A | GAAAACTAGGTGTAAAAATACTTAA [G/A] ATTTAATATTGTAGCAATTATGCCT |
| intron 2 | 98485 | 75 | 20 | 0 | 0.2285278 | -/TT() | CATACTAATTCATAACTTTTTT [-/TT/()] CGTTTTAGAAAGGCCCTGTGGACAT |
| intron 3 | 98283 | 94 | 1 | 0 | 0 | T/C | ATATATTTTCAAGGTTATTATATTT [T/C] TCTATGAGCATTTAAAAAAGTAATA |
| intron 3 | 98188 | 94 | 1 | 0 | 0 | T/G | GGATACCATATTATCTCCTTAACAT [T/G] GAAAAATTTAAATGAAGTATAACTT |
| exon 4 | 96315 | 94 | 1 | 0 | 0 | G/A Arg 127 His | CAATTGCTAGGTGAGATTAATTACC [G/A Arg 127 His] TGAATGTGACACAGATGGATGGACC |
| intron 4 | 96211 | 94 | 1 | 0 | 0 | -/T/() | AATAAATATCTAAGATTTAAAAAAA [-/T/()] GTCTTACATTAAAATATCT'iAAAGT |
| exon 7 | 87139 | 46 | 19 | 30 | -7.9849797 | A/C (C) Ala 307 Ala | ATCCTGCAACCCGGGGAAATACAGC [A/C (C) Ala 307 Ala] AAATGCACAAGTACTGGCTGGATAC |
| intron 7 | 83071 | 94 | 1 | 0 | 0 | -/ATGAGATATAGAA/ () | AGACCTTCTTGTTACATATCTCAGT [-/ATGAGATATAGAA/ ()] CATCTGAGTTCTATCATTTGTTTTG |
| intron 7 | 83059 | 94 | 1 | 0 | 0 | T/C | TTACATATCTCAGTCATCTGAGTTC [T/C] ATCATTTGTTTTGACCTAGAAACCC |
| intron 7 | 82966 | 48 | 16 | 31 | -10.039955 | G/T | TGATAAAAATTTATCTCTAATATGA [G/T] TGTTTATTACAGTAAAATTTCTTTA |
| intron 7 | 82957 | 94 | 1 | 0 | 0 | A/G | TTTATCTCTAATATGAGTGTTTATT [A/G] CAGTAAAATTTCTTTATACTTTTTT |
| exon 9 | 82232 | 95 | 1 | 0 | 0 | C/A Gln 400 Lys | TCCTTATTTGGAAAATGGATATAAT [C/A Gln 400 Lys] AAAATCATGGAAGAAAGTTTGTACA |
| exon 9 | 82226 | 46 | 18 | 30 | -8.6058781 | C/T His 402 Tyr | TTTGGAAAATGGATATAATCAAAAT [C/T] His 402 Tyr] ATGGAAGAAAGTTTGTACAGGGTAA |
| intron 9 | 58652 | 93 | | 1 0 | 0 | T/C | TATATTTACATATTACTTAAATTCT [T/C] ATAAAATGTTATTGATCATATGCTT |
| exon 10 | 58516 | 59 | 27 | 7 | -0.8677698 | G/A Ala 473 Ala | ATACATATGCCTTAAAAGAAAAAGC [G/A Ala 473 Ala] AAATATCAATGCAAACTAGGATATG |
| intron 10 | 58319 | 93 | 1 | 0 | 0 | A/G | TGGGGGCTGATATAATTTCATTTGA [A/G] AAGATAAGAAAAAAAAACCTGCAGG |
| intron 10 | 58260 | 93 | 1 | 0 | 0 | C/G | AGACATCAATTTTTTTTCCTTTTCA [C/G] ATTAATTACTCAGATATTAGTCTGT |
| intion 10 | 56838 | 54 | 11 | 29 | -13.007209 | G/T | TTTGTACGGTACCTATTTATTAGTA [G/T] ATCTAATCAATAAAGCTTTTTCTTC |
| exon 12 | 47084 | 94 | 1 | 0 | 0 | G/A Val 609 Ile | ATTTACAATAGTTGGACCTAATTCC [G/A VaL 609 Ile] TTCAGTGCTACCACTTTGGATTGTC |
| intron 12 | 46992 | 95 | 1 | 0 | 0 | T/G | ATTGCTGAAATAAGAATTAGAACTT [T/G] GAATACCAACTITfITCTTATTAAT |
| exon 13 | 45721 | 71 | 19 | 4 | -1.0457792 | A/G Gln 672 Gln | TAATGAAGGGACCTAATAAAATTCA [A/G Gln 672 Gln] TGTGTTGATGGAGAGTGGACAACTT |
| exon 15 | 43875 | 92 | 1 | 0 | 0 | A/G Gly 783 Gly | CTAACATAAGGTACAGATGTAGAGG [A/G Gly 783 Gly] AAAGAAGGATGGATACACACAGTCT |
| intron 15 | 40549 | 60 | 26 | 7 | -0.9218916 | G/A | AATCTAGAATTATTCCTTGGCAGTT [G/A] TTTTCTTTCAGAATTTTGAGTATAT |
| intron 15 | 40445 | 90 | 4 | 0 | 0 | C/T | CTTGTGGAAATTCCATTTTATGTAA [C/T] CATTCACTTTTCATTGGCTTTTTTC |
| intron 15 | 40412 | 54 | 10 | 29 | -13.609694 | G/C | TTTTCATTGGCTTTTTTCAATACTT [G/C] GTCTATAACTTTTGATAATTTGATT |
| intron 15 | 40335 | 93 | 1 | 0 | 0 | G/C | TCATTAAACTTATTTGATTTCCTTT [G/C] AGATTTCTGGGTGTGGGTTTCTATT |
| intron 15 | 40179 | 60 | 26 | 7 | -0.9218916 | C/T | CCACATGGTAGTATTCCATCTGGAT [C/T] TTAAGCTATCTTCACTTTTATTTAT |
| intron 15 | 35577 | 95 | 1 | 0 | 0 | T/G | CATATAAATTATTTTTCATCAAAAA [T/G] TCTAATTTTAATATTTTTATTTTTT |
| intron 15 | 35537 | 55 | 12 | 28 | -12.229741 | C/A | TTTTATTTTTTATTTTTTATTATAA [C/A] ATTAATTATATTTTTAATATTTTTT |
| intron 16 | 35263 | 94 | 1 | 0 | 0 | C/T | ATGAGGTTAATATTCTCTTGTGCTT [C/T] GTGTAAACAAGAGAGAAGTTCTTTC |
| exon 17 | 34821 | 90 | 5 | 0 | 0 | C/T His 878 His | GTTCACAACCACCTCAGATAGAACA [C/T His 878 His] GGAACCATTAATTCATCCAGGTCTT |
| exon 17 | 34786 | 94 | 1 | 0 | 0 | G/T Ser 890 Ile | AATTCATCCACGTCTTCACAAGAAA [G/T Ser 890 Ile] TTATGCACATGGCACTAAATTGAGT |
| inron 17 | 31825 | 93 | 1 | 0 | 0 | A/C | ATTTGTGTTACTTCTCTGTGATGTC [A/C] TAGTAGCTCCTGTATTGTTTATTTT |
| exon 18 | 31689 | 70 | 19 | 5 | -1.4115003 | G/T Glu 936 Asp | GCCTTCCTTGTAAATCTCCACCTGA [G/T Glu 936 Asp] ATTTCATGGTGTTGTAGCTCACA |
| intron 18 | 30673 | 89 | 1 | 0 | 0 | T/G | GCTACGGCTACCAATATTTCTTCAG [T/G] CTTCTAATATCATTTCTATCTTGTA |
| intron 18 | 30547 | 78 | 11 | 5 | -2.9065654 | T/C | TGTTGTACAGTATTCATTGATTCTA [T/C] ATATCGCTATTTTAGAATCCATTAC |
| intron 18 | 30546 | 93 | 1 | 0 | 0 | A/G | GTTGTACAGTATTCATTGATTCTAT [A/G] TATCGCTATTTTAGAATCCATTACA |
| exon 19 | 30396 | 93 | 1 | 0 | 0 | G/T Val 1007 Leu | CATACCCATGGGAGAGAAGAAGGAT [G/T Val 1007 Leu] TGTATAAGGCGGGTGAGCAAGTGAC |
| intron 19 | 28886 | 65 | 29 | 0 | 0.9350138 | T/C | GGTGGAACCACTTCTTTTTTTTCTA [T/C] TCAGACACCTCCTGTGTGAATCCGC |
| exon 20 | 28877 | 65 | 29 | 0 | 0.9350138 | C/T Thr 1046 Thr | ACTTCTTTTTTTTCAGACAC [C/T Thr 1046 Thr] TCCTGTGTGAATCCGCCCACAGTAC |
| exon 20 | 28867 | 91 | 3 | 0 | 0 | A/T Asn 1050 Tyr | TTTCTATTCAGACACCTCAGTGTG [A/T Asn 1050 Tyr] ATCCGCCCACAGTACAAAATGCTTA |
| intron 20 | 28592 | 75 | 2 | 0 | 0 | A/G | AATAGATTTTTCAAATGCAAATAAA [A/G] TGACTGATGGTGCTTAAAATTCAAT |
| intron 20 | 26589 | 88 | 1 | 0 | 0 | G/C | TGATATTATATACAGTGCTGTGTTT [G/C] CGTTTGCCTTATTTGAACTTGTATT |
| exon 22 | 25219 | 89 | 1 | 0 | 0 | C/A Pro 1166 Gln | GTTTACTGTTTTTTATTTTCAGATC [C/A Pro 1166 Gln] GTGTGTAATATCCCGAGAAATTATG |
| exon 22 | 25088 | 88 | 1 | 0 | 0 | C/T Arg 1210 Cys | TAAACGGGGATATCGTCTTTCATCA [C/T Arg 1210 Cys] GTTCTCACACATTGCGAACAACATG |

**Table 8. Variations in CFHL1 that may be correlated with the occurrence of AMD. Each variation is shown in the context of its surrounding DNA sequence. Location of each variation refers to the position on GenBank Accession AL049741.7, or the complementary DNA strand of GenBank Accession AL049741.7.**

| **Region** | **Position** | **Common/Common** | **Common/Rare** | **Rare/Rare** | **Log HW P-value** | **Change** | **Sequence Context** |
|---|---|---|---|---|---|---|---|
| promoter | 24634 | 49 | 9 | 22 | -10.77769145 | A/G | AAATACCCATTCTCAAAGTCCCATC [A/G] GAACAAAATTATTTTGAAGTAAAAT |
| promoter | 24630 | 57 | 24 | 2 | 0 | C/G | ACCCATTCTCAAAGTCCCATCAGAA [C/G] AAAATTATTTTGAAGTAAAATTTGT |
| promoter | 24620 | 50 | 9 | 24 | -11.71118554 | T/C | AAAGTCCCATCAGAACAAAATTATT [T/C] TGAAGTAAAATTTGTTCAACAATTT |
| promoter | 24607 | 49 | 8 | 22 | -11.37722688 | T/G | AACAAAATTATTTTGAAGTAAAATT [T/G] GTTCAACAATTTTGGGAACCATTAC |
| promoter | 24558 | 74 | 2 | 0 | 0 | G/T | ACATACCAAAAATTATTCTTGATTT [G/T] ACTTTTTATAGTCTAAAAATATGAA |
| promoter | 24543 | 49 | 6 | 20 | -11.82719892 | -/C/() | TCTIGAITTGACTITITATAGTCTA [-/C/()] AAAATATGAAAACTATTAAGAAGTT |
| promoter | 24482 | 68 | 19 | 5 | -1.372873106 | C/T | TTTTTTTTTTTTTTTTTTTTTGAGA [C/T] GGAGTCTCGCTCTGTCACCCTGGCT |
| promoter | 24445 | 74 | 19 | 0 | 0.229044145 | G/A | CTGTCACCCTGGCTGGAGGGGAGTG [G/A] TGCGATCTCAGCTCACTGCGAACTC |
| promoter | 24426 | 68 | 20 | 5 | -1.259964884 | C/T | GGAGTGGTGCGATCTCAGCTCACTG [C/T] GAACTCCGCCTCCCGAGTTCACGCC |
| promoter | 24412 | 74 | 19 | 0 | 0.229044145 | C/T | TCAGCTCACTGCGAACTCCGCCTCC [C/T] GAGTTCACGCCATTCTCCTGCCTCA |
| promoter | 24404 | 74 | 12 2 | 1 | -0.363372133 | C/T | CTGCGAACTCCGCCTCCCGAGTTCA [C/T] GCCATTCTCCTGCCTCAGCCTCCCA |
| promoter | 24303 | 80 | 14 | 0 | 0 | T/G | TTTCAGTAGAGATGGGGTTTCACCA [T/G] GTTAGCCAGGATGGTCTGAAGTTAC |
| promoter | 24182 | 74 | 19 | 1 | 0 | C/T | CTGATCACCTTCACTTGCTTGCCTA [C/T] TGATGTAGCTGAACTCTTGGCTAGA |
| promoter | 24141 | 92 | 1 | 0 | 0 | C/T | CTTGGCTAGAAAAAAGAAGGGGCTT [C/T] CTCTTTCCTCTTCAATGGCCCATTT |
| exon 1 | 23873 | 93 | 1 | 0 | 0 | C/G | TCATGCTCATAACTGTTAATGAAAG [C/G] AGATTCAAAGCAACACCACCACCAC |
| exon 1 | 23857 | 93 | 1 | 0 | 0 | C/A | TAATGAAAGCAGATTCAAAGCAACA [G/A] CACCACCACTGAAGTATTTTAGTT |
| intron 1 | 23622 | 77 | 12 | 5 | -2.667405836 | C/G | ATTTTAAATGAGTTATAATATTAAT [C/G] TATTTTATGGAAATACTTTCTAACA |
| intron 1 | 23583 | 78 | 13 | 0 | 0 | A/G | TACTTTCTAACATGCAATTAGCAGG [A/G] AAATAGAATAAAATTAGTTCTCTCC |
| intron 1 | 18334 | 71 | 0 | 20 | -20.66326969 | -/T/ () | AGTCATGTACTCCTAGTTAGTGATG [-/T/ ()] CTTTTCATTCCTAATTTGTACACTG |
| intron 1 | 18264 | 73 | 0 | 19 | -20.20008135 | C/T | GCATTTAAGCTAAATGAAAGAAAAA [C/T] ACTATAAGTGAGATGATTAAAATAT |
| intron 2 | 17916 | 74 | 10 | 7 | -4.384231059 | G/A | GAATAGAGAAGGATATGCCAGACAA [G/A] ATCATAAGGTCTTGATAATCACAGG |
| intron 2 | 16939 | 65 | 17 | 8 | -2.859665125 | C/T | ATCCACTCGCCTCAGCCTCCCAAAG [C/T] GCAGAGATTACCAGAGTGAGCCACT |
| intron 2 | 16934 | 60 | 11 | 20 | -10.15791403 | A/G | CTCGCCTCAGCCTCCCAAAGCGCAG [A/G] GATTACCAGAGTGAGCCACTTCACC |
| intron 2 | 16837 | 89 | 1 | 0 | 0 | T/G | ACTTCCATCTTGTACATTAATCCGT [T/G] TTTGGTCCTTAGGACTGTGTTTCTT |
| intron 3 | 16599 | 60 | 11 | 19 | -9.704247488 | G/A | TATGCTGTTATCTATTATAAAGTTT [G/A] AGAGAAATAAATCTTTTTTACAGGT |
| intron 3 | 16543 | 59 | 11 | 20 | -10.05211275 | T/A | ATAGGTTTTGCCACATACTTTTATC [T/A] TTATTCATCTGATTTTCAGTTCCAA |
| intron 4 | 13227 | 85 | 5 | 0 | 0 | T/C | TTGATATTATATAAAGTGCTGTGTT [T/C] GTATTTGCCTTATTTGAACTTGTAT |
| oxon 5 | 13128 | 89 | 1 | 0 | 0 | T/C Pro 211 Pro | ATTCTACGGGAAAATGTGGGCCCCC [TIC Pro 211 Pro] CCACCTATTGACAATGGGGACATTA |
| exon 5 | 13092 | 66 | 17 | 7 | -2.450785359 | G/A Pro 223 Pro | ACAATGGGGACATTACTTCATTCCC [G/A Pro 223 Pro] TTGTCAGTATATGCTCCAGCTTCAT |
| exon 6 | 11741 | 59 | 11 | 20 | -10.05211275 | G/T Arg 302 Arg | AATCAGCTGAATTTGTGTGTAAACG [G/T Arg 302 Arg] GGATATCGTCTTTCATCACGTTCTC |
| exon 6 | 11705 | 19 | 11 | 60 | -9.704247488 | T/A (a) Arg 314 Arg | TTTCATCACGTTCTCACACATTGCG [T/A (a) Arg 314 Arg] ACAACATGTTGGGATGGGAAACTGG |
| exon 6 | 11593 | 19 | 11 | 60 | -9.704247488 | A/C | TTAGTATTAAATCAGTTCTTAATTT [A/C] ATTTTTAGTATTGTACTCCTT |

**Table 9. Variations in CFHL3 that may be correlated with the occurrence of AMD. Each variation is shown in the context of its surrounding DNA sequenpe. Location of each variation refers to the position on GenBank Accession AL049741.8, or the complementary DNA strand of GenBank Accession AL049741.8.**

| **Region** | **Position** | **Common/Common** | **Common/Rare** | **Rare/Rare** | **Log HW P-value** | **Change** | **Sequence Context** |
|---|---|---|---|---|---|---|---|
| promoter | 3779 | 86 | 2 | 0 | 0 | A/G | ATTTTGACCATTTGTGGGGGGGGGG [A/G] AAAAAACCTTGCCATGCCAAACAGC |
| promoter | 4364 | 63 | 17 | 9 | -3.220465172 | T/G | AATCCACAGATGATTGTGAAACCAC [T/G] AACTGGAATTATTGAAGCATTTTGT |
| promoter | 4465 | 64 | 17 | 9 | -3.26153442 | A/C | TCATGGTAGTGCACTTAAATTCAGA [A/G] CCACACTTGGTAACTAATAATGAAA |
| promoter | 4502 | 64 | 17 | 10 | -3.699998612 | C/A | AACTAATAATGAAAGATTTCAAACC [C/A] CAAACAGGGGAACTGAAACTTTTGT |
| exon 1 | 4607 | 88 | 1 | 0 | 0 | G/C Gly 18 Ala | ACCTTGTGGGTTTCCTGTGCTAATG [G/C Gly 18 Ala] ACAAGGTAAGTTAAAAGAGATCTAA |
| intron 2 | 9382 | 79 | 2 | 1 | -1.435387193 | T/C | ATGTTTATGCGATCTTATTTAAATA [T/C] GGTAACAATAATTTTAATATACTTT |
| intron 4 | 19710 | 56 | 15 | 8 | -2.935633472 | -/T/() | TCCCCACATATAAAGTATTTTTTTT [-/T/()] CAGATTCTTCAGAAAAGTGTGGGCC |
| exon 5 | 19820 | 56 | 14 | 10 | -4.079180573 | C/T Pro 241 Ser | GTCAAGAGTCGAGTACCAATGCCAG [C/T Pro 241 Ser] CCTACTATGAACTTCAGGGTTCTAA |
| exon 5 | 19885 | 58 | 14 | 8 | -3.249405761 | A/T Pro 262 Pro | GTAATGGAGAGTGGTCGGAACCACC [A/T Pro 262 Pro] AGATGCATACGTAAGTTCTTAAAAT |
| intron 5 | 19917 | 58 | 14 | 8 | -3.249405761 | T/A | ATACGTAAGTTCTTAAAATTCTAGA [T/A] CCTGAGAAAATCAGAGTAATAAGTT |
| intron 5 | 19928 | 79 | 1 | 0 | 0 | T/C | CTTAAAATTCTAGATCCTGAGAAAA [T/C] CAGAGTAATAAGTTTGATATTTGCT |
| intron 5 | 20057 | 78 | 0 | 1 | -2.195899652 | G/A | CAGATCTTAATATATAAGTGTATAA [G/A] CTTGGAAAATTCCATGTAAACAATG |
| intron 5 | 22921 | 69 | 1 | 0 | 0 | G/T | TATTTTATCCTAAACTACTCATTAG [G/T] ATGCATTTTATTTGCTCATGAAAGA |
| exon 6 | 23027 | 69 | 1 | 0 | 0 | TA/- ? 280 ? | GAAGAAAACATGAATAAAAATAACA [TA/- ? 280 ?] AAGTTAAAAGGAAGAAGTGACAGAA |
| exon 6 | 23203 | 66 | 3 | 1 | -1.147796072 | G/A | ATAAGGCAGCATTGTTACCCTAAAT [G/A] TATGTCCAACTTCCACTTTTCCACT |
| exon 6 | 23322 | 68 | 0 | 3 | -5.252863221 | A/G | AAAGAAAATTAATATAATAGTTTCA [A/G] TTTGCAACTTAATATATTCTCAAAA |

**Table 10. Variations in CFHL4 that may be correlated with the occurrence of AMD. Each variation is shown in the context of its surrounding DNA sequence in Chromosome 7:32512024-33512123.**

| **Region** | **Position** | **Common/Common** | **Common/Rare** | **Rare/Rare** | **Log HW P-value** | **Change** | **Sequence Context** |
|---|---|---|---|---|---|---|---|
| promoter | 7013 | 93 | 1 | 0 | 0 | C/T | GTTTATTTTCAACGTGATGTCAACA [C/T] GGCTCCTATCTTCATTTTCTTCTCC |
| promoter | 7369 | 91 | 4 | 0 | 0 | C/G | AATAGTTGCAGAAGCCTTTCATTCC [C/G] TGTATTAAAACTCTCTTTACTTAAA |
| promoter | 7577 | 91 | 5 | 0 | 0 | C/A | CTGAACTTTGATATTTACTAAGTGA [C/A] CTTAAAGCCCTAGCTTTGTGGTAGT |
| promoter | 7585 | 95 | 1 | 0 | 0 | C/G | TGATATTTACTAAGTGACCTTAAAG [C/G] CCTAGCTTTGTGGTAGTGCACTTAA |
| exon 2 | 22144 | 94 | 2 | 0 | 0 | T/C Asp 76 Asp | *GGGATTACATTCACTGCACACAAGA [T/C Asp 76 Asp] GGGTGGTTGCCAACAGTCCCATGCC |
| exon 3 | 32436 | 94 | 1 | 0 | 0 | T/C Ile 132 Ile | CAGATGGAAATTCTTCAGGTTCAAT [T/C Ile 132 Ile] ACATGTTTGCAAAATGGATGGTCAG |
| intron 5 | 37640 | 88 | 4 | 2 | -2.226371993 | T/G | GCTAAAGTCAGTATGTAGCACAAAT [T/G] AATAACTATTAACTATTTGGATTAT |
| intron 5 | 37701 | 69 | 18 | 6 | -1.933221388 | G/A | TATTTTATCCTAAACTACTCATTAG [G/A] ATGCATTTTATTTGCTCATGAAAGG |
| exon 6 | 37884 | 74 | 19 | 2 | -0.208237586 | G/A Gly 306 Glu | ACCATTGAATTTATGTGTAAATTGG [G/A Gly 306 Glu] ATATAATGCGAATACATCAGTTCTA |

## Claims

1. A method of identifying or aiding in identifying an individual at risk for developing age related macular degeneration or of diagnosing or aiding in diagnosing age related macular degeneration in an individual, comprising assaying a sample obtained from the individual for the presence of a variant CFH gene that is correlated with the occurrence of age related macular degeneration in humans, wherein the presence of a variant CFH gene indicates that the individual has or is at risk for developing age related macular degeneration and the variant CFH gene contains a mutation between tyrosine and histidine at amino acid 402, the risk allele being histidine.

2. A method of identifying or aiding in identifying an individual at risk for developing age related macular degeneration or of diagnosing or aiding in diagnosing age related macular degeneration in an individual, comprising assaying a sample obtained from the individual for the presence of a variant CFH gene that is correlated with the occurrence of age related macular degeneration in humans, wherein the presence of a variant CFH gene indicates that the individual has or is at risk for developing age related macular degeneration and the sample is assayed for the presence of a variant CFH gene by SNP genotyping, the SNP being rs380390.

3. A method of identifying or aiding in identifying an individual at risk for developing age related macular degeneration or of diagnosing or aiding in diagnosing age related macular degeneration in an individual, comprising assaying a sample obtained from the individual for the presence of a variant CFH gene that is correlated with the occurrence of age related macular degeneration in humans, wherein the presence of a variant CFH gene indicates that the individual has or is at risk for developing age related macular degeneration, wherein the variant CFH gene comprises a haplotype block which consists of the A allele of rs2019727, the C allele of rs10489456, the T allele of rs3753396, the C allele of rs380390, the C allele of rs2284664 and the G allele of rs1329428.

4. A method of identifying or aiding in identifying an individual at risk for developing age related macular degeneration or of diagnosing or aiding in diagnosing age related macular degeneration in an individual, comprising assaying a sample obtained from the individual for the presence of a variant CFH gene that is correlated with the occurrence of age related macular degeneration in humans, wherein the presence of a variant CFH gene indicates that the individual has or is at risk for developing age related macular degeneration and the sample is assayed for the presence of a variant CFH gene by SNP genotyping, the SNP being rs1061170.

## Patentansprüche

1. Ein Verfahren zur Identifizierung oder zur Unterstützung bei der Identifizierung eines Individuums, das dem Risiko einer Entwicklung einer altersbedingten Maculadegeneration ausgesetzt ist, oder zur Diagnose oder zur Unterstützung der Diagnose einer altersbedingten Maculadegeneration bei einem Individuum, umfassend die Prüfung einer von dem Individuum gewonnenen Probe zur Feststellung eines abweichenden CFH-Gens, das mit dem Auftreten einer altersbedingten Maculadegeneration bei Menschen korreliert, wobei die Anwesenheit eines abweichenden CFH-Gens darauf hindeutet, dass das Individuum eine altersbedingte Maculadegeneration hat oder dem Risiko einer altersbedingten Maculadegeneration ausgesetzt ist und das veränderte CFH-Gen eine Mutation zwischen Tyrosin und Histidin in der Aminosäure 402 beinhaltet, wobei das risikobehaftete Allel Histidin ist.

2. Ein Verfahren zur Identifizierung oder zur Unterstützung bei der Identifizierung eines Individuums, das dem Risiko einer Entwicklung einer altersbedingten Maculadegeneration ausgesetzt ist, oder zur Diagnose oder zur Unterstützung der Diagnose einer altersbedingten Maculadegeneration bei einem Individuum, umfassend die Prüfung einer von dem Individuum gewonnenen Probe zur Feststellung eines abweichenden CFH-Gens, das mit dem Auftreten einer altersbedingten Maculadegeneration bei Menschen korreliert, wobei die Anwesenheit eines abweichenden CFH-Gens darauf hindeutet, dass das Individuum eine altersbedingte Maculadegeneration hat oder dem Risiko einer altersbedingten Maculadegeneration ausgesetzt ist, und die Probe wird auf die Anwesenheit eines abweichenden CFH-Gens, das durch ein SNP genotypisiert wird, untersucht, wobei das SNP rs380390 ist.

3. Ein Verfahren zur Identifizierung oder zur Unterstützung bei der Identifizierung eines Individuums, das dem Risiko einer Entwicklung einer altersbedingten Maculadegeneration ausgesetzt ist, oder zur Diagnose oder zur Unterstützung der Diagnose einer altersbedingten Maculadegeneration bei einem Individuum, umfassend die Prüfung einer von dem Individuum gewonnenen Probe zur Feststellung eines abweichenden CFH-Gens, das mit dem Auftreten einer altersbedingten Maculadegeneration bei Menschen korreliert, wobei die Anwesenheit eines abweichenden CFH-Gens darauf hindeutet, dass das Individuum eine altersbedingte Maculadegeneration hat oder dem Risiko einer altersbedingten Maculadegeneration ausgesetzt ist, wobei das abweichende CFH-Gen einen haplotypen Block beinhaltet, der aus dem A-Allel von rs2019727, dem C-Allel von rs10489456, dem T-Allel von rs3753396, dem C-Allel von 380390, dem C-Allel von rs2284664 und dem G-Allel von rs1329428 besteht.

4. Ein Verfahren zur Identifizierung oder zur Unterstützung bei der Identifizierung eines Individuums, das dem Risiko einer Entwicklung einer altersbedingten Maculadegeneration ausgesetzt ist, oder zur Diagnose oder zur Unterstützung der Diagnose einer altersbedingten Maculadegeneration bei einem Individuum, umfassend die Prüfung einer von dem Individuum gewonnenen Probe zur Feststellung eines abweichenden CFH-Gens, das mit dem Auftreten einer altersbedingten Maculadegeneration bei Menschen korreliert, wobei die Anwesenheit eines abweichenden CFH-Gens darauf hindeutet, dass das Individuum eine altersbedingte Maculadegeneration hat oder dem Risiko einer altersbedingten Maculadegeneration ausgesetzt ist, und die Probe wird auf die Anwesenheit eines abweichenden CFH-Gens, das durch ein SNP genotypisiert wird untersucht, wobei das SNP rs1061170 ist.

## Revendications

1. Procédé d'identification ou d'aide à l'identification d'un individu susceptible de développer une dégénérescence maculaire liée à l'âge ou de diagnostic ou d'aide au diagnostic d'une dégénérescence maculaire liée à l'âge chez un individu, comprenant l'étape consistant à doser un échantillon obtenu de l'individu à la recherche de la présence d'un variant du gène CFH qui est corrélé à l'apparition de la dégénérescence maculaire liée à l'âge chez l'homme, la présence d'un variant du gène CFH indiquant que l'individu présente ou est susceptible de développer une dégénérescence maculaire liée à l'âge et le variant du gène CFH contient une mutation entre la tyrosine et l'histidine au niveau de l'acide aminé 402, l'allèle associé au risque étant l'histidine.

2. Procédé d'identification ou d'aide à l'identification d'un individu susceptible de développer une dégénérescence maculaire liée à l'âge ou de diagnostic ou d'aide au diagnostic d'une dégénérescence maculaire liée à l'âge chez un individu, comprenant l'étape consistant à doser un échantillon obtenu de l'individu à la recherche de la présence d'un variant du gène CFH qui est corrélé à l'apparition de la dégénérescence maculaire liée à l'âge chez l'homme, la présence d'un variant du gène CFH indiquant que l'individu présente ou est susceptible de développer une dégénérescence maculaire liée à l'âge et l'échantillon est analysé à la recherche de la présence d'un variant du gène CFH par le biais d'un génotypage de SNP, le SNP étant le rs380390.

3. Procédé d'identification ou d'aide à l'identification d'un individu susceptible de développer une dégénérescence maculaire liée à l'âge ou de diagnostic ou d'aide au diagnostic d'une dégénérescence maculaire liée à l'âge chez un individu, comprenant l'étape consistant à doser un échantillon obtenu de l'individu à la recherche de la présence d'un variant du gène CFH qui est corrélé à l'apparition de la dégénérescence maculaire liée à l'âge chez l'homme, la présence d'un variant du gène CFH indiquant que l'individu présente ou est susceptible de développer une dégénérescence maculaire liée à l'âge, le variant du gène CFH comprenant un bloc haplotype consistant en l'allèle A du rs2019727, l'allèle C du rs10489456, l'allèle T du rs3753396, l'allèle C du rs380390, l'allèle C du rs2284664 et l'allèle G du rs1329428.

4. Procédé d'identification ou d'aide à l'identification d'un individu susceptible de développer une dégénérescence maculaire liée à l'âge ou de diagnostic ou d'aide au diagnostic d'une dégénérescence maculaire liée à l'âge chez un individu, comprenant l'étape consistant à doser un échantillon obtenu de l'individu à la recherche de la présence d'un variant du gène CFH qui est corrélé à l'apparition de la dégénérescence maculaire liée à l'âge chez l'homme, la présence d'un variant du gène CFH indiquant que l'individu présente ou est susceptible de développer une dégénérescence maculaire liée à l'âge et l'échantillon est analysé à la recherche de la présence d'un variant du gène CFH par le biais d'un génotypage de SNP, le SNP étant le rs1061170.


### SEQUENCE LISTING

<110> Yale University
   Hoh, Josephine
<120> METHODS AND COMPOSITIONS FOR TREATING OCULAR DISORDERS
<130> YU-PWO-038
<150> US 60/629,363
   <151> 2004-11-18
<150> US 60/649,479
   <151> 2005-02-02
<150> US 60/672,346
   <151> 2005-04-18
<160> 59
<170> FastSEQ for Windows version 4.0
<210> 1
   <211> 4004
   <212> DNA
   <213> Homo sapiens
<400> 1
   ccttttgcag caagttcttt cctgcactaa tcacaattct tggaagagga gaactggacg 60
   ttgtgaacag agttagctgg taaatgtcct cttaaaagat ccaaaaaatg agacttctag 120
   caaagattat ttgccttatg ttatgggcta tttgtgtagc agaagattgc aatgaacttc 180
   ctccaagaag aaatacagaa attctgacag gttcctggtc tgaccaaaca tatccagaag 240
   gcacccaggc tatctataaa tgccgccctg gatatagatc tcttggaaat gtaataatgg 300
   tatgcaggaa gggagaatgg gttgctctta atccattaag gaaatgtcag aaaaggccct 360
   gtggacatcc tggagatact ccttttggta cttttaccct tacaggagga aatgtgtttg 420
   aatatggtgt aaaagctgtg tatacatgta atgaggggta tcaattgcta ggtgagatta 480
   attaccgtga atgtgacaca gatggatgga ccaatgatat tcctatatgt gaagttgtga 540
   agtgtttacc agtgacagca ccagagaatg gaaaaattgt cagtagtgca atggaaccag 600
   atcgggaata ccattttgga caagcagtac ggtttgtatg taactcaggc tacaagattg 660
   aaggagatga agaaatgcat tgttcagacg atggtttttg gagtaaagag aaaccaaagt 720
   gtgtggaaat ttcatgcaaa tccccagatg ttataaatgg atctcctata tctcagaaga 780
   ttatttataa ggagaatgaa cgatttcaat ataaatgtaa catgggttat gaatacagtg 840
   aaagaggaga tgctgtatgc actgaatctg gatggcgtcc gttgccttca tgtgaagaaa 900
   aatcatgtga taatccttat attccaaatg gtgactactc acctttaagg attaaacaca 960
   gaactggaga tgaaatcacg taccagtgta gaaatggttt ttatcctgca acccggggaa 1020
   atacagcaaa atgcacaagt actggctgga tacctgctcc gagatgtacc ttgaaacctt 1080
   gtgattatcc agacattaaa catggaggtc tatatcatga gaatatgcgt agaccatact 1140
   ttccagtagc tgtaggaaaa tattactcct attactgtga tgaacatttt gagactccgt 1200
   caggaagtta ctgggatcac attcattgca cacaagatgg atggtcgcca gcagtaccat 1260
   gcctcagaaa atgttatttt ccttatttgg aaaatggata taatcaaaat catggaagaa 1320
   agtttgtaca gggtaaatct atagacgttg cctgccatcc tggctacgct cttccaaaag 1380
   cgcagaccac agttacatgt atggagaatg gctggtctcc tactcccaga tgcatccgtg 1440
   tcaaaacatg ttccaaatca agtatagata ttgagaatgg gtttatttct gaatctcagt 1500
   atacatatgc cttaaaagaa aaagcgaaat atcaatgcaa actaggatat gtaacagcag 1560
   atggtgaaac atcaggatca attacatgtg ggaaagatgg atggtcagct caacccacgt 1620
   gcattaaatc ttgtgatatc ccagtattta tgaatgccag aactaaaaat gacttcacat 1680
   ggtttaagct gaatgacaca ttggactatg aatgccatga tggttatgaa agcaatactg 1740
   gaagcaccac tggttccata gtgtgtggtt acaatggttg gtctgattta cccatatgtt 1800
   atgaaagaga atgcgaactt cctaaaatag atgtacactt agttcctgat cgcaagaaag 1860
   accagtataa agttggagag gtgttgaaat tctcctgcaa accaggattt acaatagttg 1920
   gacctaattc cgttcagtgc taccactttg gattgtctcc tgacctccca atatgtaaag 1980
   agcaagtaca atcatgtggt ccacctcctg aactcctcaa tgggaatgtt aaggaaaaaa 2040
   cgaaagaaga atatggacac agtgaagtgg tggaatatta ttgcaatcct agatttctaa 2100
   tgaagggacc taataaaatt caatgtgttg atggagagtg gacaacttta ccagtgtgta 2160
   ttgtggagga gagtacctgt ggagatatac ctgaacttga acatggctgg gcccagcttt 2220
   cttcccctcc ttattactat ggagattcag tggaattcaa ttgctcagaa tcatttacaa 2280
   tgattggaca cagatcaatt acgtgtattc atggagtatg gacccaactt ccccagtgtg 2340
   tggcaataga taaacttaag aagtgcaaat catcaaattt aattatactt gaggaacatt 2400
   taaaaaacaa gaaggaattc gatcataatt ctaacataag gtacagatgt agaggaaaag 2460
   aaggatggat acacacagtc tgcataaatg gaagatggga tccagaagtg aactgctcaa 2520
   tggcacaaat acaattatgc ccacctccac ctcagattcc caattctcac aatatgacaa 2580
   ccacactgaa ttatcgggat ggagaaaaag tatctgttct ttgccaagaa aattatctaa 2640
   ttcaggaagg agaagaaatt acatgcaaag atggaagatg gcagtcaata ccactctgtg 2700
   ttgaaaaaat tccatgttca caaccacctc agatagaaca cggaaccatt aattcatcca 2760
   ggtcttcaca agaaagttat gcacatggga ctaaattgag ttatacttgt gagggtggtt 2820
   tcaggatatc tgaagaaaat gaaacaacat gctacatggg aaaatggagt tctccacctc 2880
   agtgtgaagg ccttccttgt aaatctccac ctgagatttc tcatggtgtt gtagctcaca 2940
   tgtcagacag ttatcagtat ggagaagaag ttacgtacaa atgttttgaa ggttttggaa 3000
   ttgatgggcc tgcaattgca aaatgcttag gagaaaaatg gtctcaccct ccatcatgca 3060
   taaaaacaga ttgtctcagt ttacctagct ttgaaaatgc catacccatg ggagagaaga 3120
   aggatgtgta taaggcgggt gagcaagtga cttacacttg tgcaacatat tacaaaatgg 3180
   atggagccag taatgtaaca tgcattaata gcagatggac aggaaggcca acatgcagag 3240
   acacctcctg tgtgaatccg cccacagtac aaaatgctta tatagtgtcg agacagatga 3300
   gtaaatatcc atctggtgag agagtacgtt atcaatgtag gagcccttat gaaatgtttg 3360
   gggatgaaga agtgatgtgt ttaaatggaa actggacgga accacctcaa tgcaaagatt 3420
   ctacaggaaa atgtgggccc cctccaccta ttgacaatgg ggacattact tcattcccgt 3480
   tgtcagtata tgctccagct tcatcagttg agtaccaatg ccagaacttg tatcaacttg 3540
   agggtaacaa gcgaataaca tgtagaaatg gacaatggtc agaaccacca aaatgcttac 3600
   atccgtgtgt aatatcccga gaaattatgg aaaattataa catagcatta aggtggacag 3660
   ccaaacagaa gctttattcg agaacaggtg aatcagttga atttgtgtgt aaacggggat 3720
   atcgtctttc atcacgttct cacacattgc gaacaacatg ttgggatggg aaactggagt 3780
   atccaacttg tgcaaaaaga tagaatcaat cataaagtgc acacctttat tcagaacttt 3840
   agtattaaat cagttctcaa tttcattttt tatgtattgt tttactcctt tttattcata 3900
   cgtaaaattt tggattaatt tgtgaaaatg taattataag ctgagaccgg tggctctctt 3960
   cttaaaagca ccatattaaa tcctggaaaa ctaaaaaaaa aaaa                  4004
<210> 2
   <211> 1702
   <212> DNA
   <213> Homo sapiens
<400> 2
   ccttttgcag caagttcttt cctgcactaa tcacaattct tggaagagga gaactggacg 60
   ttgtgaacag agttagctgg taaatgtcct cttaaaagat ccaaaaaatg agacttctag 120
   caaagattat ttgccttatg ttatgggcta tttgtgtagc agaagattgc aatgaacttc 180
   ctccaagaag aaatacagaa attctgacag gttcctggtc tgaccaaaca tatccagaag 240
   gcacccaggc tatctataaa tgccgccctg gatatagatc tcttggaaat gtaataatgg 300
   tatgcaggaa gggagaatgg gttgctctta atccattaag gaaatgtcag aaaaggccct 360
   gtggacatcc tggagatact ccttttggta cttttaccct tacaggagga aatgtgtttg 420
   aatatggtgt aaaagctgtg tatacatgta atgaggggta tcaattgcta ggtgagatta 480
   attaccgtga atgtgacaca gatggatgga ccaatgatat tcctatatgt gaagttgtga 540
   agtgtttacc agtgacagca ccagagaatg gaaaaattgt cagtagtgca atggaaccag 600
   atcgggaata ccatttggga caagcagtac ggtttgtatg taactcaggc tacaagattg 660
   aaggagatga agaaatgcat tgttcagacg atggtttttg gagtaaagag aaaccaaagt 720
   gtgtggaaat ttcatgcaaa tccccagatg ttataaatgg atctcctata tctcagaaga 780
   ttatttataa ggagaatgaa cgatttcaat ataaatgtaa catgggttat gaatacagtg 840
   aaagaggaga tgctgtatgc actgaatctg gatggcgtcc gttgccttca tgtgaagaaa 900
   aatcatgtga taatccttat attccaaatg gtgactactc acctttaagg attaaacaca 960
   gaactggaga tgaaatcacg taccagtgta gaaatggttt ttatcctgca acccggggaa 1020
   atacagcaaa atgcacaagt actggctgga tacctgctcc gagatgtacc ttgaaacctt 1080
   gtgattatcc agacattaaa catggaggtc tatatcatga gaatatgcgt agaccatact 1140
   ttccagtagc tgtaggaaaa tattactcct attactgtga tgaacatttt gagactccgt 1200
   caggaagtta ctgggatcac attcattgca cacaagatgg atggtcgcca gcagtaccat 1260
   gcctcagaaa atgttatttt ccttatttgg aaaatggata taatcaaaat catggaagaa 1320
   agtttgtaca gggtaaatct atagacgttg cctgccatcc tggctacgct cttccaaaag 1380
   cgcagaccac agttacatgt atggagaatg gctggtctcc tactcccaga tgcatccgtg 1440
   tcagctttac cctctgaact tctgatcgaa ggtcatccct ctccagcttg agtggatcaa 1500
   agatgacaag ggccaatgga accaagtttg agtcttgcca ggtcaatact tgggtcctga 1560
   gtatggtgac tagtatctgt tttgttatgt gtgtattatt ccagccagaa tgggaaatgc 1620
   taattcagct cctccaggca gcccaatggg gctggtggct ttgagattat taaactcttt 1680
   ctctgctgca aaaaaaaaaa aa                                          1702
<210> 3
   <211> 4252
   <212> DNA
   <213> Mus musculus
<400> 3
   aagtctttcc ctgctgtgac cacagttcat agcagagagg aactggatgg tacagcacag 60
   atttctcttg gagtcagttg gtcccagaaa gatccaaatt atgagactgt cagcaagaat 120
   tatttggctt atattatgga ctgtttgtgc agcagaagat tgtaaaggtc ctcctccaag 180
   agaaaattca gaaattctct caggctcgtg gtcagaacaa ctatatccag aaggcaccca 240
   ggctacctac aaatgccgcc ctggataccg aacacttggc actattgtaa aagtatgcaa 300
   gaatggaaaa tgggtggcgt ctaacccatc caggatatgt cggaaaaagc cttgtgggca 360
   tcccggagac acaccctttg ggtcctttag gctggcagtt ggatctcaat ttgagtttgg 420
   tgcaaaggtt gtttatacct gtgatgatgg gtatcaacta ttaggtgaaa ttgattaccg 480
   tgaatgtggt gcagatggct ggatcaatga tattccacta tgtgaagttg tgaagtgtct 540
   acctgtgaca gaactcgaga atggaagaat tgtgagtggt gcagcagaaa cagaccagga 600
   atactatttt ggacaggtgg tgcggtttga atgcaattca ggcttcaaga ttgaaggaca 660
   taaggaaatt cattgctcag aaaatggcct ttggagcaat gaaaagccac gatgtgtgga 720
   aattctctgc acaccaccgc gagtggaaaa tggagatggt ataaatgtga aaccagttta 780
   caaggagaat gaaagatacc actataagtg taagcatggt tatgtgccca aagaaagagg 840
   ggatgccgtc tgcacaggct ctggatggag ttctcagcct ttctgtgaag aaaagagatg 900
   ctcacctcct tatattctaa atggtatcta cacacctcac aggattatac acagaagtga 960
   tgatgaaatc agatatgaat gtaattatgg cttctatcct gtaactggat caactgtttc 1020
   aaagtgtaca cccactggct ggatccctgt tccaagatgt accttgaaac catgtgaatt 1080
   tccacaattc aaatatggac gtctgtatta tgaagagagc ctgagaccca acttcccagt 1140
   atctatagga aataagtaca gctataagtg tgacaacggg ttttcaccac cttctgggta 1200
   ttcctgggac taccttcgtt gcacagcaca agggtgggag cctgaagtcc catgcgtcag 1260
   gaaatgtgtt ttccattatg tggagaatgg agactctgca tactgggaaa aagtatatgt 1320
   gcagggtcag tctttaaaag tccagtgtta caatggctat agtcttcaaa atggtcaaga 1380
   cacaatgaca tgtacagaga atggctggtc ccctcctccc aaatgcatcc gtatcaagac 1440
   atgttcagca tcagatatac acattgacaa tggatttctt tctgaatctt cttctatata 1500
   tgctctaaat agagaaacat cctatagatg taagcaggga tatgtgacaa atactggaga 1560
   aatatcagga tcaataactt gccttcaaaa tggatggtca cctcaaccct catgcattaa 1620
   gtcttgtgat atgcctgtat ttgagaattc tataactaag aatactagga catggtttaa 1680
   gctcaatgac aaattagact atgaatgtct cgttggattt gaaaatgaat ataaacatac 1740
   caaaggctct ataacatgta cttattatgg atggtctgat acaccctcat gttatgaaag 1800
   agaatgcagt gttcccactc tagaccgaaa actagtcgtt tcccccagaa aagaaaaata 1860
   cagagttgga gatttgttgg aattctcctg ccattcagga cacagagttg ggccagattc 1920
   agtgcaatgc taccactttg gatggtctcc tggtttccct acatgtaaag gtcaagtagc 1980
   atcatgtgca ccacctcttg aaattcttaa tggggaaatt aatggagcaa aaaaagttga 2040
   atacagccat ggtgaagtgg tgaaatatga ttgcaaacct agattcctac tgaagggacc 2100
   caataaaatc cagtgtgttg atgggaattg gacaaccttg cctgtatgta ttgaggagga 2160
   gagaacatgt ggagacattc ctgaacttga acatggctct gccaagtgtt ctgttcctcc 2220
   ctaccaccat ggagattcag tggagttcat ttgtgaagaa aacttcacaa tgattggaca 2280
   tgggtcagtt tcttgcatta gtggaaaatg gacccagctt cctaaatgtg ttgcaacaga 2340
   ccaactggag aagtgtagag tgctgaagtc aactggcata gaagcaataa aaccaaaatt 2400
   gactgaattt acgcataact ccaccatgga ttacaaatgt agagacaagc aggagtacga 2460
   acgctcaatc tgtatcaatg gaaaatggga tcctgaacca aactgtacaa gcaaaacatc 2520
   ctgccctcct ccaccgcaga ttccaaatac ccaagtgatt gaaaccaccg tgaaatactt 2580
   ggatggagaa aaattatctg ttctttgcca agacaattac ctaactcagg actcagaaga 2640
   aatggtgtgc aaagatggaa ggtggcagtc attacctcgc tgcattgaaa aaattccatg 2700
   ttcccagccc cctacaatag aacatggatc tattaattta cccagatctt cagaagaaag 2760
   gagagattcc attgagtcca gcagtcatga acatggaact acattcagct atgtctgtga 2820
   tgatggtttc aggatacctg aagaaaatag gataacctgc tacatgggaa aatggagcac 2880
   tccacctcgc tgtgttggac ttccttgtgg acctccacct tcaattcctc ttggtactgt 2940
   ttctcttgag ctagagagtt accaacatgg ggaagaggtt acataccatt gttctacagg 3000
   ctttggaatt gatggaccag catttattat atgcgaagga ggaaagtggt ctgacccacc 3060
   aaaatgcata aaaacggatt gtgacgtttt acccacagtt aaaaatgcca taataagagg 3120
   aaagagcaaa aaatcatata ggacaggaga acaagtgaca ttcagatgtc aatctcctta 3180
   tcaaatgaat ggctcagaca ctgtgacatg tgttaatagt cggtggattg gacagccagt 3240
   atgcaaagat aattcctgtg tggatccacc acatgtgcca aatgctacta tagtaacaag 3300
   gaccaagaat aaatatctac atggtgacag agtacgttat gaatgtaata aacctttgga 3360
   actatttggg caagtggaag tgatgtgtga aaatgggata tggacagaaa aaccaaagtg 3420
   ccgagactca acagggaaat gtgggcctcc tccacctatt gacaatggag acatcacctc 3480
   cttgtcatta ccagtatatg aaccattatc atcagttgaa tatcaatgcc agaagtatta 3540
   tctccttaag ggaaagaaga caataacatg tacaaatgga aagtggtctg agccaccaac 3600
   atgcttacat gcatgtgtaa taccagaaaa cattatggaa tcacacaata taattctcaa 3660
   atggagacac actgaaaaga tttattccca ttcaggggag gatattgaat ttggatgtaa 3720
   atatggatat tataaagcaa gagattcacc gccatttcgt acaaagtgca ttaatggcac 3780
   catcaattat cccacttgtg tataaaatca taatacattt attagttgat tttattgttt 3840
   agaaaggcac atgcatgtga ctaatatact ttcaatttgc attgaagtat tgtttaactc 3900
   atgtcttctc ataaatataa acatttttgt tatatggtga ttaacttgta actttaaaaa 3960
   ctattgccaa aatgcaaaag cagtaattca aaactcctaa tctaaaatat gatatgtcca 4020
   aggacaaact atttcaatca agaaagtaga tgtaagttct tcaacatctg tttctattca 4080
   gaactttctc agattttcct ggataccttt tgatgtaagg tcctgattta cagtggataa 4140
   aggatatatt gactgattct tcaaattaat atgatttccc aaagcatgta acaaccaaac 4200
   tatcatatat tatatgacta atgcatacaa ttaattacta tataatactt tc         4252
<210> 4
   <211> 4250
   <212> DNA
   <213> Rattus norvegicus
<400> 4
   acagcacata cttctcttcg agtcaactgc tcccagatag atccaagaca tgagactgtc 60
   agcaagaatt atttggctta tattatggac tgtttgtgta gcagaagatt gtaaaggtcc 120
   tcctccaaga gaaaattcag aaattctctc aggttcgtgg tctgaacaac tatattcaga 180
   aggcactcag gcaacctaca aatgccgccc tggataccga acacttggta ctattgtaaa 240
   agtatgcaag aatggagaat gggtaccttc taacccatca aggatatgtc ggaaaaggcc 300
   atgtgggcat cccggagaca caccctttgg gtcctttagg ctggcagttg gatctgaatt 360
   tgaatttggt gcaaaggttg tttatacatg tgatgaaggg taccaactct taggtgaaat 420
   tgattaccgt gaatgtgatg cagatgggtg gaccaatgat attccaatat gtgaagttgt 480
   gaagtgcttg ccagtgacag aactggagaa tggaagaatt gtgagtggtg cagccgaacc 540
   agaccaggaa tattattttg gacaggtggt acgctttgaa tgcaactccg gcttcaagat 600
   tgaaggacag aaagaaatgc actgctcaga aaatggcctc tggagcaatg aaaagccaca 660
   gtgtgtggaa atttcttgcc tgccaccacg agttgaaaat ggagatggta tatatctgaa 720
   accagtttac aaggagaatg aaagattcca atataaatgt aagcaaggtt ttgtgtacaa 780
   agaaagaggg gatgctgtct gcacgggttc tggatggaat cctcagcctt cctgtgaaga 840
   aatgacatgt ttgactccat atattccaaa tggtatctac acacctcaca ggattaaaca 900
   cagaattgat gatgaaatca gatatgaatg taaaaatggc ttctatcctg caacccgatc 960
   acctgtttca aagtgtacaa ttactggctg gatccctgct ccaagatgta gcttgaaacc 1020
   ttgtgatttt ccacaattca aacatggacg tctgtattat gaagaaagcc ggagacccta 1080
   cttcccagta cctataggaa aggagtacag ctattactgt gacaacgggt ttacaacgcc 1140
   ttcacagtca tactgggact accttcgttg cacagtaaat gggtgggagc ctgaagttcc 1200
   atgcctcagg caatgtattt tccattatgt ggaatatgga gaatctttat actggcaaag 1260
   aagatatata gagggtcagt ctgcaaaagt ccagtgtcac agtggctata gtcttccaaa 1320
   tggtcaagat acaatattat gtacagaaaa tggctggtcc cctcctccca aatgcgtccg 1380
   tatcaagact tgttcagtat cagatataga aattgaaaat gggttttttt ctgaatctga 1440
   ttatacatat gctctaaata gaaaaacacg gtatagatgt aaacagggat atgtaacaaa 1500
   taccggagaa atatcaggaa taattacttg tcttcaagat ggatggtcac ctcgaccctc 1560
   atgcattaag tcttgtgata tgcctgtatt tgagaatgct atgactaaga ataataacac 1620
   atggtttaaa ctcaatgaca aattagacta tgaatgtcac attggatatg aaaatgaata 1680
   taaacatacc aaaggctcta taacatgtac ttatgatgga tggtctagta caccctcctg 1740
   ttatgaaaga gaatgcagca ttcccctgtt acaccaagac ttagttgttt ttcccagaga 1800
   agtaaaatac aaagttggag attcgttgag tttctcttgc cgttcaggac acagagttgg 1860
   agcagattta gtgcaatgct accactttgg atggtcccct aatttcccaa cgtgtgaagg 1920
   ccaagtaaaa tcatgtgacc aacctcttga aatcccgaat ggggaaataa agggaacaaa 1980
   aaaagttgaa tacagccatg gtgacgtggt ggaatatgat tgcaaaccta gatttctact 2040
   gaagggaccc aataaaatcc agtgtgttga cgggaagtgg acaaccttgc cgatatgcgt 2100
   tgagtatgag agaacatgtg gagaccttcc tgcacttgag catggctctg tccagttatc 2160
   tgtccctccc taccaccacg gagattcagt ggagttcact tgtgcagaaa ccttcacaat 2220
   gattgggcat gcagtagttt tctgcattag tggaaggtgg accgagcttc ctcaatgtgt 2280
   tgcaacagat caactggaga agtgtaaagc cccgaagtca actggcatag atgcaattca 2340
   tccaaataag aatgaattta atcataactt tagtgtgagt tacagatgta gacaaaagca 2400
   ggagtatgaa cattcaatct gcatcaatgg aagatgggat cctgaaccaa actgtacaag 2460
   aaatgagaaa agattctgcc ctcctccccc acagattcca aatgcccaag tgattgaaac 2520
   cacagtgaaa tacttggatg gagagaaagt atctgttctt tgccaagatg gttacctaac 2580
   tcagggccca gaagaaatgg tgtgtaaaca tggaaggtgg cagtcgttac cacgctgcac 2640
   ggaaaaaatt ccatgttccc agccccctaa aattgaacat ggatctatta agtcgcccag 2700
   gtcctcagaa gagagagatt taattgagtc cagcagttat gaacacggaa ctacattcag 2760
   ctatgtctgt gatgatggat tcaggatatc tgaagaaaat agggtaacct gcaacatggg 2820
   aaaatggagc tctctgcctc gttgtgttgg aataccttgt ggacccccac cttcaattcc 2880
   tcttggtatt gtttctcatg aactagaaag ttaccaatat ggagaggagg ttacatacaa 2940
   ttgttctgaa ggctttggaa ttgatggacc agcatttatt aaatgtgtag gaggacagtg 3000
   gtctgaacca cccaaatgca taaaaactga ttgtgacaac ttgcccacat ttgaaattgc 3060
   caaaccgaca gaaaagaaaa aaaaatcata caggtcagga gaacaagtga cattcagatg 3120
   tccacctccg tatcgaatgg atggctctga cattgtcaca tgtgttaata cgaagtggat 3180
   tggacagccg gtatgcaaag ataattcctg tgtgaatcca ccacatgtgc caaatgctac 3240
   tatactaaca aggcacaaga ctaaatatcc atctggtgac aaagtacgtt atgactgtaa 3300
   taaacctttt gaattatttg gggaagtgga agtgatgtgc caaaacggga tttggacaga 3360
   accaccgaaa tgcaaagatt caacagggaa atgtgggcct cctccaccta ttgacaatgg 3420
   agacatcacc tccttgtcat taccagtata tgcaccatta tcatcagttg aatatcaatg 3480
   ccagaactat tatctactta agggaaataa gatagtaaca tgtagaaatg gaaagtggtc 3540
   tcagccacca acctgcttac atgcatgtgt gataccagaa gatattatgg aaaaacataa 3600
   tatagttctc agatggaggg aaaatgcaaa gatttattcc caatcagggg agaatattga 3660
   attcatgtgt aaacctggat atagaaaatt cagaggatca cctccgtttc gtacaaagtg 3720
   cattgagggt cacatcaatt atcccacttg tgtataaaat cgctatacaa ttattagtaa 3780
   accttatgga tgaacctttg tttagaaatg cacatgtata ttactaatac agtttgaatt 3840
   tacatttgaa atattgttta gctcatttct tctaataagt atataaactt tttttatatg 3900
   gtggttaatc agtaacttta cagactgttg ccacaaagca agaacattgc attcaaaact 3960
   cctaatccaa aatatgatat gtccaaggac aaactatgtc taagcaagaa aataaatgtt 4020
   agttcttcaa tgtctgtttt tattcaggac ttttcagatt ttcttggata ccttttgttg 4080
   ttaggttctg attcacagtg agtggaagac acactgactc tgacttcaaa ttagtattac 4140
   ttgccaatac ataacaacca aactatcata atatcacaaa tgtatacagc taattactgt 4200
   gtcctacctt tgtatcaata aagaaatcta agaaagttct tgcttatgaa            4250
<210> 5
   <211> 1231
   <212> PRT
   <213> Homo sapiens
<400> 5
<210> 6
   <211> 449
   <212> PRT
   <213> Homo sapiens
<400> 6
   Met Arg Leu Leu Ala Lys Ile Ile Cys Leu Met Leu Trp Ala Ile Cys
   1 5 10 15
   Val Ala Glu Asp Cys Asn Glu Leu Pro Pro Arg Arg Asn Thr Glu Ile
   20 25 30
   Leu Thr Gly Ser Trp Ser Asp Gln Thr Tyr Pro Glu Gly Thr Gln Ala
   35 40 45
   Ile Tyr Lys Cys Arg Pro Gly Tyr Arg Ser Leu Gly Asn Val Ile Met
   50 55 60
   Val Cys Arg Lys Gly Glu Trp Val Ala Leu Asn Pro Leu Arg Lys Cys
   65 70 75 80
   Gln Lys Arg Pro Cys Gly His Pro Gly Asp Thr Pro Phe Gly Thr Phe
   85 90 95
   Thr Leu Thr Gly Gly Asn Val Phe Glu Tyr Gly Val Lys Ala Val Tyr
   100 105 110
   Thr Cys Asn Glu Gly Tyr Gln Leu Leu Gly Glu Ile Asn Tyr Arg Glu
   115 120 125
   Cys Asp Thr Asp Gly Trp Thr Asn Asp Ile Pro Ile Cys Glu Val Val
   130 135 140
   Lys Cys Leu Pro Val Thr Ala Pro Glu Asn Gly Lys Ile Val Ser Ser
   145 150 155 160
   Ala Met Glu Pro Asp Arg Glu Tyr His Phe Gly Gln Ala Val Arg Phe
   165 170 175
   Val Cys Asn Ser Gly Tyr Lys Ile Glu Gly Asp Glu Glu Met His Cys
   180 185 190
   Ser Asp Asp Gly Phe Trp Ser Lys Glu Lys Pro Lys Cys Val Glu Ile
   195 200 205
   Ser Cys Lys Ser Pro Asp Val Ile Asn Gly Ser Pro Ile Ser Gln Lys
   210 215 220
   Ile Ile Tyr Lys Glu Asn Glu Arg Phe Gln Tyr Lys Cys Asn Met Gly
   225 230 235 240
   Tyr Glu Tyr Ser Glu Arg Gly Asp Ala Val Cys Thr Glu Ser Gly Trp
   245 250 255
   Arg Pro Leu Pro Ser Cys Glu Glu Lys Ser Cys Asp Asn Pro Tyr Ile
   260 265 270
   Pro Asn Gly Asp Tyr Ser Pro Leu Arg Ile Lys His Arg Thr Gly Asp
   275 280 285
   Glu Ile Thr Tyr Gln Cys Arg Asn Gly Phe Tyr Pro Ala Thr Arg Gly
   290 295 300
   Asn Thr Ala Lys Cys Thr Ser Thr Gly Trp Ile Pro Ala Pro Arg Cys
   305 310 315 320
   Thr Leu Lys Pro Cys Asp Tyr Pro Asp Ile Lys His Gly Gly Leu Tyr
   325 330 335
   His Glu Asn Met Arg Arg Pro Tyr Phe Pro Val Ala Val Gly Lys Tyr
   340 345 350
   Tyr Ser Tyr Tyr Cys Asp Glu His Phe Glu Thr Pro Ser Gly Ser Tyr
   355 360 365
   Trp Asp His Ile His Cys Thr Gln Asp Gly Trp Ser Pro Ala Val Pro
   370 375 380
   Cys Leu Arg Lys Cys Tyr Phe Pro Tyr Leu Glu Asn Gly Tyr Asn Gln
   385 390 395 400
   Asn His Gly Arg Lys Phe Val Gln Gly Lys Ser Ile Asp Val Ala Cys
   405 410 415
   His Pro Gly Tyr Ala Leu Pro Lys Ala Gln Thr Thr Val Thr Cys Met
   420 425 430
   Glu Asn Gly Trp Ser Pro Thr Pro Arg Cys Ile Arg Val Ser Phe Thr
   435 440 445
   Leu
<210> 7
   <211> 1234
   <212> PRT
   <213> Mus musculus
<400> 7
   Met Arg Leu Ser Ala Arg Ile Ile Trp Leu Ile Leu Trp Thr Val Cys
    1               5                  10                  15
   Ala Ala Glu Asp Cys Lys Gly Pro Pro Pro Arg Glu Asn Ser Glu Ile
               20                  25                  30
   Leu Ser Gly Ser Trp Ser Glu Gln Leu Tyr Pro Glu Gly Thr Gln Ala
           35                  40                  45
   Thr Tyr Lys Cys Arg Pro Gly Tyr Arg Thr Leu Gly Thr Ile Val Lys
       50                  55                  60
   Val Cys Lys Asn Gly Lys Trp Val Ala Ser Asn Pro Ser Arg Ile Cys
   65                  70                  75                  80
   Arg Lys Lys Pro Cys Gly His Pro Gly Asp Thr Pro Phe Gly Ser Phe
                   85                  90                  95
   Arg Leu Ala Val Gly Ser Gln Phe Glu Phe Gly Ala Lys Val Val Tyr
               100                 105                 110
   Thr Cys Asp Asp Gly Tyr Gln Leu Leu Gly Glu Ile Asp Tyr Arg Glu
           115                 120                 125
   Cys Gly Ala Asp Gly Trp Ile Asn Asp Ile Pro Leu Cys Glu Val Val
       130                 135                 140
   Lys Cys Leu Pro Val Thr Glu Leu Glu Asn Gly Arg Ile Val Ser Gly
   145                 150                 155                 160
   Ala Ala Glu Thr Asp Gln Glu Tyr Tyr Phe Gly Gln Val Val Arg Phe
                   165                 170                 175
   Glu Cys Asn Ser Gly Phe Lys Ile Glu Gly His Lys Glu Ile His Cys
               180                 185                 190
   Ser Glu Asn Gly Leu Trp Ser Asn Glu Lys Pro Arg Cys Val Glu Ile
           195                 200                 205
   Leu Cys Thr Pro Pro Arg Val Glu Asn Gly Asp Gly Ile Asn Val Lys
       210                 215                 220
   Pro Val Tyr Lys Glu Asn Glu Arg Tyr His Tyr Lys Cys Lys His Gly
   225                 230                 235                 240
   Tyr Val Pro Lys Glu Arg Gly Asp Ala Val Cys Thr Gly Ser Gly Trp
                   245                 250                 255
   Ser Ser Gln Pro Phe Cys Glu Glu Lys Arg Cys Ser Pro Pro Tyr Ile
               260                 265                 270
   Leu Asn Gly Ile Tyr Thr Pro His Arg Ile Ile His Arg Ser Asp Asp
           275                 280                 285
   Glu Ile Arg Tyr Glu Cys Asn Tyr Gly Phe Tyr Pro Val Thr Gly Ser
       290                 295                 300
   Thr Val Ser Lys Cys Thr Pro Thr Gly Trp Ile Pro Val Pro Arg Cys
   305                 310                 315                 320
   Thr Leu Lys Pro Cys Glu Phe Pro Gln Phe Lys Tyr Gly Arg Leu Tyr
                   325                 330                 335
   Tyr Glu Glu Ser Leu Arg Pro Asn Phe Pro Val Ser Ile Gly Asn Lys
               340                 345                 350
   Tyr Ser Tyr Lys Cys Asp Asn Gly Phe Ser Pro Pro Ser Gly Tyr Ser
           355                 360                 365
   Trp Asp Tyr Leu Arg Cys Thr Ala Gln Gly Trp Glu Pro Glu Val Pro
       370                 375                 380
   Cys Val Arg Lys Cys Val Phe His Tyr Val Glu Asn Gly Asp Ser Ala
   385                 390                 395                 400
   Tyr Trp Glu Lys Val Tyr Val Gln Gly Gln Ser Leu Lys Val Gln Cys
                   405                 410                 415
   Tyr Asn Gly Tyr Ser Leu Gln Asn Gly Gln Asp Thr Met Thr Cys Thr
               420                 425                 430
   Glu Asn Gly Trp Ser Pro Pro Pro Lys Cys Ile Arg Ile Lys Thr Cys
           435                 440                 445
   Ser Ala Ser Asp Ile His Ile Asp Asn Gly Phe Leu Ser Glu Ser Ser
       450                 455                 460
   Ser Ile Tyr Ala Leu Asn Arg Glu Thr Ser Tyr Arg Cys Lys Gln Gly
   465                 470                 475                 480
   Tyr Val Thr Asn Thr Gly Glu Ile Ser Gly Ser Ile Thr Cys Leu Gln
                   485                 490                 495
   Asn Gly Trp Ser Pro Gln Pro Ser Cys Ile Lys Ser Cys Asp Met Pro
               500                 505                 510
   Val Phe Glu Asn Ser Ile Thr Lys Asn Thr Arg Thr Trp Phe Lys Leu
           515                 520                 525
   Asn Asp Lys Leu Asp Tyr Glu Cys Leu Val Gly Phe Glu Asn Glu Tyr
       530                 535                 540
   Lys His Thr Lys Gly Ser Ile Thr Cys Thr Tyr Tyr Gly Trp Ser Asp
   545                 550                 555                 560
   Thr Pro Ser Cys Tyr Glu Arg Glu Cys Ser Val Pro Thr Leu Asp Arg
                   565                 570                 575
   Lys Leu Val Val Ser Pro Arg Lys Glu Lys Tyr Arg Val Gly Asp Leu
               580                 585                 590
   Leu Glu Phe Ser Cys His Ser Gly His Arg Val Gly Pro Asp Ser Val
           595                 600                 605
   Gln Cys Tyr His Phe Gly Trp Ser Pro Gly Phe Pro Thr Cys Lys Gly
       610                 615                 620
   Gln Val Ala Ser Cys Ala Pro Pro Leu Glu Ile Leu Asn Gly Glu Ile
   625                 630                 635                 640
   Asn Gly Ala Lys Lys Val Glu Tyr Ser His Gly Glu Val Val Lys Tyr
                   645                 650                 655
   Asp Cys Lys Pro Arg Phe Leu Leu Lys Gly Pro Asn Lys Ile Gln Cys
               660                 665                 670
   Val Asp Gly Asn Trp Thr Thr Leu Pro Val Cys Ile Glu Glu Glu Arg
           675                 680                 685
   Thr Cys Gly Asp Ile Pro Glu Leu Glu His Gly Ser Ala Lys Cys Ser
       690                 695                 700
   Val Pro Pro Tyr His His Gly Asp Ser Val Glu Phe Ile Cys Glu Glu
   705                 710                 715                 720
   Asn Phe Thr Met Ile Gly His Gly Ser Val Ser Cys Ile Ser Gly Lys
                   725                 730                 735
   Trp Thr Gln Leu Pro Lys Cys Val Ala Thr Asp Gln Leu Glu Lys Cys
               740                 745                 750
   Arg Val Leu Lys Ser Thr Gly Ile Glu Ala Ile Lys Pro Lys Leu Thr
           755                 760                 765
   Glu Phe Thr His Asn Ser Thr Met Asp Tyr Lys Cys Arg Asp Lys Gln
       770                 775                 780
   Glu Tyr Glu Arg Ser Ile Cys Ile Asn Gly Lys Trp Asp Pro Glu Pro
   785                 790                 795                 800
   Asn Cys Thr Ser Lys Thr Ser Cys Pro Pro Pro Pro Gln Ile Pro Asn
                   805                 810                 815
   Thr Gln Val Ile Glu Thr Thr Val Lys Tyr Leu Asp Gly Glu Lys Leu
               820                 825                 830
   Ser Val Leu Cys Gln Asp Asn Tyr Leu Thr Gln Asp Ser Glu Glu Met
           835                 840                 845
   Val Cys Lys Asp Gly Arg Trp Gln Ser Leu Pro Arg Cys Ile Glu Lys
       850                 855                 860
   Ile Pro Cys Ser Gln Pro Pro Thr Ile Glu His Gly Ser Ile Asn Leu
   865                 870                 875                 880
   Pro Arg Ser Ser Glu Glu Arg Arg Asp Ser Ile Glu Ser Ser Ser His
                   885                 890                 895
   Glu His Gly Thr Thr Phe Ser Tyr Val Cys Asp Asp Gly Phe Arg Ile
               900                 905                 910
   Pro Glu Glu Asn Arg Ile Thr Cys Tyr Met Gly Lys Trp Ser Thr Pro
           915                 920                 925
   Pro Arg Cys Val Gly Leu Pro Cys Gly Pro Pro Pro Ser Ile Pro Leu
       930                 935                 940
   Gly Thr Val Ser Leu Glu Leu Glu Ser Tyr Gln His Gly Glu Glu Val
   945                 950                 955                 960
   Thr Tyr His Cys Ser Thr Gly Phe Gly Ile Asp Gly Pro Ala Phe Ile
                   965                 970                 975
   Ile Cys Glu Gly Gly Lys Trp Ser Asp Pro Pro Lys Cys Ile Lys Thr
               980                 985                 990
   Asp Cys Asp Val Leu Pro Thr Val Lys Asn Ala Ile Ile Arg Gly Lys
           995                 1000                1005
   Ser Lys Lys Ser Tyr Arg Thr Gly Glu Gln Val Thr Phe Arg Cys Gln
       1010                1015                1020
   Ser Pro Tyr Gln Met Asn Gly Ser Asp Thr Val Thr Cys Val Asn Ser
   1025                1030                1035                1040
   Arg Trp Ile Gly Gln Pro Val Cys Lys Asp Asn Ser Cys Val Asp Pro
                   1045                1050                1055
   Pro His Val Pro Asn Ala Thr Ile Val Thr Arg Thr Lys Asn Lys Tyr
               1060                1065                1070
   Leu His Gly Asp Arg Val Arg Tyr Glu Cys Asn Lys Pro Leu Glu Leu
           1075                1080                1085
   Phe Gly Gln Val Glu Val Met Cys Glu Asn Gly Ile Trp Thr Glu Lys
       1090                1095                1100
   Pro Lys Cys Arg Asp Ser Thr Gly Lys Cys Gly Pro Pro Pro Pro Ile
   1105                1110                1115                1120
   Asp Asn Gly Asp Ile Thr Ser Leu Ser Leu Pro Val Tyr Glu Pro Leu
                   1125                1130                1135
   Ser Ser Val Glu Tyr Gln Cys Gln Lys Tyr Tyr Leu Leu Lys Gly Lys
               1140                1145                1150
   Lys Thr Ile Thr Cys Thr Asn Gly Lys Trp Ser Glu Pro Pro Thr Cys
           1155                1160                1165
   Leu His Ala Cys Val Ile Pro Glu Asn Ile Met Glu Ser His Asn Ile
       1170                1175                1180
   Ile Leu Lys Trp Arg His Thr Glu Lys Ile Tyr Ser His Ser Gly Glu
   1185                1190                1195                1200
   Asp Ile Glu Phe Gly Cys Lys Tyr Gly Tyr Tyr Lys Ala Arg Asp Ser
                   1205                1210                1215
   Pro Pro Phe Arg Thr Lys Cys Ile Asn Gly Thr Ile Asn Tyr Pro Thr
               1220                1225                1230
   Cys Val
<210> 8
   <211> 1235
   <212> PRT
   <213> Rattus norvegicus
<400> 8
   Met Arg Leu Ser Ala Arg Ile Ile Trp Leu Ile Leu Trp Thr Val Cys
   1               5                   10                  15
   Val Ala Glu Asp Cys Lys Gly Pro Pro Pro Arg Glu Asn Ser Glu Ile
               20                  25                  30
   Leu Ser Gly Ser Trp Ser Glu Gln Leu Tyr Ser Glu Gly Thr Gln Ala
           35                  40                  45
   Thr Tyr Lys Cys Arg Pro Gly Tyr Arg Thr Leu Gly Thr Ile Val Lys
       50                  55                  60
   Val Cys Lys Asn Gly Glu Trp Val Pro Ser Asn Pro Ser Arg Ile Cys
   65                  70                  75                  80
   Arg Lys Arg Pro Cys Gly His Pro Gly Asp Thr Pro Phe Gly Ser Phe
                   85                  90                  95
   Arg Leu Ala Val Gly Ser Glu Phe Glu Phe Gly Ala Lys Val Val Tyr
               100                 105                 110
   Thr Cys Asp Glu Gly Tyr Gln Leu Leu Gly Glu Ile Asp Tyr Arg Glu
           115                 120                 125
   Cys Asp Ala Asp Gly Trp Thr Asn Asp Ile Pro Ile Cys Glu Val Val
       130                 135                 140
   Lys Cys Leu Pro Val Thr Glu Leu Glu Asn Gly Arg Ile Val Ser Gly
   145                 150                 155                 160
   Ala Ala Glu Pro Asp Gln Glu Tyr Tyr Phe Gly Gln Val Val Arg Phe
                   165                 170                 175
   Glu Cys Asn Ser Gly Phe Lys Ile Glu Gly Gln Lys Glu Met His Cys
               180                 185                 190
   Ser Glu Asn Gly Leu Trp Ser Asn Glu Lys Pro Gln Cys Val Glu Ile
           195                 200                 205
   Ser Cys Leu Pro Pro Arg Val Glu Asn Gly Asp Gly Ile Tyr Leu Lys
       210                 215                 220
   Pro Val Tyr Lys Glu Asn Glu Arg Phe Gln Tyr Lys Cys Lys Gln Gly
   225                 230                 235                 240
   Phe Val Tyr Lys Glu Arg Gly Asp Ala Val Cys Thr Gly Ser Gly Trp
                   245                 250                 255
   Asn Pro Gln Pro Ser Cys Glu Glu Met Thr Cys Leu Thr Pro Tyr Ile
               260                 265                 270
   Pro Asn Gly Ile Tyr Thr Pro His Arg Ile Lys His Arg Ile Asp Asp
           275                 280                 285
   Glu Ile Arg Tyr Glu Cys Lys Asn Gly Phe Tyr Pro Ala Thr Arg Ser
       290                 295                 300
   Pro Val Ser Lys Cys Thr Ile Thr Gly Trp Ile Pro Ala Pro Arg Cys
   305                 310                 315                 320
   Ser Leu Lys Pro Cys Asp Phe Pro Gln Phe Lys His Gly Arg Leu Tyr
                   325                 330                 335
   Tyr Glu Glu Ser Arg Arg Pro Tyr Phe Pro Val Pro Ile Gly Lys Glu
               340                 345                 350
   Tyr Ser Tyr Tyr Cys Asp Asn Gly Phe Thr Thr Pro Ser Gln Ser Tyr
           355                 360                 365
   Trp Asp Tyr Leu Arg Cys Thr Val Asn Gly Trp Glu Pro Glu Val Pro
       370                 375                 380
   Cys Leu Arg Gln Cys Ile Phe His Tyr Val Glu Tyr Gly Glu Ser Leu
   385                 390                 395                 400
   Tyr Trp Gln Arg Arg Tyr Ile Glu Gly Gln Ser Ala Lys Val Gln Cys
                   405                 410                 415
   His Ser Gly Tyr Ser Leu Pro Asn Gly Gln Asp Thr Ile Leu Cys Thr
               420                 425                 430
   Glu Asn Gly Trp Ser Pro Pro Pro Lys Cys Val Arg Ile Lys Thr Cys
           435                 440                 445
   Ser Val Ser Asp Ile Glu Ile Glu Asn Gly Phe Phe Ser Glu Ser Asp
       450                 455                 460
   Tyr Thr Tyr Ala Leu Asn Arg Lys Thr Arg Tyr Arg Cys Lys Gln Gly
   465                 470                 475                 480
   Tyr Val Thr Asn Thr Gly Glu Ile Ser Gly Ile Ile Thr Cys Leu Gln
                   485                 490                 495
   Asp Gly Trp Ser Pro Arg Pro Ser Cys Ile Lys Ser Cys Asp Met Pro
               500                 505                 510
   Val Phe Glu Asn Ala Met Thr Lys Asn Asn Asn Thr Trp Phe Lys Leu
           515                 520                 525
   Asn Asp Lys Leu Asp Tyr Glu Cys His Ile Gly Tyr Glu Asn Glu Tyr
       530                 535                 540
   Lys His Thr Lys Gly Ser Ile Thr Cys Thr Tyr Asp Gly Trp Ser Ser
   545                 550                 555                 560
   Thr Pro Ser Cys Tyr Glu Arg Glu Cys Ser Ile Pro Leu Leu His Gln
                   565                 570                 575
   Asp Leu Val Val Phe Pro Arg Glu Val Lys Tyr Lys Val Gly Asp Ser
               580                 585                 590
   Leu Ser Phe Ser Cys Arg Ser Gly His Arg Val Gly Ala Asp Leu Val
           595                 600                 605
   Gln Cys Tyr His Phe Gly Trp Ser Pro Asn Phe Pro Thr Cys Glu Gly
       610                 615                 620
   Gln Val Lys Ser Cys Asp Gln Pro Leu Glu Ile Pro Asn Gly Glu Ile
   625                 630                 635                 640
   Lys Gly Thr Lys Lys Val Glu Tyr Ser His Gly Asp Val Val Glu Tyr
                   645                 650                 655
   Asp Cys Lys Pro Arg Phe Leu Leu Lys Gly Pro Asn Lys Ile Gln Cys
               660                 665                 670
   Val Asp Gly Lys Trp Thr Thr Leu Pro Ile Cys Val Glu Tyr Glu Arg
           675                 680                 685
   Thr Cys Gly Asp Leu Pro Ala Leu Glu His Gly Ser Val Gln Leu Ser
       690                 695                 700
   Val Pro Pro Tyr His His Gly Asp Ser Val Glu Phe Thr Cys Ala Glu
   705                 710                 715                 720
   Thr Phe Thr Met Ile Gly His Ala Val Val Phe Cys Ile Ser Gly Arg
                   725                 730                 735
   Trp Thr Glu Leu Pro Gln Cys Val Ala Thr Asp Gln Leu Glu Lys Cys
               740                 745                 750
   Lys Ala Pro Lys Ser Thr Gly Ile Asp Ala Ile His Pro Asn Lys Asn
           755                 760                 765
   Glu Phe Asn His Asn Phe Ser Val Ser Tyr Arg Cys Arg Gln Lys Gln
       770                 775                 780
   Glu Tyr Glu His Ser Ile Cys Ile Asn Gly Arg Trp Asp Pro Glu Pro
   785                 790                 795                 800
   Asn Cys Thr Arg Asn Glu Lys Arg Phe Cys Pro Pro Pro Pro Gln Ile
                   805                 810                 815
   Pro Asn Ala Gln Val Ile Glu Thr Thr Val Lys Tyr Leu Asp Gly Glu
               820                 825                 830
   Lys Val Ser Val Leu Cys Gln Asp Gly Tyr Leu Thr Gln Gly Pro Glu
           835                 840                 845
   Glu Met Val Cys Lys His Gly Arg Trp Gln Ser Leu Pro Arg Cys Thr
       850                 855                 860
   Glu Lys Ile Pro Cys Ser Gln Pro Pro Lys Ile Glu His Gly Ser Ile
   865                 870                 875                 880
   Lys Ser Pro Arg Ser Ser Glu Glu Arg Asp Leu Ile Glu Ser Ser Ser
                   885                 890                 895
   Tyr Glu His Gly Thr Thr Phe Ser Tyr Val Cys Asp Asp Gly Phe Arg
               900                 905                 910
   Ile Ser Glu Glu Asn Arg Val Thr Cys Asn Met Gly Lys Trp Ser Ser
           915                 920                 925
   Leu Pro Arg Cys Val Gly Ile Pro Cys Gly Pro Pro Pro Ser Ile Pro
       930                 935                 940
   Leu Gly Ile Val Ser His Glu Leu Glu Ser Tyr Gln Tyr Gly Glu Glu
   945                 950                 955                 960
   Val Thr Tyr Asn Cys Ser Glu Gly Phe Gly Ile Asp Gly Pro Ala Phe
                   965                 970                 975
   Ile Lys Cys Val Gly Gly Gln Trp Ser Glu Pro Pro Lys Cys Ile Lys
               980                 985                 990
   Thr Asp Cys Asp Asn Leu Pro Thr Phe Glu Ile Ala Lys Pro Thr Glu
           995                 1000                1005
   Lys Lys Lys Lys Ser Tyr Arg Ser Gly Glu Gln Val Thr Phe Arg Cys
       1010                1015                1020
   Pro Pro Pro Tyr Arg Met Asp Gly Ser Asp Ile Val Thr Cys Val Asn
   1025                1030                1035                1040
   Thr Lys Trp Ile Gly Gln Pro Val Cys Lys Asp Asn Ser Cys Val Asn
                   1045                1050                1055
   Pro Pro His Val Pro Asn Ala Thr Ile Leu Thr Arg His Lys Thr Lys
               1060                1065                1070
   Tyr Pro Ser Gly Asp Lys Val Arg Tyr Asp Cys Asn Lys Pro Phe Glu
           1075                1080                1085
   Leu Phe Gly Glu Val Glu Val Met Cys Gln Asn Gly Ile Trp Thr Glu
       1090                1095                1100
   Pro Pro Lys Cys Lys Asp Ser Thr Gly Lys Cys Gly Pro Pro Pro Pro
   1105                1110                1115                1120
   Ile Asp Asn Gly Asp Ile Thr Ser Leu Ser Leu Pro Val Tyr Ala Pro
                   1125                1130                1135
   Leu Ser Ser Val Glu Tyr Gln Cys Gln Asn Tyr Tyr Leu Leu Lys Gly
               1140                1145                1150
   Asn Lys Ile Val Thr Cys Arg Asn Gly Lys Trp Ser Gln Pro Pro Thr
           1155                1160                1165
   Cys Leu His Ala Cys Val Ile Pro Glu Asp Ile Met Glu Lys His Asn
       1170                1175                1180
   Ile Val Leu Arg Trp Arg Glu Asn Ala Lys Ile Tyr Ser Gln Ser Gly
   1185                1190                1195                1200
   Glu Asn Ile Glu Phe Met Cys Lys Pro Gly Tyr Arg Lys Phe Arg Gly
                   1205                1210                1215
   Ser Pro Pro Phe Arg Thr Lys Cys Ile Glu Gly His Ile Asn Tyr Pro
               1220                1225                1230
   Thr Cys Val
           1235
<210> 9
   <211> 150626
   <212> DNA
   <213> Homo sapiens
<400> 9
<210> 10
   <211> 23
   <212> DNA
   <213> Artificial Sequence
<220>
   <223> Forward promoter primer for CFH gene
<400> 10
   agaatcgtgg tctctgtgtg tgg 23
<210> 11
   <211> 22
   <212> DNA
   <213> Artificial Sequence
<220>
   <223> Reverse promoter primer for CFH gene
<400> 11
   agcagctggt gatatcctct gg 22
<210> 12
   <211> 23
   <212> DNA
   <213> Artificial Sequence
<220>
   <223> Forward promoter primer for CFH gene
<400> 12
   tcaaatgaga gtgagccagt tgc 23
<210> 13
   <211> 23
   <212> DNA
   <213> Artificial Sequence
<220>
   <223> Reverse promoter primer for CFH gene
<400> 13
   ctgttcacaa cgtccagttc tcc 23
<210> 14
   <211> 22
   <212 DNA
   <213> Artificial Sequence
<220>
   <223> Forward exon 1 primer for CFH gene
<400> 14
   gtgggagtgc agtgagaatt gg 22
<210> 15
   <211> 21
   <212> DNA
   <213> Artificial Sequence
<220>
   <223> Reverse exon 1 primer for CFH gene
<400> 15
   aactcaacaa tgtcaaaagc c 21
<210> 16
   <211> 23
   <212> DNA
   <213> Artificial Sequence
<220>
   <223> Forward exon 2 primer for CFH gene
<400> 16
   gatagacctg tgactgtcta ggc 23
<210> 17
   <211> 23
   <212> DNA
   <213> Artificial Sequence
<220>
   <223> Reverse exon 2 primer for CFH gene
<400> 17
   ggcaatagtg atataattca ggc 23
<210> 18
   <211> 20
   <212> DNA
   <213> Artificial Sequence
<220>
   <223> Forward exon 3 primer for CFH gene
<400> 18
   acctcagcct cccaaagtgc 20
<210> 19
   <211> 23
   <212> DNA
   <213> Artificial Sequence
<220>
   <223> Reverse exon 3 primer for CFH gene
<400> 19
   tgcatactgt tttcccactc tcc 23
<210> 20
   <211> 23
   <212> DNA
   <213> Artificial Sequence
<220>
   <223> Forward exon 4 primer for CFH gene
<400> 20
   aaggaggagg agaaggagga agg 23
<210> 21
   <211> 20
   <212> DNA
   <213> Artificial Sequence
<220>
   <223> Reverse exon 4 primer for CFH gene
<400> 21
   caggctgcat tcgtttttgg 20
<210> 22
   <211> 24
   <212> DNA
   <213> Artificial Sequence
<220>
   <223> Forward exon 5 primer for CFH gene
<400> 22
   ccactcccat agaaaagaat cagg 24
<210> 23
   <211> 23
   <212> DNA
   <213> Artificial Sequence
<220>
   <223> Reverse exon 5 primer for CFH gene
<400> 23
   acttctttgc accagtctct tcc 23
<210> 24
   <211> 21
   <212> DNA
   <213> Artificial Sequence
<220>
   <223> Forward exon 6 primer for CFH gene
<400> 24
   gataaatcat ttattaagcg g 21
<210> 25
   <211> 22
   <212> DNA
   <213> Artificial Sequence
<220>
   <223> Reverse exon 6 primer for CFH gene
<400> 25
   gaaccttgaa cacagaaaat gc 22
<210> 26
   <211> 21
   <212> DNA
   <213> Artificial Sequence
<220>
   <223> Forward exon 7 primer for CFH gene
<400> 26
   ggatgacttt ggagaagaag g 21
<210> 27
   <211> 20
   <212> DNA
   <213> Artificial Sequence
<220>
   <223> Reverse exon 7 primer for CFH gene
<400> 27
   tatgagtttc ggcaacttcg 20
<210> 28
   <211> 21
   <212> DNA
   <213> Artificial Sequence
<220>
   <223> Forward exon 8 primer for CFH gene
<400> 28
   tcatcttcat taacaaagac c 21
<210> 29
   <211> 22
   <212> DNA
   <213> Artificial Sequence
<220>
   <223> Reverse exon 8 primer for CFH gene
<400> 29
   agatctattt tggtcacttt gc 22
<210> 30
   <211> 22
   <212> DNA
   <213> Artificial Sequence
<220>
   <223> Forward exon 9 primer for CFH gene
<400> 30
   ctttgttagt aactttagtt cg 22
<210> 31
   <211> 20
   <212> DNA
   <213> Artificial Sequence
<220>
   <223> Reverse exon 9 primer for CFH gene
<400> 31
   ttatacacag ttgaaaaacc 20
<210> 32
   <211> 22
   <212> DNA
   <213> Artificial Sequence
<220>
   <223> Forward exon 10 primer for CFH gene
<400> 32
   ggcaactctg agcttatttt cc 22
<210> 33
   <211> 22
   <212> DNA
   <213> Artificial Sequence
<220>
   <223> Reverse exon 10 primer for CFH gene
<400> 33
   agagtaggaa aagcctgaat gg 22
<210> 34
   <211> 20
   <212> DNA
   <213> Artificial Sequence
<220>
   <223> Forward exon 11 primer for CFH gene
<400> 34
   catagattat ttttgtacgg 20
<210> 35
   <211> 20
   <212> DNA
   <213> Artificial Sequence
<220>
   <223> Reverse exon 11 primer for CFH gene
<400> 35
   caaaactccc ttcttttccc 20
<210> 36
   <211> 23
   <212> DNA
   <213> Artificial Sequence
<220>
   <223> Forward exon 12 primer for CFH gene
<400> 36
   atctgatgcc cctctgtatg acc 23
   <210> 37
   <211> 22
   <212> DNA
   <213> Artificial Sequence
<220>
   <223> Reverse exon 12 primer for CFH gene
<400> 37
   attcagtact caatacatgt cc 22
<210> 38
   <211> 21
   <212> DNA
   <213> Artificial Sequence
<220>
   <223> Forward exon 13 primer for CFH gene
<400> 38
   caccattctt gattgtttag g 21
<210> 39
   <211> 20
   <212> DNA
   <213> Artificial Sequence
<220>
   <223> Reverse exon 13 primer for CFH gene
<400> 39
   gaatctccat agtaataagg 20
<210> 40
   <211> 21
   <212> DNA
   <213> Artificial Sequence
<220>
   <223> Forward exon 14 primer for CFH gene
<400> 40
   caatgtgttg atggagagtg g 21
<210> 41
   <211> 21
   <212> DNA
   <213> Artificial Sequence
<220>
   <223> Reverse exon 14 primer for CFH gene
<400> 41
   attgaattat aagcaatatg c 21
<210> 42
   <211> 22
   <212> DNA
   <213> Artificial Sequence
<220>
   <223> Forward exon 15 primer for CFH gene
<400> 42
   catttcagcg acagaataca gg 22
<210> 43
   <211> 20
   <212> DNA
   <213> Artificial Sequence
<220>
   <223> Reverse exon 15 primer for CFH gene
<400> 43
   gtgtgtgtgt gtgtgtgtgc 20
<210> 44
   <211> 23
   <212> DNA
   <213> Artificial Sequence
<220>
   <223> Forward intron 15 primer for CFH gene
<400> 44
   aaggcaggaa agtgtcctta tgc 23
<210> 45
   <211> 21
   <212> DNA
   <213> Artificial Sequence
<220>
   <223> Reverse intron 15 primer for CFH gene
<400> 45
   gtcaaattac tgaaaatcac c 21
<210> 46
   <211> 21
   <212> DNA
   <213> Artificial Sequence
<220>
   <223> Forward exon 16 primer for CFH gene
<400> 46
   aactgttaca cagctgaaaa g 21
<210> 47
   <211> 20
   <212> DNA
   <213> Artificial Sequence
<220>
   <223> Reverse exon 16 primer for CFH gene
<400> 47
   gtggtgattg attaatgtgc 20
<210> 48
   <211> 21
   <212> DNA
   <213> Artificial Sequence
<220>
   <223> Forward exon 17 primer for CFH gene
<400> 48
   ggtggaggaa tatatctttg c 21
<210> 49
   <211> 21
   <212> DNA
   <213> Artificial Sequence
<220>
   <223> Reverse exon 17 primer for CFH gene
<400> 49
   atagaataga ttcaatcatg c 21
<210> 50
   <211> 25
   <212> DNA
   <213> Artificial Sequence
<220>
   <223> Forward exon 18 primer for CFH gene
<400> 50
   cgatagacag acagacacca gaagg 25
<210> 51
   <211> 25
   <212> DNA
   <213> Artificial Sequence
<220>
   <223> Reverse exon 18 primer for CFH gene
<400> 51
   cagctataat ttcccacagc agtcc 25
<210> 52
   <211> 26
   <212> DNA
   <213> Artificial Sequence
<220>
   <223> Forward exon 19 primer for CFH gene
<400> 52
   gtgtaatctc aattgctacg gctacc 26
<210> 53
   <211> 23
   <212> DNA
   <213> Artificial Sequence
<220>
   <223> Reverse exon 19 primer for CFH gene
<400> 53
   caagtagctg ggacttcaga tgc 23
<210> 54
   <211> 20
   <212> DNA
   <213> Artificial Sequence
<220>
   <223> Forward exon 20 primer for CFH gene
<400> 54
   tagtttcatg tcttttcctc 20
<210> 55
   <211> 21
   <212> DNA
   <213> Artificial Sequence
<220>
   <223> Reverse exon 20 primer for CFH gene
<400> 55
   gaattttaag caccatcagt c 21
<210> 56
   <211> 21
   <212> DNA
   <213> Artificial Sequence
<220>
   <223> Forward exon 21 primer for CFH gene
<400> 56
   ccaggactca tttctttcac c 21
<210> 57
   <211> 21
   <212> DNA
   <213> Artificial Sequence
<220>
   <223> Reverse exon 21 primer for CFH gene
<400> 57
   ctttctgaca gaaatatttg g 21
<210> 58
   <211> 20
   <212> DNA
   <213> Artificial Sequence
<220>
   <223> Forward exon 22 primer for CFH gene
<400> 58
   tgatgtttct acatagttgg 20
<210> 59
   <211> 25
   <212> DNA
   <213> Artificial Sequence
<220>
   <223> Reverse exon 22 primer for CFH gene
<400> 59
   ggagtaaaac aatacataaa aaatg 25